(19) Europäisches Patentamt
European Patent Office
Office européen des brevets

(11) EP 4 671 371 A1

(12) **EUROPEAN PATENT APPLICATION**
published in accordance with Art. 153(4) EPC

(43) Date of publication:
**31.12.2025 Bulletin 2026/01**

(21) Application number: **24759785.9**

(22) Date of filing: **23.02.2024**

(51) International Patent Classification (IPC):
***C12N 15/113*** *(2010.01)* ***A61K 31/713*** *(2006.01)*
***A61P 31/20*** *(2006.01)*

(52) Cooperative Patent Classification (CPC):
**A61K 31/713; A61P 31/20; C12N 15/113**

(86) International application number:
**PCT/CN2024/078398**

(87) International publication number:
**WO 2024/175113 (29.08.2024 Gazette 2024/35)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority:
**24.02.2023 CN 202310177446**
**28.02.2023 CN 202310191440**
**23.04.2023 CN 202310453443**
**13.11.2023 CN 202311511833**
**19.01.2024 CN 202410084357**

(71) Applicant: **Yaopharma Co., Ltd.**
**Chongqing 401121 (CN)**

(72) Inventors:
- **HE, Haiying**
  **Shanghai 200131 (CN)**
- **HU, Yanbin**
  **Shanghai 200131 (CN)**
- **LU, Jianyu**
  **Shanghai 200131 (CN)**
- **CHEN, Shuhui**
  **Shanghai 200131 (CN)**

(74) Representative: **Murgitroyd & Company**
**165-169 Scotland Street**
**Glasgow G5 8PL (GB)**

(54) **DOUBLE-STRANDED SIRNA ANALOGS COMPRISING R AND E AND CONJUGATES THEREOF**

(57) Provided are a series of double-stranded siRNA analogs comprising R and/or E, conjugates thereof, salts of the conjugates, and uses.

EP 4 671 371 A1

## Description

SPECIFICATION

The present invention claims the right of the following priorities:

CN2023101774460, application date: February 24, 2023;
CN2023101914409, application date: February 28, 2023;
CN2023104534435, application date: April 23, 2023;
CN202311511833X, application date: November 13, 2023;
CN2024100843576, application date: January 19, 2024.

TECHNICAL FIELD

The present disclosure relates to the field of biomedicine, specifically to a double-stranded siRNA analog comprising E and R, a conjugate thereof, a salt of the conjugate thereof, and a use thereof.

BACKGROUND

Viral hepatitis B, referred to as hepatitis B, is a disease caused by the infection of hepatitis B virus (HBV). Hepatitis B virus is a hepatotropic virus that mainly exists in and damages liver cells, causing liver cell inflammation, necrosis, and fibrosis. Viral hepatitis B is divided into acute and chronic types. Most cases of acute hepatitis B in adults can be cured through their own immune mechanisms. However, chronic hepatitis B (CHB) has become a major global healthcare challenge and remains a leading cause of chronic liver disease, cirrhosis, and hepatocellular carcinoma (HCC). It is estimated that 2 billion people worldwide are infected with chronic hepatitis B virus, with over 350 million having developed hepatitis B, and nearly 600,000 deaths annually due to complications from chronic hepatitis B. China is a high-prevalence region for hepatitis B, with a large cumulative number of cases and serious harm. Data indicates that there are about 93 million hepatitis B virus carriers in China, among which about 20 million are diagnosed with chronic hepatitis B. Of these, 10% to 20% may progress to cirrhosis, and 1% to 5% may develop into hepatocellular carcinoma.

The key to functional cure for hepatitis B lies in the clearance of HBsAg (hepatitis B virus surface antigen) and the production of surface antibodies. In chronically infected patients, reduction of HBsAg and seroconversion are rarely observed. Currently approved anti-HBV drugs on the market primarily include immunomodulators (interferon-$\alpha$ and pegylated interferon-$\alpha$-2$\alpha$) and antiviral therapeutic drugs (lamivudine, adefovir dipivoxil, entecavir, telbivudine, tenofovir, clevudine, *etc.*). Among them, antiviral therapeutic drugs belong to nucleotide drugs, whose mechanism of action is to inhibit the synthesis of HBV DNA and cannot directly reduce HBsAg levels. There are clinical therapies available to reduce HBsAg, but the efficacy is poor. Therefore, if viral gene expression can be silenced at the genetic level to block HBV production and replication-particularly the generation of HBsAg and HBeAg (hepatitis B S antigen and E antigen)-it would fundamentally reduce viral metabolism and hepatocyte infection.

Small interfering RNA (siRNA), based on the mechanism of RNA interference (RNAi), can inhibit or block the expression of target genes in a sequence-specific manner, exerting an inhibitory effect at the level of mRNA translation to protein, thereby achieving the purpose of treating diseases. This most ideal hepatitis B treatment approach requires stabilization modification of siRNA and the use of a corresponding delivery system to target the target organs and cells to improve metabolic stability, but current siRNA cannot effectively reduce the content of hepatitis B virus S antigen and E antigen. Meanwhile, siRNA can partially complementarily pair with certain mRNA fragments, thereby regulating the expression of genes corresponding to the mRNA. In particular, the complementary pairing of the 5' end seed region of the antisense strand of siRNA with a non-targeted gene will partially or completely silence the expression of the gene, which is the main reason for the off-target effect of siRNA *in vivo* and *in vitro.* siRNA for the treatment of hepatitis B has exposed this shortcoming in both the clinical and preclinical stages. Although some modifications to nucleotides can reduce the risk of off-target, the effectiveness of their silencing has also been reduced, and the therapeutic safety window needs to be improved.

SUMMARY

The present disclosure provides a double-stranded siRNA analog, a conjugate thereof, a salt thereof, or a salt of the conjugate thereof, comprising a sense strand and an antisense strand capable of forming a double-stranded region, wherein the double-stranded siRNA analog is any one selected from the group consisting of the double strands shown in Table 1, and each nucleotide on the double strand is independently and optionally modified.

In some embodiments of the present disclosure, the double-stranded siRNA analog is selected from the group

consisting of S1, S2, S3, S4, S5, S6, S7, S8, S9, S10, S11, S12, S13, S14, S15, S16, S17, S18, S19, S20, S21, S22, S23, S24, S25, S26, S27, and S28.

In some embodiments of the present disclosure, the double-stranded siRNA analog is selected from the group consisting of S1, S2, S3, S4, S5, S6, S7, S8, S9, S10, and S11.

The present disclosure provides a double-stranded siRNA analog, a conjugate thereof, a salt thereof, or a salt of the conjugate thereof, selected from the group consisting of S1, S2, S3, S4, S5, S6, S7, S8, S9, S10, S11, S12, S13, S14, S15, S16, S17, S18, S19, S20, S21, S22, S23, S24, S25, and S26.

The unmodified double-stranded siRNA analogs provided by the present disclosure are shown in Table 1.

**Table 1. Unmodified double-stranded siRNA analogs**

| No. | SEQ ID NO | Strand type | Direction | Sequence | Direction |
|---|---|---|---|---|---|
| **S1** | 6 | AS | 5' | RGUGAAGCGAAGUGCACACGGU | 3' |
| | 34 | SS | 3' | CCACUUCGCUUCACGUG | 5' |
| **S2** | 7 | AS | 5' | RGUEAAGCGAAGUGCACACGGU | 3' |
| | 8 | SS | 3' | CCACUUCGCUUCACEUG | 5' |
| **S3** | 9 | AS | 5' | RGUGAAECGAAGUGCACACGGU | 3' |
| | 8 | SS | 3' | CCACUUCGCUUCACEUG | 5' |
| **S4** | 7 | AS | 5' | RGUEAAGCGAAGUGCACACGGU | 3' |
| | 10 | SS | 3' | CCACUUCGCUUCACGUE | 5' |
| **S5** | 11 | AS | 5' | RGUGAAGCEAAGUGCACACGGU | 3' |
| | 8 | SS | 3' | CCACUUCGCUUCACEUG | 5' |
| **S6** | 12 | AS | 5' | RGUGAAGCGAAEUGCACACGGU | 3' |
| | 8 | SS | 3' | CCACUUCGCUUCACEUG | 5' |
| **S7** | 13 | AS | 5' | RGUGAAGCGAAGUGCACACEGU | 3' |
| | 8 | SS | 3' | CCACUUCGCUUCACEUG | 5' |
| **S8** | 14 | AS | 5' | RGUGAAGCGAAGUGCACACGEU | 3' |
| | 8 | SS | 3' | CCACUUCGCUUCACEUG | 5' |
| **S9** | 13 | AS | 5' | RGUGAAGCGAAGUGCACACEGU | 3' |
| | 15 | SS | 3' | CCACUUCGCUUCACEUGU | 5' |
| **S10** | 16 | AS | 5' | UGCAGAUGUGAAGCGAAGUGCACACEGU | 3' |
| | 17 | SS | 3' | ACACUUCGCUUCACRUG | 5' |
| **S11** | 4 | AS | 5' | UGCAGAUGUGAAGCGAAGUGCACACGGU | 3' |
| | 17 | SS | 3' | ACACUUCGCUUCACRUG | 5' |
| **S26** | 4 | AS | 5' | UGCAGAUGUGAAGCGAAGUGCACACGGU | 3' |
| | 5 | SS | 3' | ACACUUCGCUUCACGUG | 5' |
| **S27** | 33 | AS | 5' | GGUGAAGCGAAGUGCACACGGU | 3' |
| | 34 | SS | 3' | CCACUUCGCUUCACGUG | 5' |
| **S28** | 33 | AS | 5' | GGUGAAGCGAAGUGCACACGGU | 3' |
| | 3 | SS | 3' | CCACUUCGCUUCACGUGU | 5' |

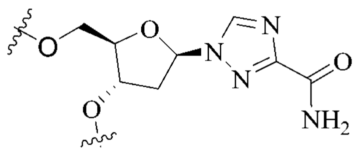

The R and E in the present disclosure are R and

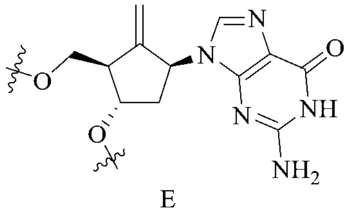

E

,

respectively.

In some embodiments of the present disclosure, when the R or E is located at the 5' end of the AS or SS of the siRNA sequence, the 5' position of R or E may be a hydroxyl group, a phosphate ester bond, or a phosphorothioate ester bond, and the 2' position of R or E is optionally modified.

In some embodiments of the present disclosure, the sense strand of the double-stranded siRNA analog comprises 0, 1, 2, 3, 4, 5, or 6 unmodified nucleotides.

In some embodiments of the present disclosure, the antisense strand of the double-stranded siRNA analog comprises 0, 1, 2, 3, 4, 5, or 6 unmodified nucleotides.

In some embodiments of the present disclosure, the sense strand of the double-stranded siRNA analog comprises 0, 1, 2, or 3 nucleotides in which the nucleoside is replaced by R.

In some embodiments of the present disclosure, the antisense strand of the double-stranded siRNA analog comprises 0, 1, 2, or 3 nucleotides in which the nucleoside is replaced by R.

In some embodiments of the present disclosure, the sense strand of the double-stranded siRNA analog comprises 0, 1, 2, or 3 nucleotides in which the nucleoside is replaced by E.

In some embodiments of the present disclosure, the antisense strand of the double-stranded siRNA analog comprises 0, 1, 2, or 3 nucleotides in which the nucleoside is replaced by E.

In some embodiments of the present disclosure, the double-stranded siRNA analog is any one selected from the group consisting of the double strands shown in Table 2.

**Table 2. Modified double-stranded siRNA analogs**

| No. | SEQ ID NO | Strand type | Direction | Sequence | Direction |
|---|---|---|---|---|---|
| S12 | 18 | AS | 5' | mRsfGsmUmGmAmAmGmCmGmAmAm GmUfGmCfAmCmAmCsmGsmGU | 3' |
| | 19 | SS | 3' | mCmCmAmCmUmUmCmGmCmUfUfCfA mCmGsmUsmG | 5' |
| S13 | 20 | AS | 5' | $^{MOE}$RsfGsmUEmAmAmGmCmGmAmAm GmUfGmCfAmCmAmCsmGsmGU | 3' |
| | 21 | SS | 3' | mCmCmAmCmUmUmCmGmCmUfUfCfA mCEsmUsmG | 5' |
| S14 | 22 | AS | 5' | $^{MOE}$RsfGsmUmGmAmAEmCmGmAmAm GmUfGmCfAmCmAmCsmGsmGU | 3' |
| | 21 | SS | 3' | mCmCmAmCmUmUmCmGmCmUfUfCfA mCEsmUsmG | 5' |

(continued)

| No. | SEQ ID NO | Strand type | Direction | Sequence | Direction |
|---|---|---|---|---|---|
| S15 | 20 | AS | 5' | MOERsfGsmUEmAmAmGmCmGmAmAm GmUfGmCfAmCmAmCsmGsmGU | 3' |
| | 23 | SS | 3' | mCmCmAmCmUmUmCmGmCmUfUfCfA mCmGsmUsE | 5' |
| S16 | 24 | AS | 5' | mRsfGsmUEmAmAmGmCmGmAmAmG mUfGmCfAmCmAmCsmGsmGU | 3' |
| | 21 | SS | 3' | mCmCmAmCmUmUmCmGmCmUfUfCfA mCEsmUsmG | 5' |
| S17 | 25 | AS | 5' | mRsfGsmUmGmAmAmGmCEmAmAmG mUfGmCfAmCmAmCsmGsmGU | 3' |
| | 21 | SS | 3' | mCmCmAmCmUmUmCmGmCmUfUfCfA mCEsmUsmG | 5' |
| S18 | 26 | AS | 5' | mRsfGsmUmGmAmAmGmCmGmAmAE mUfGmCfAmCmAmCsmGsmGU | 3' |
| | 21 | SS | 3' | mCmCmAmCmUmUmCmGmCmUfUfCfA mCEsmUsmG | 5' |
| S19 | 27 | AS | 5' | mRsfGsmUmGmAmAmGmCmGmAmAm GmUfGmCfAmCmAmCsEsmGU | 3' |
| | 21 | SS | 3' | mCmCmAmCmUmUmCmGmCmUfUfCfA mCEsmUsmG | 5' |
| S20 | 28 | AS | 5' | mRsfGsmUmGmAmAmGmCmGmAmAm GmUfGmCfAmCmAmCsmGsEU | 3' |
| | 21 | SS | 3' | mCmCmAmCmUmUmCmGmCmUfUfCfA mCEsmUsmG | 5' |
| S21 | 29 | AS | 5' | mRsfGsmUmGmAmAEmCmGmAmAmG mUfGmCfAmCmAmCsmGsmGU | 3' |
| | 21 | SS | 3' | mCmCmAmCmUmUmCmGmCmUfUfCfA mCEsmUsmG | 5' |

(continued)

| No. | SEQ ID NO | Strand type | Direction | Sequence | Direction |
|---|---|---|---|---|---|
| S22 | 27 | AS | 5' | mRsfGsmUmGmAmAmGmCmGmAmAmGmUfGmCfAmCmAmCsEsmGU | 3' |
| | 30 | SS | 3' | mCmCmAmCmUmUmCmGmCmUfUfCfAmCEmUsmGsmU | 5' |
| S23 | 31 | AS | 5' | mRsfGsmUmGmAmAmGmCmGmAmAmGmUfGmCfAmCmAmCEsmGsU | 3' |
| | 21 | SS | 3' | mCmCmAmCmUmUmCmGmCmUfUfCfAmCEsmUsmG | 5' |
| S24 | 32 | AS | 5' | UGCAGAmUsfGsmUmGmAmAmGmCmGmAmAmGmUfGmCfAmCmAmCEsmGsmU | 3' |
| | 2 | SS | 3' | mAmCmAmCmUmUmCmGmCmUfUfCfAmCmRsmUsmG | 5' |
| S25 | 1 | AS | 5' | UGCAGAmUsfGsmUmGmAmAmGmCmGmAmAmGmUfGmCfAmCmAmCmGsmGsmU | 3' |
| | **2** | SS | 3' | mAmCmAmCmUmUmCmGmCmUfUfCfAmCmRsmUsmG | 5' |

In some embodiments of the present disclosure, the mR is

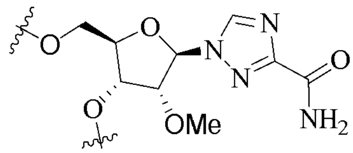

,

and the intermediate for synthesizing the mR is shown in compound 2:

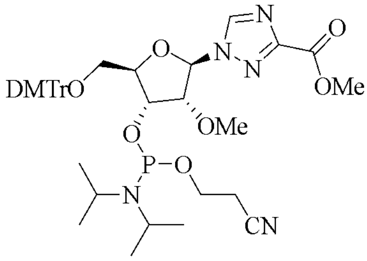

2 .

In some embodiments of the present disclosure, the $^{MOE}R$ is

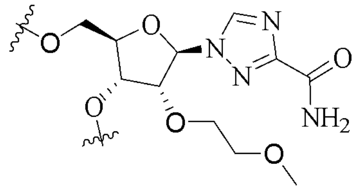

,

and the intermediate for synthesizing the $^{MOE}R$ is shown in compound 1:

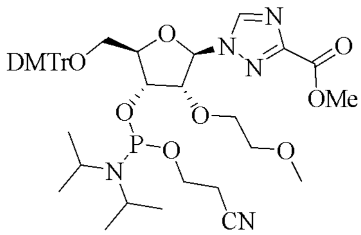

1

.

In some embodiments of the present disclosure, the E is

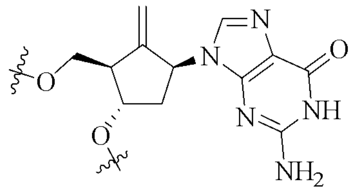

,

and the intermediate for synthesizing the E is shown in compound 3:

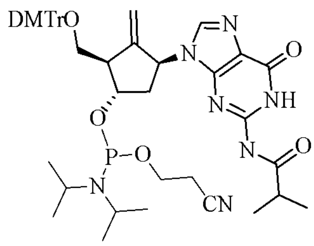

3

.

In some embodiments of the present disclosure, the conjugate of the double-stranded siRNA analog or the salt of the conjugate thereof is formed by conjugating the double-stranded siRNA analog with a pharmaceutically acceptable conjugating group.

In some embodiments of the present disclosure, the pharmaceutically acceptable conjugating group in the conjugate of the double-stranded siRNA analog or the salt of the conjugate thereof comprises 1 to 5 GalNAc groups.

In some embodiments of the present disclosure, the pharmaceutically acceptable conjugating group is linked to any position of the double-stranded siRNA analog.

In some embodiments of the present disclosure, the pharmaceutically acceptable conjugating group is linked to the 3' end of the sense strand of the double-stranded siRNA analog.

In some embodiments of the present disclosure, the pharmaceutically acceptable conjugating group in the conjugate of the double-stranded siRNA analog or the salt thereof is selected from the group consisting of D1, D2, D3, and L96,

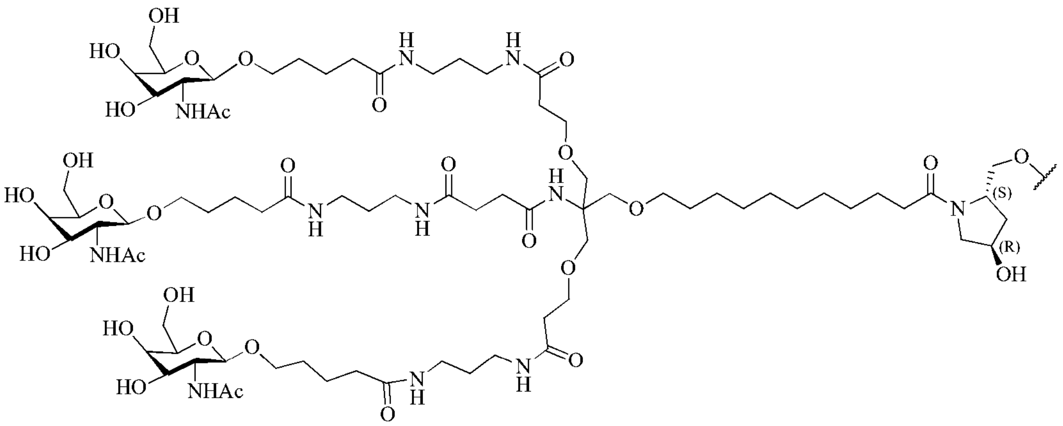

D1

,

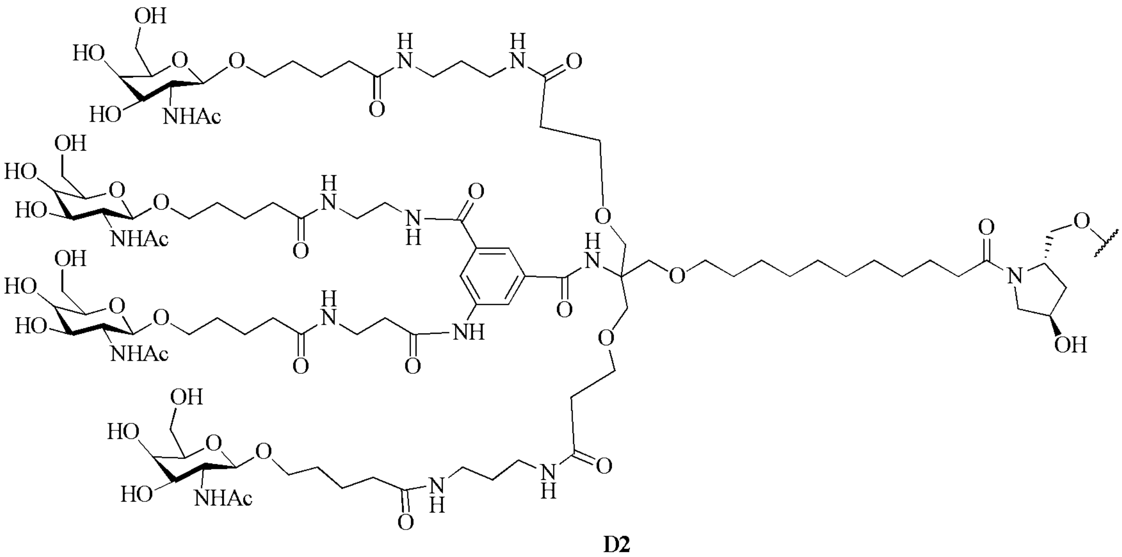

D2

,

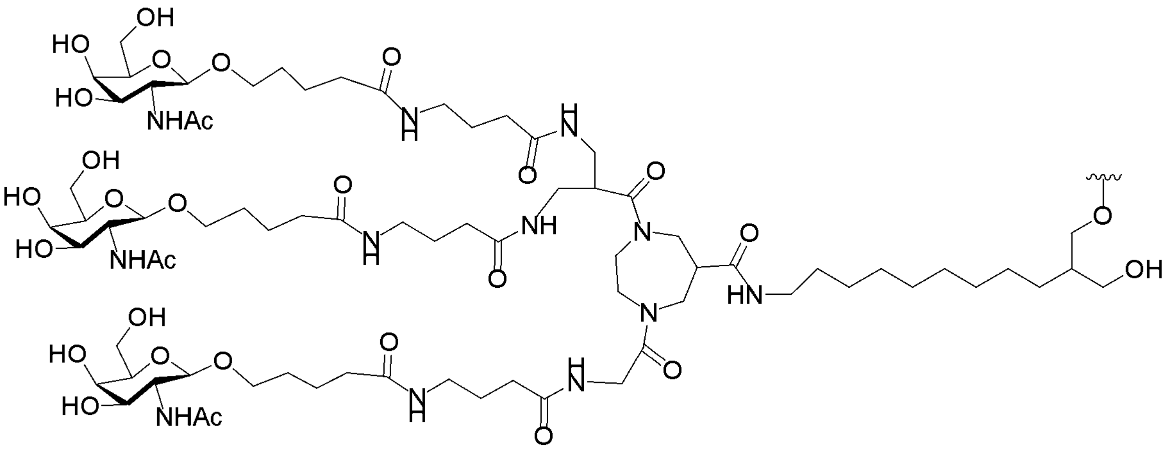

D3

,

and

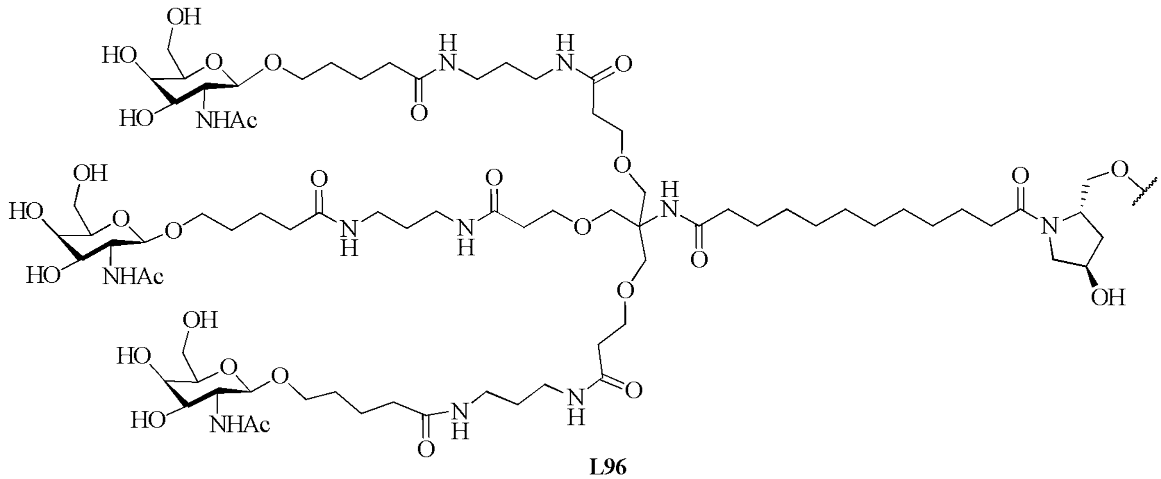

L96

.

In some embodiments of the present disclosure, the 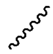 in the conjugating group represents that the conjugating group is linked to the double-stranded siRNA analog at this site via a phosphate diester bond or a phosphorothioate ester bond.

There are still some embodiments of the present disclosure which are obtained by any combination of the above embodiments.

The present disclosure also provides a conjugate of a double-stranded siRNA analog or a salt thereof, selected from the group consisting of Z1, Z2, Z3, Z4, Z5, Z6, Z7, Z8, Z9, Z10, Z11, Z12, Z13, Z14, Z15, and Z16.

The conjugates of the double-stranded siRNA analogs provided by the present disclosure are shown in Table 3.

**Table 3. Conjugates of double-stranded siRNA analogs**

| No. | Strand type | Direction | Conjuga-ting group | SEQ ID NO | Sequence | Direction |
|---|---|---|---|---|---|---|
| Z1 | AS | 5' | | 18 | mRsfGsmUmGmAmAmGmCm GmAmAmGmUfGmCfAmCmA mCsmGsmGU | 3' |
| | SS | 3' | D1 | 19 | mCmCmAmCmUmUmCmGmC mUfUfCfAmCmGsmUsmG | 5' |
| Z2 | AS | 5' | | 20 | $^{MOE}$RsfGsmUEmAmAmGmCm GmAmAmGmUfGmCfAmCmA mCsmGsmGU | 3' |
| | SS | 3' | D1 | 21 | mCmCmAmCmUmUmCmGmC mUfUfCfAmCEsmUsmG | 5' |
| Z3 | AS | 5' | | 20 | $^{MOE}$RsfGsmUEmAmAmGmCm GmAmAmGmUfGmCfAmCmA mCsmGsmGU | 3' |
| | SS | 3' | D2 | 21 | mCmCmAmCmUmUmCmGmC mUfUfCfAmCEsmUsmG | 5' |

(continued)

| No. | Strand type | Direction | Conjuga-ting group | SEQ ID NO | Sequence | Direction |
|---|---|---|---|---|---|---|
| **Z4** | AS | 5' | | 22 | $^{MOE}$RsfGsmUmGmAmAEmCmGmAmAmGmUfGmCfAmCmAmCsmGsmGU | 3' |
| | SS | 3' | D2 | 21 | mCmCmAmCmUmUmCmGmCmUfUfCfAmCEsmUsmG | 5' |
| **Z5** | AS | 5' | | 20 | $^{MOE}$RsfGsmUEmAmAmGmCmGmAmAmGmUfGmCfAmCmAmCsmGsmGU | 3' |
| | SS | 3' | D2 | 23 | mCmCmAmCmUmUmCmGmCmUfUfCfAmCmGsmUsE | 5' |
| **Z6** | AS | 5' | | 24 | mRsfGsmUEmAmAmGmCmGmAmAmGmUfGmCfAmCmAmCsmGsmGU | 3' |
| | SS | 3' | D1 | 21 | mCmCmAmCmUmUmCmGmCmUfUfCfAmCEsmUsmG | 5' |
| **Z7** | AS | 5' | | 24 | mRsfGsmUEmAmAmGmCmGmAmAmGmUfGmCfAmCmAmCsmGsmGU | 3' |
| | SS | 3' | D2 | 21 | mCmCmAmCmUmUmCmGmCmUfUfCfAmCEsmUsmG | 5' |
| **Z8** | AS | 5' | | 25 | mRsfGsmUmGmAmAmGmCEmAmAmGmUfGmCfAmCmAmCsmGsmGU | 3' |
| | SS | 3' | D2 | 21 | mCmCmAmCmUmUmCmGmCmUfUfCfAmCEsmUsmG | 5' |
| **Z9** | AS | 5' | | 26 | mRsfGsmUmGmAmAmGmCmGmAmAEmUfGmCfAmCmAmCsmGsmGU | 3' |
| | SS | 3' | D2 | 21 | mCmCmAmCmUmUmCmGmCmUfUfCfAmCEsmUsmG | 5' |

(continued)

| No. | Strand type | Direction | Conjuga -ting group | SEQ ID NO | Sequence | Direction |
|---|---|---|---|---|---|---|
| Z10 | AS | 5’ | | 27 | mRsfGsmUmGmAmAmGmCmGmAmAmGmUfGmCfAmCmAmCsEsmGU | 3’ |
| | SS | 3’ | D2 | 21 | mCmCmAmCmUmUmCmGmCmUfUfCfAmCEsmUsmG | 5’ |
| Z11 | AS | 5’ | | 28 | mRsfGsmUmGmAmAmGmCmGmAmAmGmUfGmCfAmCmAmCsmGsEU | 3’ |
| | SS | 3’ | D2 | 21 | mCmCmAmCmUmUmCmGmCmUfUfCfAmCEsmUsmG | 5’ |
| Z12 | AS | 5’ | | 29 | mRsfGsmUmGmAmAEmCmGmAmAmGmUfGmCfAmCmAmCsmGsmGU | 3’ |
| | SS | 3’ | D2 | 21 | mCmCmAmCmUmUmCmGmCmUfUfCfAmCEsmUsmG | 5’ |
| Z13 | AS | 5’ | | 27 | mRsfGsmUmGmAmAmGmCmGmAmAmGmUfGmCfAmCmAmCsEsmGU | 3’ |
| | SS | 3’ | D2 | 30 | mCmCmAmCmUmUmCmGmCmUfUfCfAmCEmUsmGsmU | 5’ |
| Z14 | AS | 5’ | | 31 | mRsfGsmUmGmAmAmGmCmGmAmAmGmUfGmCfAmCmAmCEsmGsU | 3’ |
| | SS | 3’ | D2 | 21 | mCmCmAmCmUmUmCmGmCmUfUfCfAmCEsmUsmG | 5’ |
| Z15 | AS | 5’ | | 32 | UGCAGAmUsfGsmUmGmAmAmGmCmGmAmAmGmUfGmCfAmCmAmCEsmGsmU | 3’ |
| | SS | 3’ | D3 | 2 | mAmCmAmCmUmUmCmGmCmUfUfCfAmCmRsmUsmG | 5’ |

(continued)

| No. | Strand type | Direction | Conjuga -ting group | SEQ ID NO | Sequence | Direction |
|---|---|---|---|---|---|---|
| Z16 | AS | 5’ | | 1 | UGCAGAmUsfGsmUmGmAm AmGmCmGmAmAmGmUfGm CfAmCmAmCmGsmGsmU | 3’ |
| | SS | 3’ | D3 | 2 | mAmCmAmCmUmUmCmGm CmUfUfCfAmCmRsmUsmG | 5’ |

The present disclosure also provides a use of the double-stranded siRNA analog, the conjugate thereof, the salt thereof, or the salt of the conjugate thereof in the manufacture of a medicament for treating hepatitis B.

**Technical effect**

The double-stranded siRNA analog, the conjugate thereof, the salt thereof, or the salt of the conjugate thereof of the present disclosure has good anti-HBV biological activity. The compound of the present disclosure exhibits excellent anti-HBsAg activity both *in vitro* and *in vivo,* and especially exhibits excellent *in vivo* anti-HBV DNA activity in an AAV-HBV mouse model. At the same time, the compound of the present disclosure has good *in vitro* liver S9 stability, low immunogenicity risk and off-target risk, and preliminary safety studies show that the compound of the present disclosure has good safety.

**Definition and description**

Unless otherwise specified, the following terms and phrases when used herein have the following meanings. A specific term or phrase should not be considered indefinite or unclear in the absence of a particular definition, but should be understood according to the meaning ordinarily understood by those skilled in the art. When a trading name appears herein, it is intended to refer to its corresponding commodity or active ingredient thereof.

Unless otherwise specified, the terms "comprise", "include", and "contain" or equivalents described in the present disclosure are open-ended expressions, meaning that in addition to the listed elements, components, or steps, other unspecified elements, components, or steps may also be encompassed.

The term "optional" or "optionally" means that the subsequent event or condition may occur but not requisite, that the term includes the instance in which the event or condition occurs and the instance in which the event or condition does not occur.

Unless otherwise specified, the term "nucleic acid" described in the present disclosure refers to a polymer in single-stranded or double-stranded form containing at least two nucleotides (*i.e.*, deoxyribonucleotides or ribonucleotides), and includes both DNA and RNA. A "nucleotide" contains a deoxyribose (DNA) or ribose (RNA), a base, and a phosphate ester group. Nucleotides are linked together via phosphate ester groups. A "base" includes purines and pyrimidines, which further includes natural compounds such as adenine, thymine, guanine, cytosine, uracil, inosine, and their natural analogs.

Unless otherwise specified, the term "oligo" or "oligonucleotide" described in the present disclosure refer to a polymer or oligomer of nucleotide or nucleoside monomers composed of natural bases, sugars, and intersugar (backbone) linkages, and further include polymers or oligomers containing non-natural monomers or portions thereof that function similarly.

Unless otherwise specified, the "oligo" or "oligonucleotide" described in the present disclosure is a nucleotide sequence containing 10 to 50 nucleotides or nucleotide base pairs. In some embodiments of the present disclosure, the oligonucleotide has a nucleobase sequence that is at least partially complementary to the coding sequence in a target nucleic acid or target gene expressed within a cell. The nucleotides may optionally be modified. In some embodiments of the present disclosure, after delivering the oligonucleotide to cells expressing the gene, the oligonucleotide can inhibit or block the expression of the gene *in vitro* or *in vivo.* The "oligonucleotide" includes, but is not limited to: single-stranded oligonucleotides, single-stranded antisense oligonucleotides, short interfering RNA (siRNA), double-stranded RNA (dsRNA), microRNA (miRNA), short hairpin RNA (shRNA), ribozymes, interfering RNA molecules, and Dicer enzyme substrates.

Unless otherwise specified, the "short interfering RNA (siRNA)" described in the present disclosure refers to a class of RNA molecules with a length of 14-30 base pairs, similar to miRNA, and functioning within the RNA interference (RNAi) pathway. It interferes with the translation of mRNA of a specific gene that is complementary to the nucleotide sequence, resulting in mRNA degradation. The short interfering RNA (siRNA) described in the present disclosure includes double-stranded siRNA (comprising a sense strand and an antisense strand) and single-stranded siRNA (e.g., comprising only an antisense strand).

Unless otherwise specified, the "inhibition" described in the present disclosure, when referring to the expression of a given gene, indicates that gene expression is reduced when cells, cell populations, or tissues are treated with the oligonucleotides described in the present disclosure, compared to those not so treated.

Unless otherwise specified, the term "double-stranded siRNA analog" described in the present disclosure refers to a complex of ribonucleic acid molecules with a double-stranded structure, comprising two antiparallel and substantially complementary nucleotide strands, which have "sense" and "antisense" orientations relative to the target RNA. In the present disclosure, "complementary" has the meaning well-known to those skilled in the art, *i.e.,* in a double-stranded nucleic acid molecule, the bases of one strand pair with the bases of the other strand in a complementary manner. The purine base adenine (A) always pairs with the pyrimidine base uracil (U); the purine base guanine (C) always pairs with the pyrimidine base cytosine (G). Each base pair includes one purine and one pyrimidine. When adenine on one strand always pairs with uracil on the other strand, and guanine always pairs with cytosine, the two strands are considered complementary to each other, and the sequence of one strand can be inferred from the sequence of its complementary strand.

Unless otherwise specified, the "substantially complementary" described in the present disclosure means that the corresponding positions of two sequences may be fully complementary or may contain one or more mismatches. When mismatches exist, there are typically no more than 5, 4, 3, 2, or 1 mismatched base pairs.

Unless otherwise specified, the "sequence" or "nucleotide sequence" described in the present disclosure refers to the order or sequence of nucleobases or nucleotides represented by a string of letters using standard nucleotide nomenclature.

Unless otherwise specified, the "antisense strand" (AS), "template strand", or "guide strand" described in the present disclosure refers to the strand in an oligonucleotide compound that is substantially complementary to the corresponding region of a target sequence (*e.g.*, AGT mRNA).

Unless otherwise specified, the "sense strand", "SS", "coding strand", or "passenger strand" described in the present disclosure refers to the strand capable of forming a substantially complementary region with the antisense strand. The "substantially complementary" means that the corresponding positions of the two sequences may be fully complementary or may contain one or more mismatches. When mismatches exist, there are typically no more than 3, 2, or 1 mismatched base pairs. In a double-stranded nucleic acid molecule, the bases of one strand pair with the bases of the other strand in a complementary manner. The purine base adenine (A) always pairs with the pyrimidine base uracil (U); the purine base guanine (C) always pairs with the pyrimidine base cytosine (G).

In the present disclosure, the nucleotide monomers "A", "U", "G", and "C" represent adenosine-3’-phosphate, uridine-3’-phosphate, guanosine-3’-phosphate, and cytidine-3’-phosphate, respectively.

Unless otherwise specified, the term "conjugation" described in the present disclosure refers to the covalent linkage of two or more chemical groups, each with a specific function; correspondingly, a "conjugate" refers to a compound formed by the covalent linkage of these chemical groups.

Unless otherwise specified, the term "conjugate of double-stranded siRNA analog" described in the present disclosure refers to a compound formed by linking a double-stranded siRNA analog and a pharmaceutically acceptable conjugating group, and the double-stranded siRNA analog and the pharmaceutically acceptable conjugating group are covalently linked.

Unless otherwise specified, the "pharmaceutically acceptable conjugating group" described in the present disclosure facilitates the *in vivo* delivery of nucleic acids and compositions suitable for *in vivo* therapeutic use. In some embodiments, the "pharmaceutically acceptable conjugating group" described in the present disclosure facilitates enhancement of the affinity of nucleic acids and compositions suitable for *in vivo* therapeutic use to targets (target tissues/cells). Exemplary conjugating groups include, but are not limited to, L96, compound group D1, compound group D2, compound group D3, *etc.*

Unless otherwise specified, the "modification" described in the present disclosure refers to modifications to the base or the sugar ring of the nucleotide or replaced nucleotide (such as R and E replaced), the modifications to the linkage between the nucleotides and/or the replaced nucleotides (such as R and E replaced), *etc.* Exemplarily, the "modification" described in the present disclosure includes, but is not limited to, MOE modification, methoxy modification, fluoro modification, (*E*)-vinyl phosphate ester modification, phosphorothioate ester linkage, or replacement of the nucleotide with GNA (glycerol nucleic acid), *etc.* Exemplarily, the "modification" described in the present disclosure may include one or more locked nucleic acids (LNAs). A locked nucleic acid is a nucleotide with a modified ribose group, wherein the ribose group contains an additional bridge connecting the 2’ carbon and the 4’ carbon. This structure effectively "locks" the ribose in a 3’-endo conformation.

Unless otherwise specified, the "nucleotide optionally modified" described in the present disclosure means that each nucleotide independently may be an unmodified nucleotide or a modified nucleotide, and the modifications on each modified nucleotide are also independent. The "modification" includes, but is not limited to, modifications to the nucleobase, the ribose, and the phosphate ester. The "unmodified nucleotide" refers to a nucleotide composed of a natural nucleobase, a natural sugar ring, and a natural phosphate ester. The "modified nucleotide" refers to a nucleotide containing at least one modification selected from a modified nucleobase, a modified sugar ring, and a modified phosphate ester. In some embodiments of the present disclosure, the "modified nucleotide" refers to a nucleotide composed of a modified nucleobase, and/or a modified sugar ring, and/or a modified phosphate ester. In some embodiments of the present disclosure, a "modified nucleotide" is composed of a modified nucleobase, a natural sugar ring, and a natural phosphate ester; in some embodiments of the present disclosure, a "modified nucleotide" is composed of a natural nucleobase, a modified sugar ring, and a natural phosphate ester; in some embodiments of the present disclosure, a "modified nucleotide" is composed of a natural nucleobase, a natural sugar ring, and a modified phosphate ester; in some embodiments of the present disclosure, a "modified nucleotide" is composed of a natural nucleobase, a modified sugar ring, and a modified phosphate ester; in some embodiments of the present disclosure, a "modified nucleotide" is composed of a modified nucleobase, a natural sugar ring, and a modified phosphate ester; in some embodiments of the present disclosure, a "modified nucleotide" is composed of a modified nucleobase, a modified sugar ring, and a natural phosphate ester; in some embodiments of the present disclosure, a "modified nucleotide" is composed of a modified nucleobase, a modified sugar ring, and a modified phosphate ester. Unless otherwise specified, the "natural sugar ring" described in the present disclosure is selected from the group consisting of a 2'-OH five-membered sugar ring and a 2'-deoxy five-membered sugar ring.

Unless otherwise specified, the "natural base" described in the present disclosure is selected from the group consisting of the purine bases adenine (A) and guanine (G), and the pyrimidine bases thymine (T), cytosine (C), and uracil (U).

Unless otherwise specified, the "modified nucleobase" described in the present disclosure refers to a 5- to 12-membered saturated, partially unsaturated, or aromatic heterocycle other than natural bases, including monocyclic or fused rings, specific examples of which include but are not limited to thiophene, thianthrene, furan, pyran, isobenzofuran, benzothiazine, pyrrole, imidazole, substituted or unsubstituted triazole, pyrazole, isothiazole, isoxazole, pyridazine, indolizine, indole, isoindole, isoquinoline, quinoline, naphthopyridine, quinazoline, carbazole, phenanthridine, piperidine, phenazine, phenazine, phenothiazine, furanidine, phenoxazine, tetrahydropyrrole, pyrroline, imidazolidine, imidazoline, pyrazolidine, 5-methylcytosine (5-me-C), 5-hydroxymethylcytosine, xanthine, hypoxanthine, 2-aminoadenine, 2-aminoadenine, 2-aminoguanine, 2-propyl adenine and 2-propyl guanine and other alkyl derivatives, 2-thiouracil, 2-thiothymine, 2-thiocytosine, 5-halouracil, 5-halocytosine, 5-propynyl uracil, 5-propynyl cytosine, 6-azouracil, 6-azocytosine, 6-azothymine, 5-uracil (pseudouracil), 4-thiouracil, 8-halo, 8-amino, 8-thiol, 8-thioalkyl, 8-hydroxy, and other 8-substituted adenines and guanines, 5-halo especially 5-bromo, 5-trifluoromethyl, and other 5-substituted uracils and cytosines, 7-methylguanine and 7-methyladenine, 8-azaguanine and 8-azaadenine, 7-deazaguanine and 7-deazaadenine, and 3-deazaguanine and 3-deazaadenine, *etc.*

Unless otherwise specified, the "modified sugar ring" described in the present disclosure may include, but is not limited to, one of the following modifications at the 2' position: H; F; O-, S-, or N-alkyl; O-, S-, or N-alkenyl; O-, S-, or N-alkynyl; or O-alkyl-O-alkyl, wherein the alkyl, alkenyl, and alkynyl may be substituted or unsubstituted $C_1$ to $C_{10}$ alkyl or $C_2$ to $C_{10}$ alkenyl and alkynyl. Exemplary suitable modifications include $O[(CH_2)_nO]_mCH_3$, $O(CH_2)_nOCH_3$, $O(CH_2)_nNH_2$, $O(CH_2)_nCH_3$, $O(CH_2)_nONH_2$, and $O(CH_2)_nON[(CH_2)_nCH_3)]_2$, where n and m are from 1 to 10. In other embodiments, the modification at the 2' position includes, but is not limited to, one of the following: substituted or unsubstituted $C_1$ to $C_{10}$ lower alkyl, alkylaryl, aralkyl, O-alkylaryl or O-aralkyl, SH, $SCH_3$, OCN, Cl, Br, CN, $CF_3$, $OCF_3$, $SOCH_3$, $SO_2CH_3$, $ONO_2$, $NO_2$, $N_3$, $NH_2$, heterocycloalkyl, heterocycloalkylaryl, aminoalkylamino, polyalkylamino, substituted silyl, RNA-cleaving groups, reporter groups, intercalators, groups for improving the pharmacokinetic properties of iRNA, or groups for improving the pharmacodynamic properties of iRNA, and other substituents with similar properties. In some embodiments, the modification includes, but is not limited to, 2'-methoxyethoxy (2'-O-$CH_2CH_2OCH_3$, also referred to as 2'-O-(2-methoxyethyl) or 2'-MOE).

Unless otherwise specified, the "modified phosphate ester" described in the present disclosure includes, but is not limited to, phosphorothioate ester modifications, wherein the "phosphorothioate ester" includes (*R*)- and (*S*)-isomers and/or mixtures thereof.

In some embodiments of the present disclosure, the modified nucleotide may include one or more locked nucleic acids (LNAs). A locked nucleic acid is a nucleotide with a modified ribose group, wherein the ribose group contains an additional bridge connecting the 2' carbon and the 4' carbon. This structure effectively "locks" the ribose in a 3'-endo conformation.

In some embodiments of the present disclosure, the modified nucleotide comprises one or more monomers that are UNA (unlocked nucleic acid) nucleotides. UNA is an unlocked acyclic nucleic acid where any sugar linkages have been removed, thereby forming an unlocked "sugar" residue. In one example, UNA also encompasses monomers where the

bond between C1' and C4' has been removed (*i.e.*, the covalent carbon-oxygen-carbon bond between the C1' and C4' carbons). In another example, the C2'-C3' bond of the sugar (*i.e.*, the covalent carbon-carbon bond between the C2' and C3' carbons) has been removed.

In some embodiments of the present disclosure, the modified nucleotide comprises one or more monomers that are GNA (glycol nucleic acid) nucleotides. GNA includes GNA-A, GNA-T, GNA-C, GNA-G, and GNA-U. The structure of GNA-A is

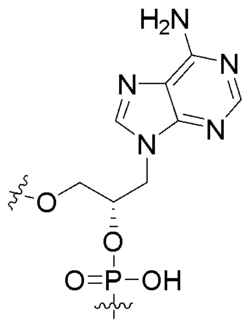

;

the structure of GNA-T is

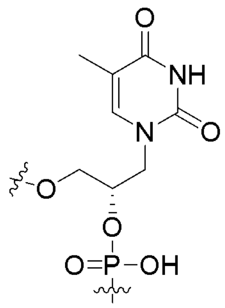

;

the structure of GNA-C is

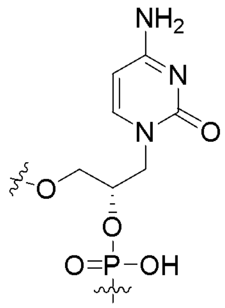

;

the structure of GNA-G is

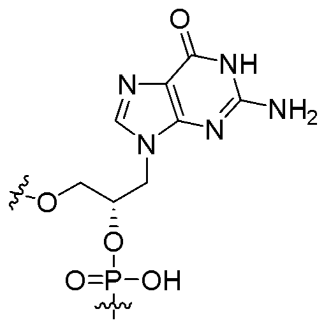

;

the structure of GNA-U is

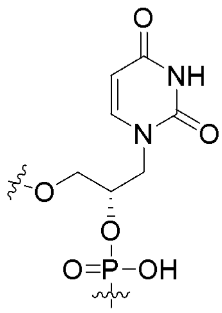

.

In some embodiments of the present disclosure, the modified nucleotide may also include one or more bicyclic sugar groups. "Bicyclic sugar" refers to a furanosyl ring modified by bridging two atoms. "Bicyclic nucleoside" ("BNA") refers to a nucleoside having a sugar group that contains a bridge connecting two carbon atoms of the sugar ring, thereby forming a bicyclic ring system. In certain embodiments, the bridge connects the 4'-carbon and the 2'-carbon of the sugar ring.

Unless otherwise specified, the "covalent linkage" described in the present disclosure includes, but is not limited to, "phosphate ester bonds" (*i.e.*, "phosphate diester bonds"), "phosphorothioate ester bonds", and other linkages.

The "modification" of nucleotides described in the present disclosure includes modifications to nucleobases, sugar rings, and internucleotide linkages, *etc.* Exemplarily, the "modification" of nucleotides described in the present disclosure includes, but is not limited to, methoxy modification, fluoro modification, (E)-vinyl phosphate ester modification, phosphorothioate ester linkage, or replacement of nucleotides with (S)-glycerol nucleic acid, *etc.*

In the present disclosure, the "m" before "R", "E", "A", "U", "G", and "C" indicates that the nucleotide is modified with 2'-O-methyl, while "f" means that the nucleotide is modified with 2'-fluoro.

Unless otherwise specified, the "R", "E", "A", "U", "G", and "C" described in the present disclosure are connected by phosphate ester groups. Exemplarily, the chemical structure of 5'-fAmRmC-3' is as follows:

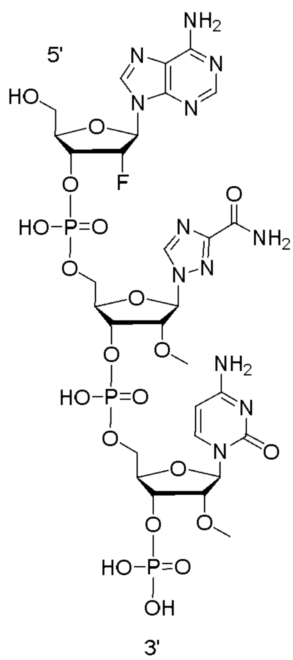

.

In the present disclosure, the "s" between "R", "E", "A", "U", "G", and "C" indicates that the nucleotides are connected via phosphorothioate ester groups.

In the present disclosure, the "MOE" before "R", "E" , "A", "U", "C", and "G" indicates that the nucleotide or substituted nucleotide group is modified with 2'-O-methoxyethyl.

In some embodiments of the present disclosure, when preceded by "m", the nucleotide or substituted nucleotide group denoted by "R", "E", "A", "U", "C", and "G" is modified with 2'-O-methyl (belonging to methoxy modification); when preceded by "f", it indicates modification with 2'-fluoro (belonging to fluoro modification); and when preceded by "MOE", it

indicates modification with 2'-O-methoxyethyl (belonging to MOE modification). In some embodiments of the present disclosure, when "s" appears between "R", "E", "A", "U", "C", and "G", it indicates that the nucleotides are linked by a phosphorothioate ester group. Exemplarily, the chemical structure of 5'-mRs$^{MOE}$GsmCmAfC-3' is as follows:

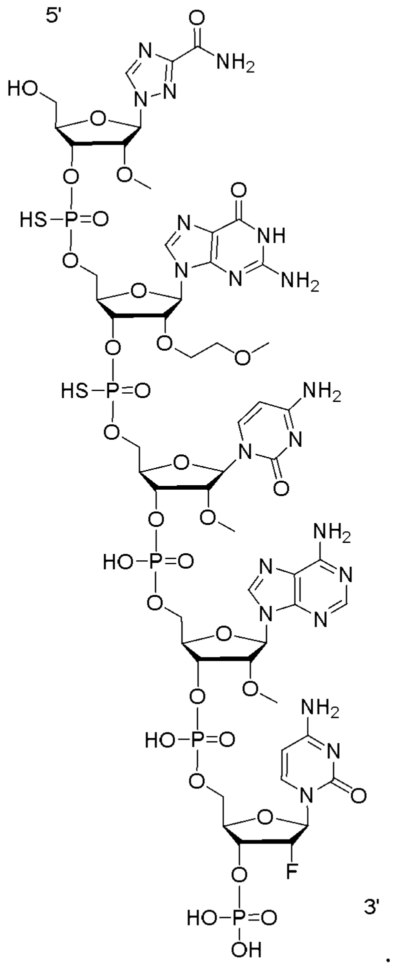

.

Unless otherwise specified, the term "more than one" or "multivalent" described in the present disclosure refers to an integer greater than or equal to 2, including but not limited to 2, 3, 4, 5, 6, 7, 8, 9, 10, 11, 12, 13, 14, 15, 16, 17, 18, 19, or 20, up to the theoretical maximum number of GalNAc derivatives that can be linked to the siRNA analog or the branched linkages.

The sense strand or antisense strand of the double-stranded siRNA analog described in the present disclosure may also include "overhangs", such as unpaired overhanging nucleotides that do not directly participate in the RNA double helix structure, wherein the RNA double helix structure is typically formed by the pairing of the "sense strand" and "antisense strand" as defined herein. Exemplarily, such overhangs may include one or more modified or unmodified U, T, and A.

The compounds of the present disclosure may exist in specific geometric or stereoisomeric forms. The present disclosure contemplates all such compounds, including (*R*)- and (*S*)-enantiomers, diastereoisomers, racemic mixtures, and other mixtures thereof, such as enantiomers or diastereomer enriched mixtures, all of which are within the scope of the present disclosure. Additional asymmetric carbon atoms may be present in substituents such as alkyl. All these isomers and their mixtures are included within the scope of the present disclosure.

Unless otherwise specified, the term "enantiomer" or "optical isomer" refers to stereoisomers that are mirror images of each other.

Unless otherwise specified, the term "diastereomer" refers to a stereoisomer in which a molecule has two or more chiral centers and the relationship between the molecules is not mirror images.

Unless otherwise specified, the absolute configuration of a stereogenic center is represented by a wedged solid bond (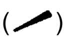) and a wedged dashed bond (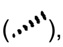), and the relative configuration of a stereogenic center is represented by a straight solid bond (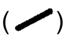) and a straight dashed bond (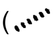), a wave line (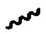) is used to represent a wedged solid bond (

) or a wedged dashed bond ( ), or the wave line ( ) is used to represent a straight solid bond ( ) and/or a straight dashed bond ( ).

Unless otherwise specified, when a group has one or more than one linkable site, any one or more than one site of the group can be linked to other groups through chemical bonds. The chemical bond between the site and other groups can be represented by a straight solid bond ( ), a straight dashed bond ( ), or a wavy line ( ).

Unless otherwise specified, the terms "enriched with one isomer", "isomers enriched", "enriched with one enantiomer" or " enantiomers enriched" refer to the content of one of the isomers or enantiomers is less than 100%, and the content of the isomer or enantiomer is greater than or equal to 60%, or greater than or equal to 70%, or greater than or equal to 80%, or greater than or equal to 90%, or greater than or equal to 95%, or greater than or equal to 96%, or greater than or equal to 97%, or greater than or equal to 98%, or greater than or equal to 99%, or greater than or equal to 99.5%, or greater than or equal to 99.6%, or greater than or equal to 99.7%, or greater than or equal to 99.8%, or greater than or equal to 99.9%.

Unless otherwise specified, the term "isomeric excess" or "enantiomeric excess" refers to the difference between the relative percentages of two isomers or two enantiomers. For example, if the content of one isomer or enantiomer is 90%, and the content of the other isomer or enantiomer is 10%, the isomeric or enantiomeric excess (ee value) is 80%.

Optically active (*R*)- and (*S*)-isomers, or *D* and *L* isomers can be prepared using chiral synthesis, chiral reagents, or other conventional techniques. If one kind of enantiomer of certain compound of the present disclosure is to be obtained, it can be obtained by asymmetric synthesis or derivative action of chiral auxiliary, wherein the resulting diastereomeric mixture is separated and the auxiliary group is cleaved to provide the pure desired enantiomer. Alternatively, when the molecule contains a basic functional group (such as amino) or an acidic functional group (such as carboxyl), a salt of a diastereoisomer is formed with an appropriate optically active acid or base, and then diastereomeric resolution is performed by conventional methods known in the art, and then the pure enantiomer is recovered. In addition, the enantiomer and the diastereoisomer are generally separated through chromatography which uses a chiral stationary phase and optionally combines with a chemical derivative method (such as generating carbamate from amine). The compound of the present disclosure may contain an unnatural proportion of atomic isotope at one or more than one atom that constitutes the compound. For example, the compound can be radiolabeled with a radioactive isotope, such as tritium ($^{3}H$), iodine-125 ($^{125}I$), or C-14 ($^{14}C$). For another example, deuterated drugs can be formed by replacing hydrogen with deuterium, the bond formed by deuterium and carbon is stronger than that of ordinary hydrogen and carbon, compared with non-deuterated drugs, deuterated drugs have the advantages of reduced toxic and side effects, increased drug stability, enhanced efficacy, extended biological half-life of drugs, *etc.* All isotopic variations of the compound of the present disclosure, whether radioactive or not, are encompassed within the scope of the present disclosure.

The term "salt" refers to a salt of the compound of the present disclosure that is prepared by reacting the compound having a specific substituent of the present disclosure with a relatively non-toxic acid or base. When the compound of the present disclosure contains a relatively acidic functional group, a base addition salt can be obtained by contacting such compounds with a sufficient amount of base in a pure solution or a suitable inert solvent. The pharmaceutically acceptable base addition salt includes a salt of sodium, potassium, calcium, ammonium, organic amine, magnesium, or similar salts. When the compound of the present disclosure contains a relatively basic functional group, an acid addition salt can be obtained by contacting the compound with a sufficient amount of acid in a pure solution or a suitable inert solvent. Examples of the pharmaceutically acceptable acid addition salt include an inorganic acid salt, wherein the inorganic acid includes, for example, hydrochloric acid, hydrobromic acid, nitric acid, carbonic acid, bicarbonate, phosphoric acid, monohydrogen phosphate, dihydrogen phosphate, sulfuric acid, hydrogen sulfate, hydroiodic acid, phosphorous acid; and an organic acid salt, wherein the organic acid includes, for example, acetic acid, propionic acid, isobutyric acid, maleic acid, malonic acid, benzoic acid, succinic acid, suberic acid, fumaric acid, lactic acid, mandelic acid, phthalic acid, benzenesulfonic acid, *p*-toluenesulfonic acid, citric acid, tartaric acid, and methanesulfonic acid; and salts of amino acid (such as arginine), and a salt of an organic acid such as glucuronic acid. Certain specific compounds of the present disclosure contain both basic and acidic functional groups, thus can be converted to any base or acid addition salt.

The salt of the present disclosure can be prepared from the parent compound that contains an acidic or basic group by conventional chemical methods. Generally, such salt can be prepared by reacting the free acid or base form of the compound with a stoichiometric amount of an appropriate base or acid in water or an organic solvent or a mixture thereof.

The compounds of the present disclosure can be prepared by a variety of synthetic methods known to those skilled in the art, including the specific embodiments listed below, the embodiments formed by their combination with other chemical synthesis methods, and equivalent alternatives known to those skilled in the art, and preferred embodiments include, but are not limited to, the examples of the present disclosure.

The structure of the compounds of the present disclosure can be confirmed by conventional methods known to those skilled in the art, and if the present disclosure involves an absolute configuration of a compound, then the absolute

configuration can be confirmed by means of conventional techniques in the art. For example, in the case of single crystal X-ray diffraction (SXRD), diffraction intensity data are collected from the cultured single crystal using a Bruker D8 venture diffractometer with CuKα radiation as the light source and scanning mode: φ/ω scan, and after collecting the relevant data, the crystal structure is further analyzed by direct method (Shelxs97), so that the absolute configuration can be confirmed.

The solvents used in the present disclosure are commercially available.

Unless otherwise specified, the solvent ratios used in column chromatography and preparative thin-layer silica gel chromatography in the present disclosure are all by volume.

**Table 4. List of abbreviations used in the present disclosure**

| | |
|---|---|
| Ac | Acetyl |
| Boc | *tert*-Butoxycarbonyl |
| Ms | Methanesulfonyl |
| Ts | *p*-Toluenesulfonyl |
| TsCl | *p*-Toluenesulfonyl chloride |
| Fomc | Fluorenylmethoxycarbonyl |
| HATU | O-(7-Azabenzotriazol-1-yl)-*N*,*N*,*N'*,*N'*-tetramethyluronium hexafluorophosphate |
| HOAt | 1-Hydroxy-7-azabenzotriazole |
| DMTr | 4,4'-Dimethoxytrityl |
| DMTr-Cl | 4,4'-Dimethoxytrityl chloride |
| DMSO | Dimethyl sulfoxide |
| $EC_{50}$ | Half-maximal effective concentration |
| $CC_{50}$ | Half-maximal cytotoxic concentration |
| *t*Bu | *tert*-Butyl |
| Ph | Phenyl |
| pre-HPLC | Preparative high-performance liquid chromatography (for compound purification) |
| pre-TLC | Preparative thin-layer chromatography (for compound purification) |
| RNA | Ribonucleic acid |
| RNAi | Ribonucleic acid interference |
| dsRNA | Double-stranded ribonucleic acid |
| pgRNA | Pregenomic ribonucleic acid |
| siRNA | Small interfering ribonucleic acid |
| HBsAg | Hepatitis B virus surface antigen |
| HBeAg | Hepatitis B virus e antigen |
| GalNAc group | 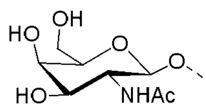 |

The compounds of the present disclosure are named according to the conventional naming principles in the art or by ChemDraw® software, and the commercially available compounds use the supplier catalog names.

DETAILED DESCRIPTION OF THE PREFERRED EMBODIMENT

The present disclosure is described in detail by the examples below, but it does not mean that there are any adverse restrictions on the present disclosure. The compounds of the present disclosure can be prepared by a variety of synthetic methods known to those skilled in the art, including the specific embodiments listed below, the embodiments formed by their combination with other chemical synthesis methods, and equivalent alternatives known to those skilled in the art, and preferred embodiments include, but are not limited to, the examples of the present disclosure. It will be

apparent to those skilled in the art that various variations and improvements can be made to specific embodiments of the present disclosure without departing from the spirit and scope of the present disclosure.

**Example 1: Synthesis of Compound 1**

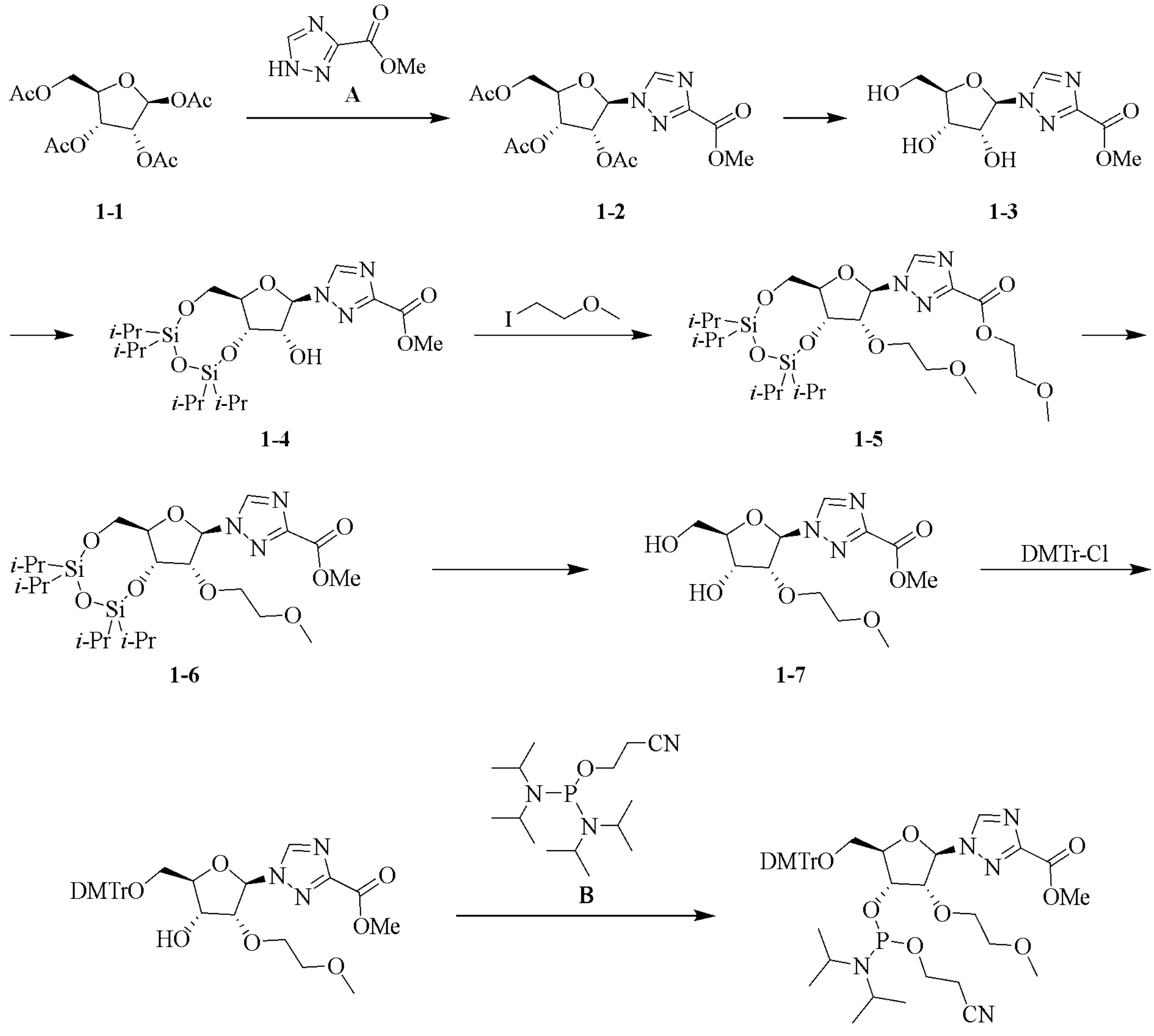


1-1 1-2 1-3

1-4 1-5

1-6 1-7

1-8 1

Step A: Compound 1-1 (30.00 g, 94.26 mmol) and compound A (11.98 g, 94.26 mmol) were dissolved in methyl acetate (220 mL). The mixture was then concentrated to near dryness at 90°C under 1 bar pressure, followed by the addition of a solution of trifluoromethanesulfonic acid (141.46 mg, 0.94 mmol) in methyl acetate (2 mL). After the addition was completed, the reaction mixture was stirred at 125°C under 30 mbar pressure for 4 hours. The reaction mixture was cooled to 70°C, and ethanol (70 mL) was added. The mixture was stirred at 70°C until a homogeneous solution was formed, after which stirring was stopped and the mixture was cooled to 50°C. The mixture was allowed to stand and cool naturally to 25°C, then further placed at 0°C and allowed to stand for 16 hours. The mixture was then filtered, and the filter cake was washed with ethanol (180 mL, 60 mL × 3). The filter cake was collected and dried under vacuum to obtain compound **1-2.** $^1$H NMR (400 MHz, $CDCl_3$): $\delta$ = 8.40 (s, 1H), 6.04 (d, $J$ = 3.42 Hz, 1H), 5.81 - 5.69 (m, 1H), 5.54 (t, $J$ = 5.38 Hz, 1H), 4.51 - 4.42 (m, 2H), 4.30 - 4.16 (m, 1H), 3.98 (s, 3H), 2.18 - 2.05 (m, 9H).

Step B: Compound **1-2** (15.00 g, 38.93 mmol) and triethylamine (4.14 g, 40.87 mmol) were dissolved in methanol (100 mL). The mixture was stirred under a nitrogen atmosphere at 50°C for 17 hours. The reaction mixture was concentrated under reduced pressure to obtain compound **1-3.** $^1$H NMR (400 MHz, $CD_3OD$): $\delta$ = 8.87 (s, 1H), 5.93 (d, $J$ = 3.42 Hz, 1H), 4.48 (dd, $J$ = 3.48, 4.83 Hz, 1H), 4.33 (t, $J$ = 5.26 Hz, 1H), 4.16 - 4.10 (m, 1H), 3.95 (s, 3H), 3.84 (dd, $J$ =

3.24, 12.29 Hz, 1H), 3.70 (dd, $J$ = 4.46, 12.29 Hz, 1H).

Step C: Compound **1-3** (10.00 g, 38.58 mmol) was dissolved in pyridine (250 mL), cooled to 0°C, and 1,3-dichloro-1,1,3,3-tetraisopropyldisiloxane (12.29 g, 38.97 mmol) was added dropwise. After the addition was completed, the reaction mixture was naturally warmed to 25°C and stirred for 16 hours. The reaction mixture was concentrated under reduced pressure, and the concentrate was thoroughly suspended in ethyl acetate (250 mL). The reaction mixture was filtered, and the filtrate was washed three times with 3 mol/L hydrochloric acid (250 mL × 3), and then washed once with 250 mL of saturated brine. The organic phase was dried over anhydrous sodium sulfate, filtered under reduced pressure, and the filtrate was concentrated under reduced pressure. The residue was purified by silica gel column chromatography (eluent: V/V/V petroleum ether/dichloromethane/ethyl acetate = 3/1/1) to obtain compound **1-4.** $^1$H NMR (400MHz, $CDCl_3$): $\delta$ = 8.43 (s, 1H), 5.95 (s, 1H), 4.73 (dd, $J$ = 4.75, 8.00 Hz, 1H), 4.41 (d, $J$ = 4.75 Hz, 1H), 4.19 - 4.09 (m, 2H), 4.03 - 3.94 (m, 4H), 3.34 - 2.71 (m, 1H), 1.15 - 1.01 (m, 28H).

Step D: Compound **1-4** (20.00 g, 39.86 mmol) was dissolved in anhydrous toluene (200 mL), and then silver oxide (92.38 g, 398.63 mmol) and 2-iodoethyl methyl ether (22.24 g, 119.56 mmol) were added sequentially. The reaction mixture was stirred at 130°C for 24 hours. After cooling naturally to room temperature, the reaction mixture was filtered, and the filtrate was concentrated under reduced pressure to obtain crude compound **1-5**, which was used directly in the next step. LC-MS (ESI) m/z: 604.3 $[M+H]^+$.

Step E: Compound **1-5** (20.00 g, 33.12 mmol) was dissolved in anhydrous methanol (200 mL), and then triethylamine (3.38 g, 33.45 mmol) was added. The reaction mixture was stirred at 25°C for 12 hours. After cooling naturally to room temperature, the reaction mixture was concentrated under reduced pressure to obtain crude compound **1-6**, which was directly used in the next step. LC-MS (ESI) m/z: 560.3 $[M+H]^+$.

Step F: At 0°C, compound **1-6** (18.50 g, 33.12 mmol) was dissolved in anhydrous tetrahydrofuran (185 mL), and then triethylamine trihydrofluoride (11.72 g, 72.70 mmol) was added. The reaction mixture was stirred at 25°C for 12 hours. After cooling naturally to room temperature, the reaction mixture was concentrated under reduced pressure. The residue was purified by silica gel column chromatography (eluent: V/V dichloromethane/methanol = 40/1 to 10/1) to obtain compound **1-7.** $^1$H NMR (400 MHz, $CD_3OD$) $\delta$ = 8.87 (s, 1H), 6.04 (d, $J$ = 3.3 Hz, 1H), 4.45 - 4.36 (m, 2H), 4.15 - 4.09 (m, 1H), 3.96 (s, 3H), 3.87 - 3.67 (m, 4H), 3.59 - 3.54 (m, 2H), 3.34 (s, 3H).

Step G: At 0°C, compound **1-7** (6.00 g, 18.91 mmol) was dissolved in anhydrous pyridine (20 mL), and then DMTr-Cl (7.69 g, 22.69 mmol) was added. The reaction mixture was naturally warmed and stirred for 12 hours at 20 to 25°C. After concentration under reduced pressure, the residue was purified by silica gel column chromatography (eluent: V/V petroleum ether/ethyl acetate = 30/1 to 0/1, containing 0.2% triethylamine) to obtain compound **1-8.** $^1$H NMR (400 MHz, DMSO-$d_6$) $\delta$ = 8.95 (s, 1H), 7.33 (d, $J$ = 7.4 Hz, 2H), 7.26 - 7.17 (m, 7H), 6.82 (t, $J$ = 7.9 Hz, 4H), 6.12 (d, $J$ = 1.8 Hz, 1H), 5.23 (d, $J$ = 6.1 Hz, 1H), 4.43 -4.29 (m, 2H), 4.12 - 4.03 (m, 1H), 3.83 (s, 3H), 3.79 - 3.75 (m, 1H), 3.72 (s, 6H), 3.67 (brs, 1H), 3.48 - 3.42 (m, 2H), 3.20 (s, 3H), 3.16 - 3.08 (m, 2H).

Step H: At 0°C, compound **1-8** (1.10 g, 1.77 mmol) was dissolved in anhydrous dichloromethane (8 mL), and then compound **B** (0.62 g, 2.61 mmol) and 4,5-dicyanoimidazole (0.10 g, 0.89 mmol) were added. After the addition was completed, the reaction mixture was stirred at 20°C for 0.5 hours. The reaction mixture was concentrated under reduced pressure, and the residue was purified by silica gel column chromatography (eluent: V/V petroleum ether/ethyl acetate = 50/1 to 1/2) to obtain compound 1. LCMS (ESI) m/z: 820.3 $[M+H]^+$.

## Example 2: Synthesis of Compound 2

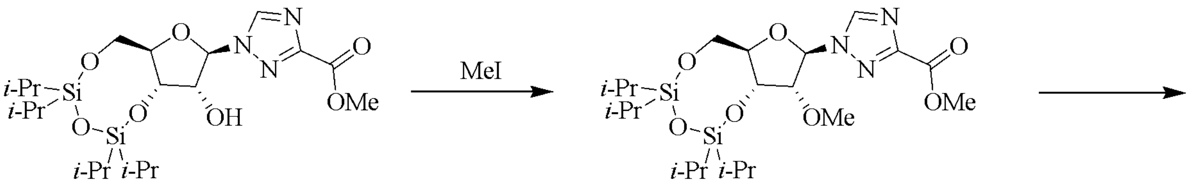


1-4 1-9

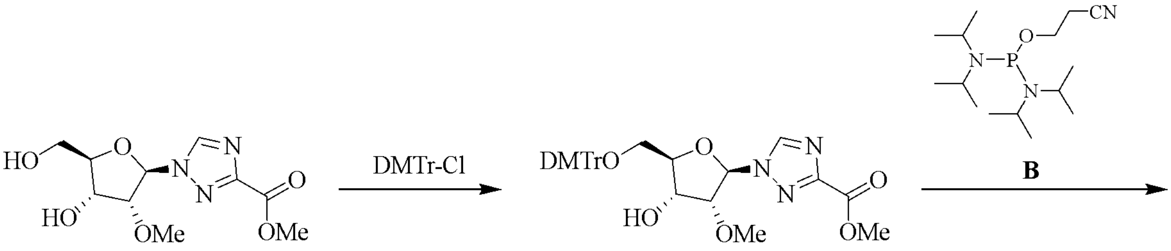


1-10 1-11

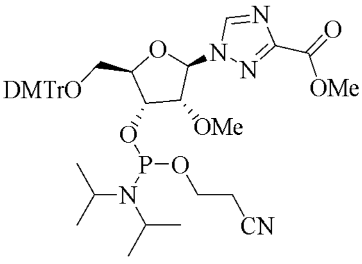

2

Step A: At room temperature, **1-4** (8.23 g, 16.40 mmol), potassium carbonate (11.34 g, 82.02 mmol), and silver oxide (19.01 g, 82.02 mmol) were added sequentially to *N,N*-dimethylformamide (50 mL), and then iodomethane (11.64 g, 82.02 mmol) was added. The reaction mixture was stirred at room temperature for 3 hours, then diluted with ethyl acetate (300 mL) and filtered. The filtrate was washed once with saturated sodium thiosulfate aqueous solution (250 mL), water (250 mL), and saturated brine (250 mL), respectively, dried over anhydrous sodium sulfate, filtered, and the filtrate was concentrated under reduced pressure. The residue was purified by silica gel column chromatography (eluent: V/V petroleum ether/ethyl acetate = 5/1) to obtain **1-9.** $^1H$ NMR (400 MHz, $CDCl_3$): $\delta$ = 8.58 (s, 1H), 5.91 (s, 1H), 4.46 (dd, *J* = 4.22, 9.35 Hz, 1H), 4.28 - 4.17 (m, 2H), 4.06 - 3.96 (m, 5H), 3.68 (s, 3H), 1.13 - 0.99 (m, 28H).

Step B: **1-9** (3.27 g, 6.34 mmol) was added to tetrahydrofuran (50 mL) at 0°C and stirred to dissolve, and then triethylamine trihydrofluoride (2.25 g, 13.95 mmol) was added dropwise. After the addition was completed, the reaction mixture was naturally warmed to room temperature and stirred for 16 hours. The reaction mixture was concentrated under reduced pressure, and the residue was purified by silica gel column chromatography (eluent: V/V dichloromethane/-methanol = 20/1) to obtain **1-10.** $^1H$ NMR (400 MHz, $CD_3OD$): $\delta$ = 8.88 (s, 1H), 6.04 (d, *J* = 3.26 Hz, 1H), 4.44 (t, *J* = 5.33 Hz, 1H), 4.20 (dd, *J* = 3.33, 4.83 Hz, 1H), 4.14 - 4.07 (m, 1H), 3.96 (s, 3H), 3.84 (dd, *J* = 3.20, 12.36 Hz, 1H), 3.69 (dd, *J* = 4.39, 12.30 Hz, 1H), 3.52 (s, 3H).

Step C: At 0°C, **1-10** (1.30 g, 4.76 mmol) was added to anhydrous pyridine (20 mL). After stirring to dissolve, DMTr-Cl (2.42 g, 7.14 mmol) was added. After the addition was completed, the reaction mixture was stirred at room temperature for 16 hours. The reaction mixture was diluted with ethyl acetate (70 mL), and then saturated sodium bicarbonate aqueous solution (20 mL) and water (40 mL) were added. After thorough stirring, the mixture was allowed to stand for phase separation. The organic phase was washed once each with water (60 mL) and saturated brine (60 mL), dried over anhydrous sodium sulfate, filtered, and the filtrate was concentrated under reduced pressure. The residue was purified by preparative HPLC (separation column: Phenomenex luna C18 (specifications: 250 mm × 50 mm, particle size: 10 μm); mobile phase: phase A: 10 mM ammonium bicarbonate aqueous solution; phase B: acetonitrile; elution gradient: 35% to 65%, 20 minutes) to obtain **1-11.** $^1H$ NMR (400 MHz, $CDCl_3$): $\delta$ = 8.44 (s, 1H), 7.45 - 7.38 (m, 2H), 7.34 - 7.28 (m, 5H), 7.27 - 7.18 (m, 2H), 6.92 - 6.70 (m, 4H), 5.97 (d, *J* = 2.88 Hz, 1H), 4.43 - 4.37 (m, 1H), 4.33 (dd, *J* = 2.88, 5.00 Hz, 1H), 4.25 - 4.19 (m, 1H), 3.98 (s, 3H), 3.80 (s, 6H), 3.58 (s, 3H), 3.49 - 3.43 (m, 1H), 3.40 - 3.33 (m, 1H), 2.55 (d, *J* = 6.88 Hz, 1H). LCMS (ESI) m/z: 574.2 $[M-H]^-$.

Step D: At 0°C, **1-11** (1.10 g, 1.91 mmol) was added to anhydrous dichloromethane (8 mL), and then compound **B**

(678.45 mg, 2.87 mmol) and 4,5-dicyanoimidazole (0.11 g, 0.96 mmol) were added. After the addition was completed, the reaction mixture was stirred at room temperature for 0.5 hours under a nitrogen atmosphere. After concentration under reduced pressure, the residue was purified by silica gel column chromatography (eluent: V/V petroleum ether/ethyl acetate = 50/1 to 1/2) to obtain compound 2. LCMS (ESI) m/z: 776.3 [M+H]$^+$.

**Example 3: Synthesis of Compound 3**

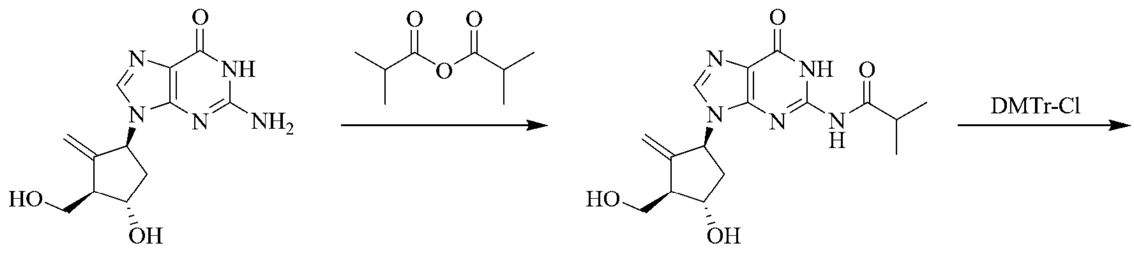


1-12 1-13

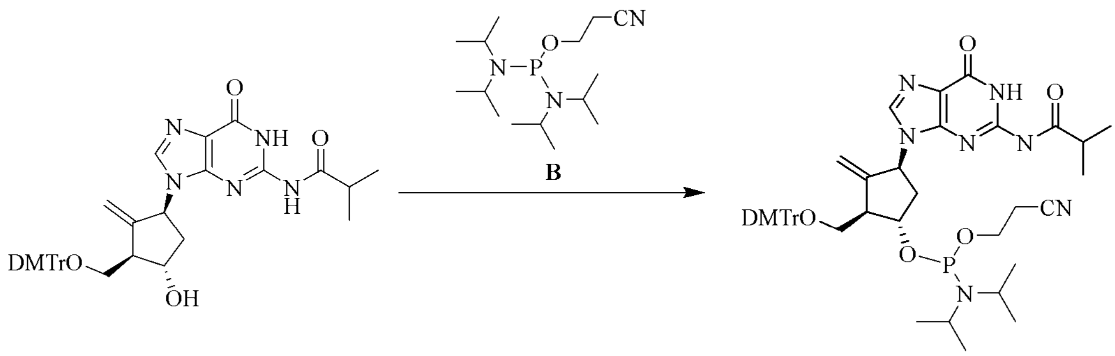


1-14 3

Step A: **1-12** (10 g, 36.06 mmol) was dissolved in anhydrous pyridine (200 mL), and then trimethylchlorosilane (48.98 g, 450.81 mmol) was added. The reaction mixture was stirred at 25°C for 2 hours, then isobutyric anhydride (71.32 g, 450.81 mmol) was added, and stirring was continued at 25°C for 12 hours. The reaction mixture was cooled, and 28% aqueous ammonia (109.37 g, 873.85 mmol) was slowly added dropwise at 0°C. After the dropwise addition was completed, stirring was continued at 0°C for 15 minutes. The mixture was filtered, and the filtrate was concentrated under reduced pressure to obtain a crude product. The crude product was purified by silica gel column chromatography (eluent: V/V dichloromethane/methanol = 1/0 to 30/1, containing 0.2% triethylamine) and preparative HPLC (chromatographic column: Kromasil Eternity XT 250 × 80 mm × 10 μm; mobile phase: phase A: 0.05% aqueous ammonia; phase B: 5% to 35% acetonitrile; 20 minutes) to obtain **1-13.** $^1$H NMR (400 MHz, DMSO-$d_6$) $\delta$ = 7.97 (s, 1H), 5.47 (dd, $J$ = 7.9, 10.3 Hz, 1H), 5.13 (br s, 1H), 4.58 (t, $J$ = 2.1 Hz, 1H), 4.33 - 4.21 (m, 1H), 3.55 (d, $J$ = 6.6 Hz, 2H), 2.84 - 2.69 (m, 1H), 2.58 - 2.52 (m, 1H), 2.43 - 2.33 (m, 1H), 2.28 (br s, 1H), 2.07 (s, 1H), 1.11 (d, $J$ = 6.8 Hz, 6H). LC-MS (ESI) m/z: 348.1 [M+H]$^+$.

Step B: **1-13** (11.9 g, 34.26 mmol) was dissolved in anhydrous pyridine (120 mL), and DMTr-Cl (13.93 g, 41.11 mmol) was added at 0°C. The reaction mixture was stirred at 20°C for 12 hours. After filtration, the filtrate was concentrated under reduced pressure to obtain a crude product. The crude product was purified by silica gel column chromatography (eluent: V/V dichloromethane/methanol = 1/0 to 30/1, containing 0.2% triethylamine) and preparative HPLC (chromatographic column: Kromasil Eternity XT 250 × 80 mm × 10 μm; mobile phase: phase A: 0.05% aqueous ammonia; phase B: 35% to 65% acetonitrile; 21 minutes) to obtain compound **1-14.** $^1$H NMR (400 MHz, DMSO-$d_6$) $\delta$ = 12.27 - 11.39 (m, 2H), 7.74 (s, 1H), 7.43 - 7.22 (m, 9H), 6.90 (d, $J$ = 8.6 Hz, 4H), 5.46 (t, $J$ = 8.8 Hz, 1H), 5.11 - 4.89 (m, 2H), 4.56 (br s, 1H), 4.22 (br s, 1H), 3.74 (s, 6H), 3.22 (d, $J$ = 6.4 Hz, 2H), 2.74 (s, 1H), 2.63 (br s, 1H), 2.24 - 2.05 (m, 2H), 1.11 (d, $J$ = 6.8Hz, 6H). LC-MS (ESI) m/z: 650.3 [M+H]$^+$.

Step C: Compound **1-14** was azeotropically dried with anhydrous acetonitrile three times for subsequent use. Under a nitrogen atmosphere at 0°C, compound **1-14** (5.10 g, 7.85 mmol) was dissolved in anhydrous dichloromethane (50 mL), and then compound **B** (3.55 g, 11.8 mmol, 3.74 mL) and 4,5-dicyanoimidazole (1.21 g, 10.2 mmol) were added. After the addition was completed, the reaction was stirred at 20°C for 2 hours, then diluted with dichloromethane (20 mL), and saturated sodium bicarbonate aqueous solution (20 mL) was added. The organic phase was separated by extraction,

washed with saturated brine, dried over anhydrous sodium sulfate, and filtered. The filtrate was concentrated, and the residue was purified by silica gel column chromatography (eluent: V/V petroleum ether/ethyl acetate = 3/1 to 1/1) to obtain compound **3.** $^1$H NMR (400 MHz, $CD_3CN$) $\delta$ = 7.55 (d, $J$ = 12.0 Hz, 1H), 7.51 - 7.44 (m, 2H), 7.39 - 7.19 (m, 7H), 6.91 - 6.80 (m, 4H), 5.43 - 5.37 (m, 1H), 5.05 (m, 1H), 4.68 (m, 1H), 4.53 (m, 1H), 3.78 (m, 1H), 3.76 (s, 6H), 3.75 - 3.43 (m, 3H), 3.37 - 3.26 (m, 2H), 2.87 (m, 1H), 2.68 - 2.55 (m, 2H), 2.54 - 2.25 (m, 3H), 1.21 - 1.09 (m, 18H). **$^{31}$P NMR** (162 MHz, $CD_3CN$) $\delta$ = -147.46 (s, 1P), -146.79 (s, 1P).

**Example 4: Synthesis of D01**

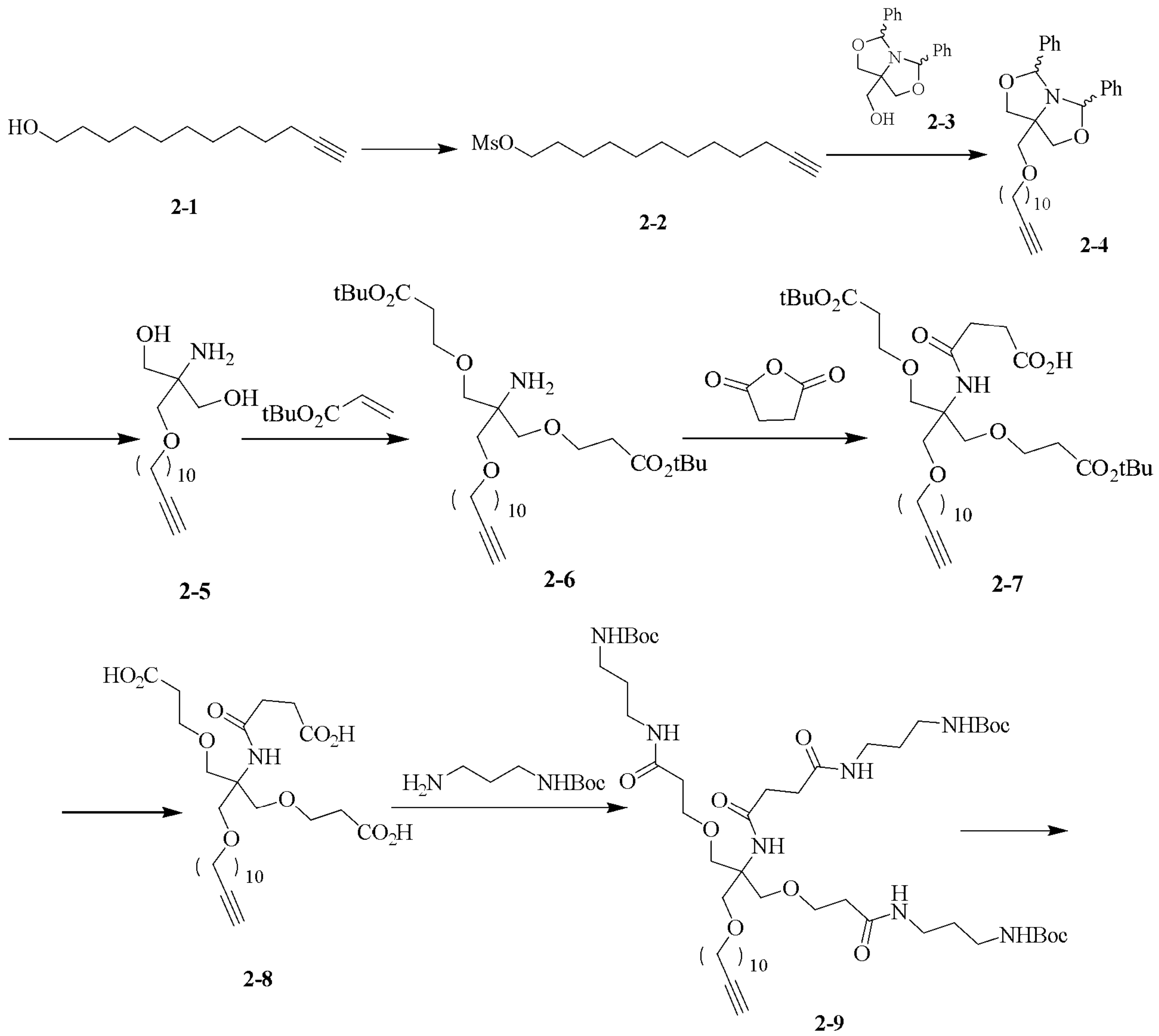

2-1 2-2 2-3 2-4

2-5 2-6 2-7

2-8 2-9

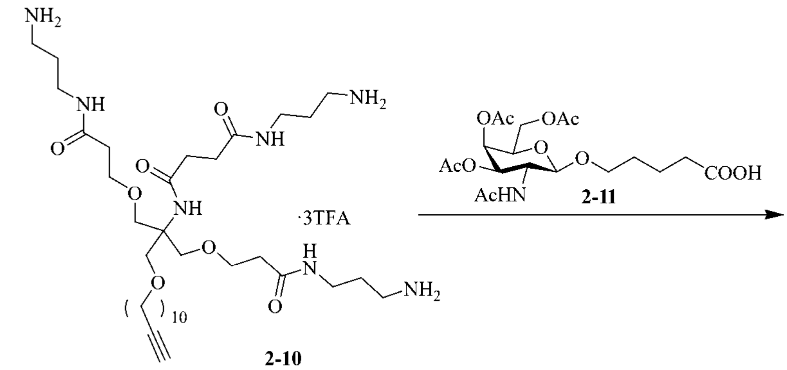

2-11

2-10

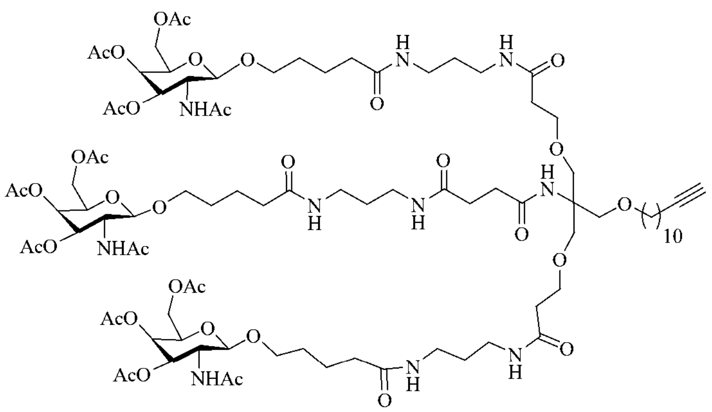

2-12

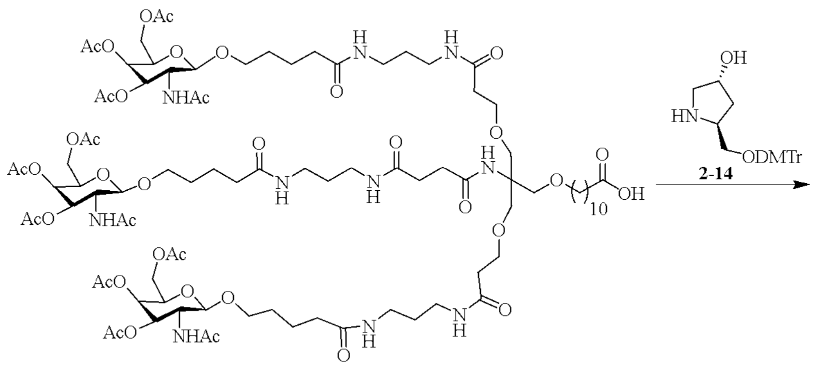

2-14

2-13

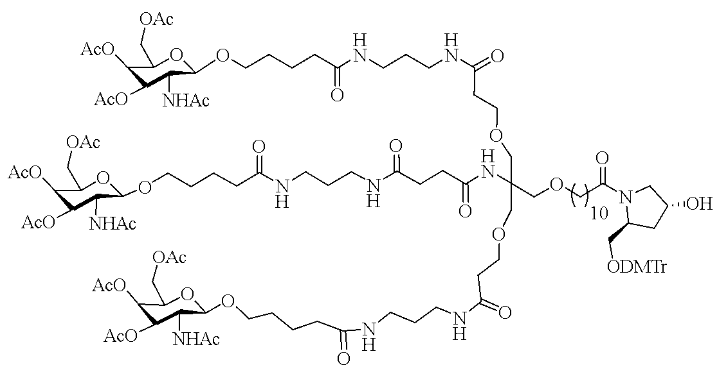

2-15

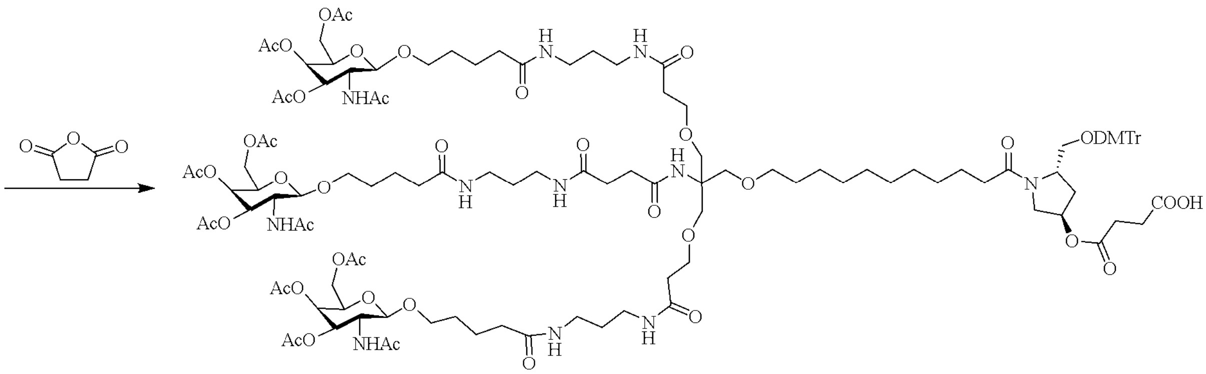

D01

Step A: 11-Dodecyn-1-ol (**2-1**, 25 g, 137.14 mmol) and triethylamine (16.65 g, 164.56 mmol) were dissolved in dichloromethane (250 mL), and methanesulfonyl chloride (18.85 g, 164.56 mmol) was added at 0°C. The mixture was stirred at 0°C for 2 hours. The reaction mixture was diluted with water (400 mL), then extracted with dichloromethane (800 mL, 400 mL × 2). The combined organic phases were washed with water (400 mL, 200 mL × 2) and saturated brine, dried over anhydrous sodium sulfate, filtered, and concentrated under reduced pressure to obtain **2-2.**

Step B: **2-3** (20 g, 67.26 mmol) was dissolved in N,N-dimethylformamide (200 mL), and sodium hydride (w/w = 60%, 4.04 g, 100.89 mmol) was added at 0°C, and then **2-2** (19.27 g, 73.99 mmol) was added. The reaction mixture was stirred at 25°C for 16 hours, quenched with water (1 L), and extracted with dichloromethane (1.6 L, 800 mL × 2). The combined organic phases were washed once with saturated brine (800 mL), dried over anhydrous sodium sulfate, filtered, and concentrated under reduced pressure to obtain **2-4.** $^1$H NMR (400 MHz, DMSO-$d_6$): $\delta$ = 7.63 - 6.89 (m, 10H), 5.64 - 5.52 (m,2H), 4.27 - 4.01 (m, 2H), 3.98 - 3.77 (m, 2H), 3.72 - 3.18 (m, 4H), 2.23 - 2.14 (m, 2H), 1.98 - 1.92 (m, 1H), 1.54 - 1.23 (m, 16H).

Step C: **2-4** (48 g, 103.98 mmol) was dissolved in methanol (870 mL), and then a solution of hydrogen chloride in methanol (4 mol/L, 400 mL) was added. The reaction mixture was stirred at 30°C for 2 hours, then an additional solution of hydrogen chloride in methanol (4 mol/L, 350 mL) was added. The reaction mixture was stirred at 30°C for another 16 hours. After the reaction mixture was concentrated under reduced pressure, 200 mL of chloroform was added, and the mixture was concentrated under reduced pressure until white solids appeared. Toluene (130 mL) and petroleum ether (130 mL) were added to the residue, and the mixture was stirred at 15°C for 16 hours. The mixture was then filtered through a Buchner funnel, and the filter cake was collected and dried under vacuum to obtain a white solid. The white solid was dissolved in dichloromethane (50 mL), and an aqueous solution (50 mL) of sodium hydroxide (6.59 g, 164.66 mmol) was added. The mixture was stirred at 20°C for 1 hour, then diluted with water (500 mL) and extracted with 1 L of dichloromethane (500 mL × 2). The combined organic phases were dried over anhydrous sodium sulfate, filtered, and the filtrate was concentrated under reduced pressure to obtain **2-5.**

Step D: **2-5** (23 g, 80.58 mmol) and sodium hydroxide (322.31 mg, 8.06 mmol) were added to a mixture of dimethyl sulfoxide (70 mL) and water (6 mL), and then tert-butyl acrylate (22.72 g, 177.28 mmol) was added. The reaction mixture was stirred at 25°C under a nitrogen atmosphere for 16 hours, then diluted with water (500 mL) and extracted with ethyl acetate (1 L, 500 mL × 2). The combined organic phases were dried over anhydrous sodium sulfate, filtered, and the filtrate was concentrated under reduced pressure. The residue was purified by silica gel column chromatography (eluent: V/V/V petroleum ether/ethyl acetate/ethanol (containing 0.1% aqueous ammonia) = 36/3/1 to 16/3/1) to obtain **2-6.** $^1$H NMR (400 MHz, DMSO-$d_6$): $\delta$ = 3.60 - 3.54 (m, 4H), 3.32 (br s, 5H), 3.15 (s, 5H), 2.74 - 2.66 (m, 1H), 2.40 (t, $J$ = 6.0 Hz, 4H), 2.18 - 2.11 (m, 2H), 1.58 - 1.38 (m, 22H), 1.34 - 1.23 (m, 12H).

Step E: To a solution of **2-6** (24.5 g, 45.22 mmol) in dichloromethane (250 mL) were added triethylamine (9.15 g, 90.45 mmol) and succinic anhydride (6.79 g, 67.83 mmol), and the mixture was stirred at 20°C for 16 hours. The reaction mixture was added with dichloromethane (1 L) and hydrochloric acid (1 mol/L, 1 L), stirred, and allowed to stand for phase separation. The organic phase was dried over anhydrous sodium sulfate, filtered, and the filtrate was concentrated under reduced pressure to obtain **2-7.** $^1$H NMR (400 MHz, $CDCl_3$): $\delta$ = 6.49 - 6.37 (m, 1H), 3.72 (s, 2H), 3.70 - 3.57 (m, 8H), 3.37 (t, $J$ = 6.7 Hz, 2H), 2.69 - 2.51 (m, 4H), 2.50 - 2.36 (m, 4H), 2.22 - 2.13 (m, 2H), 1.96 - 1.90 (m, 1H), 1.57 - 1.47 (m, 4H), 1.46 - 1.40 (m, 18H), 1.40 - 1.31 (m, 2H), 1.30 - 1.21 (m, 10H).

Step F: **2-7** (27.4 g, 42.69 mmol) was dissolved in formic acid (140 mL). The solution was stirred at 20°C for 16 hours under a nitrogen atmosphere. After concentration under reduced pressure, toluene (150 mL) was added to the residue, and the mixture was concentrated to dryness under reduced pressure; toluene (150 mL) was added again, and the mixture was concentrated to dryness under reduced pressure to obtain **2-8.** $^1$H NMR (400 MHz, $CDCl_3$): $\delta$ = 9.79 - 9.22 (m, 3H), 6.44 - 6.23 (m, 1H), 3.88 - 3.43 (m, 10H), 3.39 - 3.20 (m, 2H), 2.77 - 2.31 (m, 8H), 2.15 - 2.06 (m, 2H), 1.87 (t, $J$ = 2.6 Hz, 1H), 1.48 - 1.28 (m, 6H), 1.26 - 1.12 (m, 10H).

Step G: **2-8** (22.6 g, 42.67 mmol), *N,N*-diisopropylethylamine (33.09 g, 256.03 mmol), and HATU (51.92 g, 136.55 mmol) were dissolved in *N,N*-dimethylformamide (250 mL), and then *tert*-butyl (3-aminopropyl)carbamate (29.74 g, 170.69 mmol) was added. The reaction mixture was stirred at 20°C for 16 hours, then dichloromethane (1 L) and hydrochloric acid (1 mol/L, 1 L) were added, stirred, and allowed to stand for phase separation. The organic phase was sequentially washed with water (1 L), saturated sodium bicarbonate aqueous solution (1 L), and saturated brine (1 L), dried over anhydrous sodium sulfate, filtered, and the filtrate was concentrated under reduced pressure. The residue was purified by silica gel column chromatography (eluent: V/V/V petroleum ether/ethyl acetate/ethanol = 40/3/1 to 10/3/1) to obtain **2-9.** $^1$H NMR (400 MHz, $CDCl_3$): $\delta$ = 7.22 - 6.79 (m, 3H), 6.77 - 6.44 (m, 1H), 5.45 - 5.00 (m, 3H), 3.86 - 3.73 (m, 2H), 3.72 - 3.63 (m, 4H), 3.62 - 3.45 (m, 4H), 3.41 - 3.32 (m, 2H), 3.32 - 3.20 (m, 6H), 3.19 - 3.03 (m, 6H), 2.56 - 2.47 (m, 4H), 2.47 - 2.39 (m, 4H), 2.21 - 2.12 (m, 2H), 1.95 - 1.90 (m, 1H), 1.70 - 1.57 (m, 6H), 1.56 - 1.47 (m, 4H), 1.46 - 1.38 (m, 29H), 1.30 - 1.25 (m, 10H).

Step H: **2-9** (15 g, 15.03 mmol) was dissolved in dichloromethane (114 mL), and then trifluoroacetic acid (38 mL) was added. The reaction mixture was stirred at 20°C for 16 hours. After concentration under reduced pressure, a mixture of toluene/acetonitrile (V/V = 3/1, 200 mL) was added and concentrated to dryness under reduced pressure. The above concentration step was repeated three times to obtain **2-10.**

Step I: **2-11** (22.15 g, 49.50 mmol), *N,N*-diisopropylethylamine (7.75 g, 60.00 mmol), HOAt (6.12 g, 45.00 mmol), and HATU (20.53 g, 54.00 mmol) were dissolved in *N,N*-dimethylformamide (90 mL), and then a solution of **2-10** (15.6 g, 15.00 mmol) and *N,N*-diisopropylethylamine (21.32 g, 165.00 mmol) in *N,N*-dimethylformamide (120 mL) was added. The mixture was stirred at 20°C for 16 hours, then dichloromethane (1.2 L) and hydrochloric acid (1 mol/L, 1 L) were added. After stirring and phase separation, the organic phase was sequentially washed with water (1 L), sodium bicarbonate aqueous solution (1 L), and saturated brine (1 L), dried over anhydrous sodium sulfate, filtered, and the filtrate was concentrated under reduced pressure. The residue was purified by silica gel column chromatography (eluent: V/V/V dichloromethane/methanol = 100/1 to 10/1 to dichloromethane/ethanol = 1/1) to obtain **2-12.** $^1$H NMR (400 MHz, DMSO-$d_6$): $\delta$ = 7.87 - 7.66 (m, 9H), 7.09 (s, 1H), 5.21 (d, *J* = 3.4 Hz, 3H), 4.96 (dd, *J* = 3.4, 11.3 Hz, 3H), 4.48 (d, *J* = 8.5 Hz, 3H), 4.06 - 3.98 (m, 9H), 3.91 - 3.82 (m, 3H), 3.74 - 3.66 (m, 3H), 3.58 - 3.46 (m, 12H), 3.31 (br s, 3H), 3.07 - 2.98 (m, 12H), 2.71 (t, *J* = 2.6 Hz, 1H), 2.33 - 2.22 (m, 8H), 2.16 - 2.12 (m, 2H), 2.10 (s, 9H), 2.04 (br t, *J* = 7.1 Hz, 6H), 1.99 (s, 9H), 1.89 (s, 9H), 1.81 - 1.74 (m, 9H), 1.54 - 1.39 (m, 22H), 1.32 (dd, *J* = 4.5, 6.7 Hz, 2H), 1.24 (s, 10H).

Step J: **2-12** (1.00 g, 0.50 mmol) and *N*-methyl-*N,N,N*-trioctylammonium chloride (20.35 mg, 50.35 μmol) were dissolved in a mixture of acetic acid (2.7 mL) and n-pentane (6.3 mL). At 0°C, an aqueous solution (9 mL) of potassium permanganate (0.40 g, 2.52 mmol) was added dropwise to the mixture. The mixture was stirred at 0 to 15°C for 2 hours. The reaction was quenched with sodium bisulfite (1.27 g), and added with hydrochloric acid (2 mol/L, 5 mL) and water (30 mL), and then extracted with 120 mL of a mixture of chloroform/isopropanol (V/V = 3/1, 40 mL × 3). The combined organic phases were dried over anhydrous sodium sulfate, filtered, and the filtrate was concentrated under reduced pressure. A mixture of toluene and acetonitrile (30 mL) was added to the residue, and the mixture was concentrated to dryness under reduced pressure. The above concentration step was repeated six times to obtain **2-13.** $^1$H NMR (400 MHz, $CD_3OD$): $\delta$ = 5.34 (d, *J* = 2.9Hz, 3H), 5.06 (dd, *J* = 3.3, 11.2 Hz, 3H), 4.56 (d, *J* = 8.4 Hz, 3H), 4.19 - 4.06 (m, 9H), 4.04 - 3.98 (m, 3H), 3.87 (td, *J* = 5.7, 9.9 Hz, 4H), 3.72 - 3.64 (m, 9H), 3.57 - 3.50 (m, 3H), 3.39 (br t, *J* = 6.4 Hz, 2H), 3.22 (q, *J* = 6.4 Hz, 12H), 2.51 - 2.40 (m, 9H), 2.21 (br t, *J* = 7.3 Hz, 6H), 2.14 (s, 9H), 2.03 (s, 9H), 1.94 (d, *J* = 7.9 Hz, 18H), 1.72 - 1.57 (m, 22H), 1.39 (br s, 12H).

Step K: To a solution of **2-13** (1.00 g, 0.50 mmol) in *N,N*-dimethylformamide (10 mL) was added *N,N*-diisopropylethylamine (0.26 g, 1.99 mmol) and HATU (0.23 g, 0.60 mmol) under stirring, and then **2-14** (0.23 g, 0.55 mmol) was added. The reaction mixture was stirred at 15°C for 16 hours, then dichloromethane (50 mL) and water (50 mL) were added. The mixture was stirred and then allowed to stand for phase separation. The organic phase was sequentially washed with saturated sodium bicarbonate aqueous solution (50 mL), water (50 mL), and saturated brine (50 mL), dried over anhydrous sodium sulfate, filtered, and concentrated under reduced pressure. The residue was purified by silica gel column chromatography (eluent: V/V dichloromethane/methanol = 20/1 to 10/1, containing 0.1% triethylamine) to obtain **2-15.** $^1$H NMR (400 MHz, DMSO-$d_6$): $\delta$ = 7.90 - 7.82 (m, 6H), 7.78 (d, *J* = 4.8 Hz, 3H), 7.40 - 7.26 (m, 10H), 6.91 (dd, *J* = 3.1, 9.0 Hz, 4H), 5.26 (d, *J* = 3.4 Hz, 3H), 5.03 - 4.99 (m, 3H), 4.53 (d, *J* = 8.4 Hz, 3H), 4.43 (d, *J* = 3.8 Hz, 1H), 4.23 - 4.14 (m, 1H), 4.12 - 4.02 (m, 9H), 3.92 (td, *J* = 9.0, 11.0 Hz, 3H), 3.78 (s, 6H), 3.77 - 3.71 (m, 3H), 3.66 - 3.51 (m, 13H), 3.49 - 3.41 (m, 4H), 3.11 - 3.01 (m, 16H), 2.38 - 2.37 (m, 1H), 2.32 (br s, 9H), 2.14 (s, 9H), 2.08 (br t, *J* = 6.9 Hz, 7H), 2.04 (s, 9H), 1.93 (s, 9H), 1.82 (s, 9H), 1.57 - 1.46 (m, 22H), 1.31 - 1.26 (m, 12H).

Step L: To a solution of **2-15** (0.80 g, 0.33 mmol) in dichloromethane (8 mL) were sequentially added triethylamine (67.24 mg, 0.64 mmol), 4-dimethylaminopyridine(0.12 g, 1.00 mmol), and succinic anhydride (83.13 mg, 0.83 mmol). The mixture was stirred at 10°C for 16 hours, and then dichloromethane (50 mL), water (30 mL), and saturated brine (30 mL) were added. The organic phase after phase separation was sequentially washed with water (30 mL) and saturated brine (30 mL), dried over anhydrous sodium sulfate, filtered, and concentrated under reduced pressure. The residue was purified by preparative HPLC (separation column: Waters Xbridge C18 (specifications: 150 mm × 50 mm, particle size: 10 μm); mobile phase: [water (10 mM ammonium bicarbonate)-acetonitrile]; elution gradient: 27% to 57%, 11 minutes) to

obtain compound **D01.** $^1H$ NMR (400 MHz, DMSO-$d_6$): $\delta$ = 7.96 - 7.69 (m, 9H), 7.33 - 7.09 (m, 10H), 6.90 - 6.78 (m, 4H), 5.21 (d, $J$ = 3.3 Hz, 3H), 4.97 (dd, $J$ = 3.3, 11.2 Hz, 3H), 4.49 (d, $J$ = 8.4 Hz, 3H), 4.06 - 3.97 (m, 9H), 3.91 - 3.83 (m, 3H), 3.79 - 3.66 (m, 11H), 3.63 - 3.45 (m, 18H), 3.02 (br d, $J$ = 4.6 Hz, 14H), 2.46 - 2.37 (m, 4H), 2.35 - 2.14 (m, 12H), 2.10 (s, 9H), 2.04 (t, $J$ = 7.0 Hz, 6H), 1.99 (s, 9H), 1.88 (s, 9H), 1.77 (s, 9H), 1.57 - 1.37 (m, 22H), 1.22 (br s, 12H).

**Example 5: Synthesis of D02**

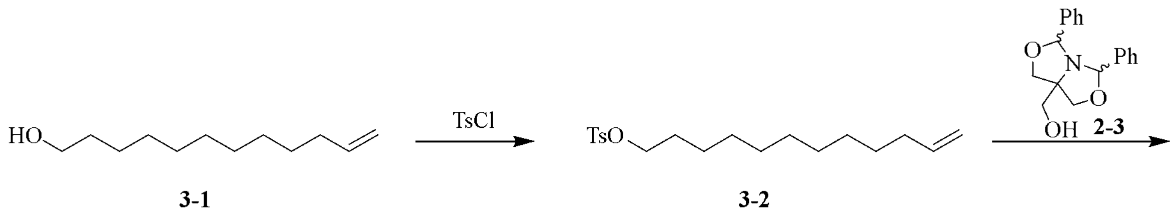

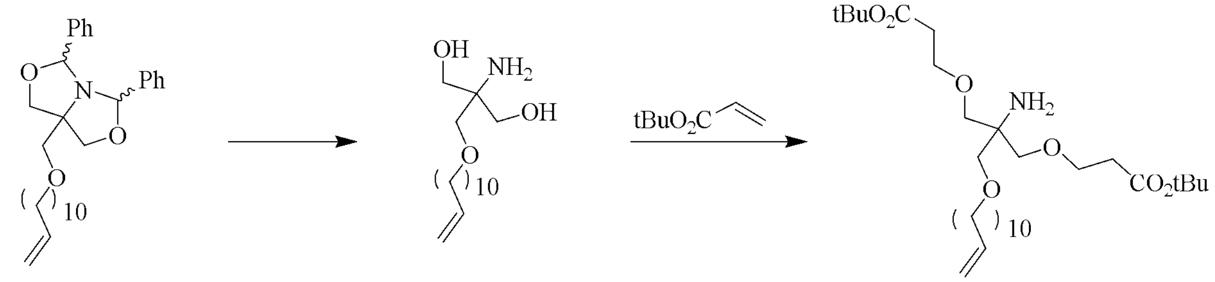

3-3 3-4 3-5

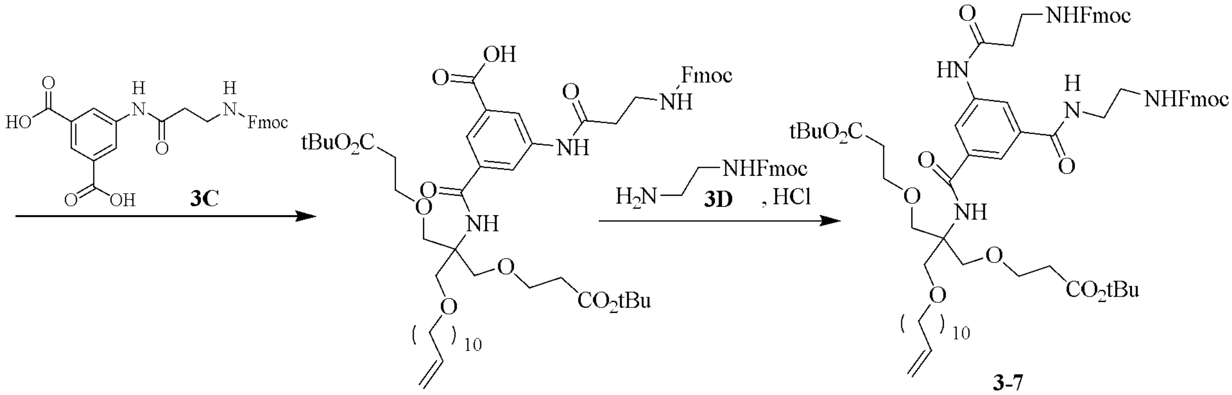


3-6 3-7

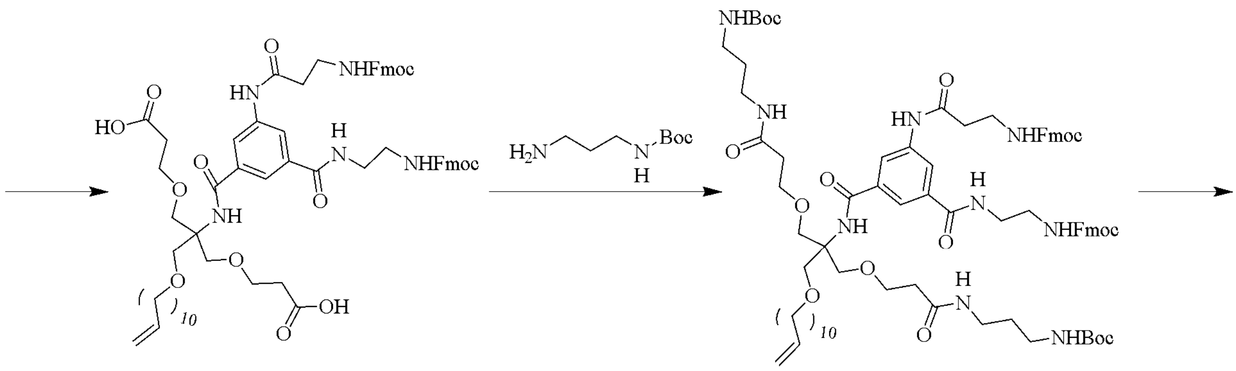

3-8 3-9

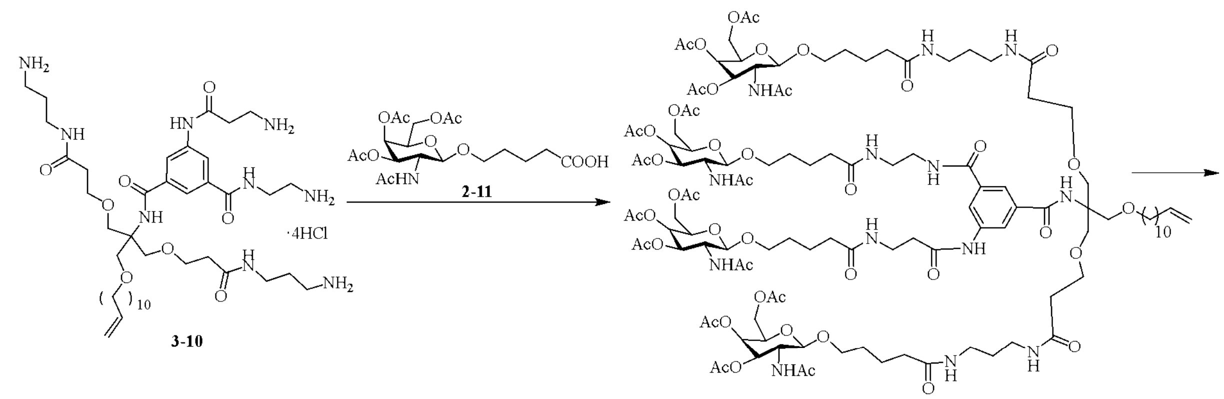


3-10 3-11

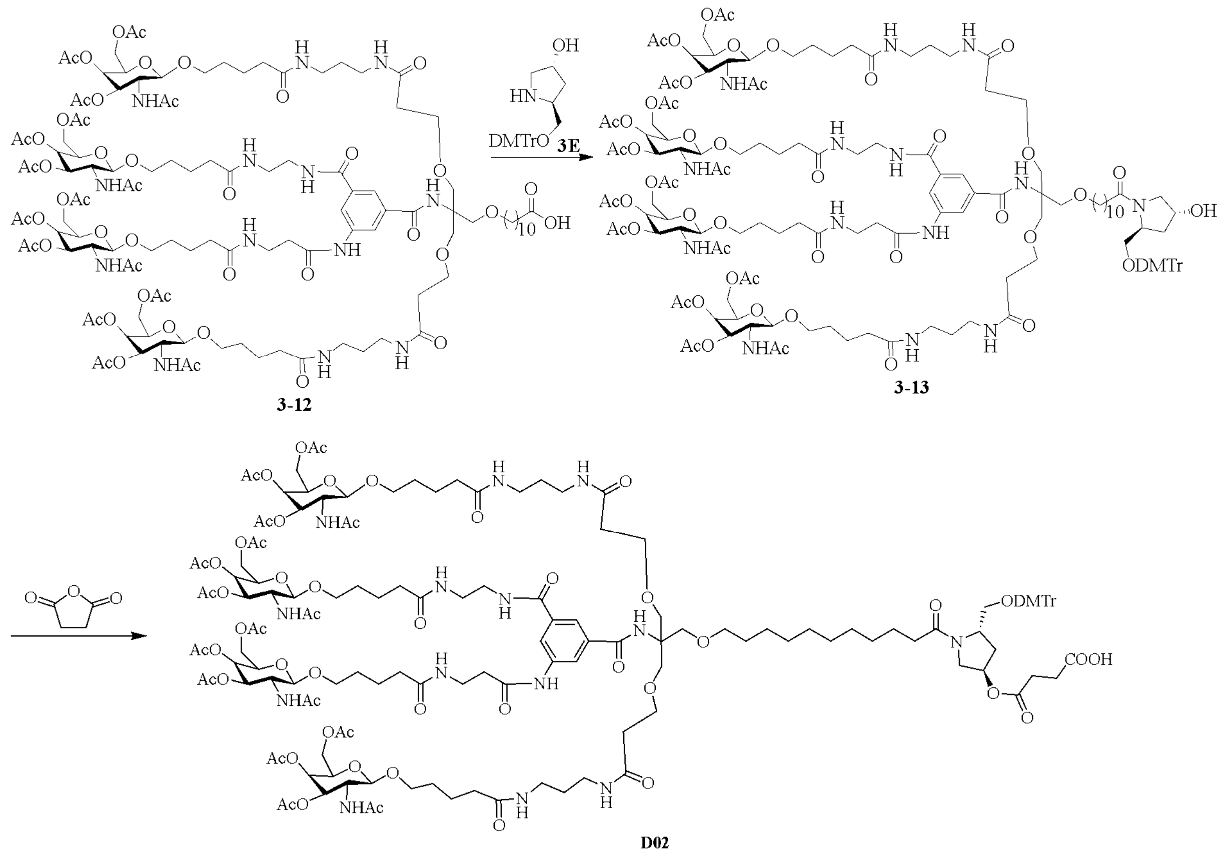

3-12

3-13

D02

Step A: To dichloromethane (500 mL) were sequentially added compound **3-1** (50 g, 271.27 mmol), trimethylamine hydrochloride (2.59 g, 27.13 mmol), and *p*-toluenesulfonyl chloride (77.98 g, 406.91 mmol), and then triethylamine (32.94 g, 325.53 mmol) was slowly added dropwise at 0°C. After the addition was completed, the reaction mixture was naturally warmed to room temperature and stirred for 12 hours. The reaction mixture was diluted with dichloromethane (500 mL) and sequentially washed once with sodium hydroxide solution (1 mol/L, 500 mL), dilute hydrochloric acid (1 mol/L, 500 mL), water (500 mL), and saturated brine (500 mL), respectively. The organic phase was dried over anhydrous sodium sulfate, filtered, and the filtrate was concentrated under reduced pressure to obtain compound **3-2.** $^1$H NMR (400 MHz, DMSO-$d_6$) $\delta$ = 7.77 (d, *J*=8.2 Hz, 2 H), 7.48 (d, *J* = 8.2 Hz, 2H), 5.79 - 5.77 (m, 1 H), 5.04 - 4.90 (m, 2 H), 3.99 (t, *J* = 6.2 Hz, 2 H), 2.42 (s, 3H), 2.00 (q, *J* = 6.75 Hz, 2 H), 1.59 - 1.47 (m, 2 H), 1.35 - 1.13 (m, 14 H).

Step B: To a solution of compound **3-2** (50 g, 147.71 mmol) in tetrahydrofuran (500 mL) were added potassium hydroxide (41.14 g, 738.54 mmol) and **2-3** (46.12 g, 155.09 mmol). The mixture was stirred at 80°C for 12 hours. After the reaction was completed, the reaction mixture was quenched with ice water (1000 mL), and washed with methyl *tert*-butyl ether (2000 mL, 1000 mL × 2). The organic phase was sequentially washed once with water (1000 mL) and saturated brine (1000 mL), respectively, then dried over anhydrous sodium sulfate. After filtration, the filtrate was concentrated under reduced pressure to obtain **3-3,** which was used directly in the next step.

Step C: Compound **3-3** (65.53 g, 141.34 mmol) was dissolved in a mixed solution of methanol (230 mL) and water (77 mL), and then concentrated hydrochloric acid (12 mol/L, 40 mL) was added. The reaction mixture was stirred at 50°C for 12 hours, then concentrated under reduced pressure until approximately 10 mL of liquid remained. Dilute hydrochloric acid (2 mol/L, 500 mL), methyl *tert*-butyl ether (500 mL), and petroleum ether (500 mL) were added to the residue, stirred evenly, and allowed to stand for a long time until phase separation occurred (divided into 3 layers). The middle liquid layer was obtained by phase separation, then sodium hydroxide solution (1 mol/L) was added to adjust the pH > 11, followed by extraction with dichloromethane twice (500 mL × 2). The combined organic phases were washed once with saturated brine (200 mL), dried over anhydrous sodium sulfate, filtered under reduced pressure, and the filtrate was concentrated under reduced pressure. Acetonitrile (90 mL) was added to the residue, and the mixture was heated to 60°C with stirring until complete dissolution was achieved. The solution was then slowly cooled to 0°C and stirred for another 30 minutes to precipitate a solid. The solid was collected by low-temperature filtration, and the filter cake was collected and dried under vacuum to obtain compound **3-4.** $^1$H NMR (400 MHz, DMSO-$d_6$) $\delta$ = 5.80 - 5.76 (m, 1H), 5.07 - 4.83 (m, 2H), 4.34 (t, *J* = 5.44 Hz, 2H), 3.21 (d, *J* = 5.26 Hz, 4H), 3.16 (s, 2H), 2.03 - 1.97 (m, 2H), 1.46 (t, *J* = 6.36 Hz, 2H), 1.32 (d, *J* = 6.72 Hz, 2H), 1.24 (br s, 12H).

Step D: To a solution of compound **3-4** (17.91 g, 62.31 mmol) in dimethyl sulfoxide (100 mL) were added sodium

hydroxide (224.3 mg, 5.61 mmol) and water (10 mL), and then *tert*-butyl acrylate (17.57 g, 137.08 mmol) was added under a nitrogen atmosphere. After the addition was completed, the reaction mixture was stirred at room temperature for 12 hours. The reaction mixture was diluted with a 0.2% sodium chloride aqueous solution (250 mL) and extracted twice with dichloromethane (400 mL, 250 mL × 2). The combined organic phases were dried over anhydrous sodium sulfate, filtered under reduced pressure, and the filtrate was concentrated under reduced pressure. The residue was purified by silica gel column chromatography (eluent: V/V/V petroleum ether/ethyl acetate/ethanol = 80/3/1 to 32/3/1) to obtain **3-5.** $^1$H NMR (400 MHz, $CDCl_3$) $\delta$ = 5.83 - 5.77 (m, 1H), 5.10 - 4.83 (m, 2H), 3.64 (t, *J* = 6.42 Hz, 5H), 3.40 - 3.24 (m, 8H), 2.44 (t, *J* = 6.36 Hz, 5H), 2.02 (q, *J* = 7.01 Hz, 2H), 1.54 - 1.48 (m, 2H), 1.43 (s, 18H), 1.38 - 1.33 (m, 2H), 1.25 (br s, 12H). LC-MS (ESI) m/z: 544.4 $[M+H]^+$.

Step E: Compound **3C** (10.47 g, 22.07 mmol) was dissolved in *N,N*-dimethylformamide (140 mL), and then HATU (6.70 g, 17.65 mmol) and triethylamine (4.10 mL) were added sequentially. The mixture was stirred at 35°C for 5 minutes, and then compound **3-5** (8.00 g, 14.71 mmol) was added. After the addition was completed, the mixture was stirred at 35°C for another 12 hours. The reaction mixture was diluted with water (100 mL) and extracted with ethyl acetate (300 mL). The organic phase was washed twice with water (100 mL) and once with saturated brine (50 mL). After concentration under reduced pressure, the residue was purified by preparative HPLC (separation column: Agela Innoval ODS-2 250 mm × 100 mm × 10 μm; mobile phase: phase A: 10 mM trifluoroacetic acid aqueous solution, phase B: acetonitrile; elution gradient: 90% to 100%, 20 min) to obtain compound **3-6.** $^1$H NMR (400 MHz, $CDCl_3$) $\delta$ = 9.08 (s, 1H), 8.34 (s, 2H), 8.29 (s, 1H), 7.73 (d, *J* = 7.60 Hz, 2H), 7.57 (d, *J* = 7.60 Hz, 2H), 7.36 (t, *J* = 7.20 Hz, 2H), 7.28 - 7.24 (m, 1H), 6.76 (s, 1H), 5.94 - 5.93 (m, 1H), 5.86 - 5.76 (m, 1H), 5.01 - 4.92 (m, 2H), 4.38 (d, *J* = 7.20 Hz, 2H), 4.19 - 4.16 (m, 1H), 3.91 - 3.83 (m, 6H), 3.75 - 3.72 (m, 4H), 3.63 - 3.59 (m, 2H), 3.46 (t, *J* = 6.80 Hz, 2H), 2.74 - 2.71 (m, 2H), 2.51 (t, *J* = 6.00 Hz, 4H), 2.04 - 2.01 (m, 2H), 1.56 - 1.54 (m, 2H), 1.42 (s, 18H), 1.30 - 1.25 (m, 16H). LC-MS (ESI) m/z: 1014.6 $[M+H]^+$.

Step F: Compound **3-6** (2.55 g, 2.55 mmol) was dissolved in*N,N*-dimethylformamide (50 mL), and then HATU (1.07 g, 2.80 mmol) and triethylamine (0.35 mL) were added. The reaction mixture was stirred at 25 to 35°C for 5 minutes, and then compound 3D (0.81 g, 2.55 mmol) and triethylamine (0.35 mL) were added. After the addition was completed, stirring was continued for 3 hours. The reaction mixture was diluted with water (50 mL) and extracted with ethyl acetate (150 mL). The organic phase was washed once with water (50 mL) and once with saturated brine (50 mL). After concentration under reduced pressure, compound **3-7** was obtained. $^1$H NMR (400 MHz, $CDCl_3$) $\delta$ = 8.40 (s, 1H), 8.20 (s, 1H), 8.04 (s, 2H), 8.00 (s, 1H), 7.83 (s, 1H), 7.75 - 7.73 (m, 4H), 7.59 - 7.57 (m, 4H), 7.47 (brs, 1H), 7.39 - 7.35 (m, 4H), 7.26 - 7.24 (m, 2H), 6.69 (s, 1H), 5.83 - 5.79 (m, 2H), 5.70 (brs, 1H), 5.02 - 4.93 (m, 2H), 4.40 - 4.36 (m, 3H), 4.21 - 4.17 (m, 2H), 3.90 - 3.88 (m, 2H), 3.80 - 3.78 (m, 4H), 3.73 - 3.70 (m, 4H), 3.62 - 3.54 (m, 3H), 3.47 - 3.40 (m, 4H), 2.62 (brs, 2H), 2.48 (t, *J* = 6.00 Hz, 4H), 2.05 - 2.01 (m, 2H), 1.55 - 1.52 (m, 2H), 1.38 (s, 18H), 1.30 - 1.26 (m, 16H). LC-MS (ESI) m/z: 1265.0 $[M+H]^+$.

Step G: Compound **3-7** (9 g, 7.12 mmol) was dissolved in anhydrous dioxane (15 mL), and then a hydrochloric acid/dioxane solution (4 mol/L, 71.17 mL) was added. The reaction mixture was stirred at room temperature for 1 hour, and after concentration under reduced pressure, compound **3-8** was obtained. $^1$H NMR (400 MHz, DMSO-$d_6$) $\delta$ = 10.22 (s, 1H), 8.56 (t, *J* = 5.3 Hz, 1H), 8.24 (s, 1H), 8.06 (s, 1H), 7.95 - 7.81 (m, 5H), 7.69 (d, *J* = 7.4 Hz, 4H), 7.47 - 7.24 (m, 10H), 5.76 (tdd, *J* = 6.6, 10.3, 17.0 Hz, 1H), 5.05 - 4.82 (m, 2H), 4.42 - 4.27 (m, 4H), 4.22 (d, *J* = 6.4 Hz, 2H), 3.71 (s, 3H), 3.68 - 3.60 (m, 6H), 3.36 (dd, *J* = 6.8, 13.4 Hz, 6H), 3.22 - 3.16 (m, 2H), 2.57 - 2.53 (m, 2H), 2.45 (t, *J* = 6.3 Hz, 3H), 2.10 - 1.86 (m, 2H), 1.58 - 1.01 (m, 18H). LC-MS (ESI) m/z: 1152.6 $[M+H]^+$.

Step H: Compound **3-8** (9 g, 7.81 mmol) was dissolved in *N,N*-dimethylformamide (100 mL), and then HATU (6.83 g, 17.96 mmol), *N,N*-diisopropylethylamine (1.01 g, 7.81 mmol, 1.36 mL), and *tert*-butyl (3-aminopropyl)carbamate (2.99 g, 17.18 mmol) were added. The reaction mixture was stirred at 25°C for 4 hours and then concentrated under reduced pressure. The residue was purified by silica gel column chromatography (eluent: V/V petroleum ether/ethyl acetate = 30/1 to 0/1) to obtain compound **3-9.**

Step I: Compound **3-9** (15 g, 10.24 mmol) was dissolved in anhydrous dichloromethane (110 mL), and then tetrahydropyrrole (1.82 g, 25.60 mmol, 2.14 mL) was added. The reaction mixture was stirred at room temperature for 3 hours and then concentrated under reduced pressure. The residue was purified by preparative HPLC (separation column: Waters Xbridge C18 (specifications: 250 mm× 100 mm, particle size: 10 μm); mobile phase: phase A: 10 mM trifluoroacetic acid aqueous solution; phase B: acetonitrile; elution gradient: 35% to 55%, 25 minutes) to obtain a colorless oil. The colorless oil (5 g) was dissolved in anhydrous dioxane (35 mL), and a hydrochloric acid/dioxane solution (4 mol/L, 38 mL) was added, and stirred at 25°C for 12 hours. After concentration under reduced pressure, compound **3-10** was obtained. $^1$H NMR (400 MHz, DMSO-$d_6$) $\delta$ = 10.66 (s, 1H), 9.04 (t, *J* = 5.1 Hz, 1H), 8.35 (s, 1H), 8.24 - 8.18 (m, 3H), 7.42 (s, 1H), 5.92 - 5.82 (m, 1H), 5.04 - 4.94 (m, 2H), 3.83 - 3.57 (m, 13H), 3.48 - 3.39 (m, 2H), 3.20 - 3.02 (m, 8H), 2.91 - 2.76 (m, 6H), 2.06 (q, *J* = 6.8 Hz, 2H), 1.73 (q, *J* = 6.9 Hz, 4H), 1.58 - 1.47 (m, 2H), 1.45 - 1.18 (m, 16H). LC-MS (ESI) m/z: 821.0 $[M+H]^+$.

Step J: Compound **3-10** (9.37 g, 20.94 mmol), N,N-diisopropylethylamine (2.71 g, 20.94 mmol), and HATU (9.27 g, 20.94 mmol) were sequentially added to *N,N*-dimethylformamide (50 mL), and the reaction mixture was stirred at 10 to 15°C for 2 hours. Compound **2-11** (4.6 g, 4.76 mmol) andN,N-diisopropylethylamine (2.46 g, 19.04 mmol) were then slowly added to the reaction mixture. After the addition was completed, the reaction mixture was stirred at 10 to 15°C for another

12 hours. The reaction mixture was slowly poured into water (500 mL) and extracted with dichloromethane (500 mL). The organic phase was washed once with saturated brine (200 mL), dried over anhydrous sodium sulfate, filtered under reduced pressure, and the filtrate was concentrated under reduced pressure. The residue was purified by preparative HPLC (chromatographic column: Welch Ultimate XB-CN, 250 mm × 70 mm × 10 μm; mobile phase: phase A: n-hexane; phase B: ethanol; 15 minutes) to obtain compound **3-11.** $^1$H NMR (400 MHz, $CDCl_3$) $\delta$ = 8.62 (br s, 1H), 8.20 (s, 1H), 8.07 (s, 1H), 7.96 - 7.88 (m, 2H), 7.82 (br d, *J* = 9.0 Hz, 6H), 7.71 (t, *J* = 5.3 Hz, 1H), 7.27 (br s, 1H), 5.22 (d, *J* = 3.3 Hz, 4H), 5.01 - 4.94 (m, 4H), 4.48 (br d, *J* = 8.5 Hz, 4H), 4.03 (s, 12H), 3.95 - 3.83 (m, 4H), 3.78 - 3.58 (m, 14H), 3.38 - 3.27 (m, 19H), 3.00 (d, *J* = 5.6 Hz, 7H), 2.38 - 2.27 (m, 4H), 2.11 (s, 12H), 2.04 (d, *J* = 6.8 Hz, 8H), 2.00 (s, 11H), 1.89 (s, 12H), 1.77 (d, *J* = 1.5 Hz, 12H), 1.57 - 1.42 (m, 20H), 1.27 - 1.15 (m, 16H).

Step K: To a mixture of dichloromethane (6 mL), acetonitrile (6 mL), and water (9 mL) were added compound **3-11** (2.0 g, 788.10 μmol) and ruthenium(III) chloride trihydrate (4.12 mg, 15.76 μmol) at room temperature. Then, sodium periodate (1.26 g, 5.9 mmol) and sodium bicarbonate (132.41 mg, 1.58 mmol) were added. The reaction mixture was stirred at room temperature for 16 hours, then slowly poured into water (50 mL) and extracted with dichloromethane (50 mL). The organic phase was washed once with saturated sodium sulfite solution (20 mL), and once with saturated brine (20 mL), dried over anhydrous sodium sulfate, filtered under reduced pressure, and the filtrate was concentrated under reduced pressure to obtain compound **3-12.**

Step L: Compound **3-12** (1.3 g, 508.66 μmol) was dissolved in dichloromethane (15 mL), and then HATU (232.09 mg, 610.40 μmol) and N,N-diisopropylethylamine (65.74 mg, 508.66 μmol) were added. The reaction mixture was stirred at 10 to 15°C for 15 minutes, then compound **3E** (234.73 mg, 559.53 μmol) was added to the reaction mixture, and stirring was continued at 10 to 15°C for 1 hour. The reaction mixture was poured into a 5% sodium bicarbonate aqueous solution (50 mL) and then extracted with dichloromethane (50 mL). The organic phase was washed once with saturated brine (20 mL), dried over anhydrous sodium sulfate, filtered under reduced pressure, and the filtrate was concentrated under reduced pressure. The residue was purified by preparative HPLC (chromatographic column: Welch Ultimate XB-SiOH, 250 mm × 70 mm × 10 μm; mobile phase: phase A: *n*-hexane; phase B: ethanol; 15 minutes) to obtain compound **3-13.** $^1$H NMR (400 MHz, $CDCl_3$) $\delta$ = 8.69 - 8.57 (m, 1H), 8.20 (s, 1H), 8.07 (s, 1H), 7.91 (q, J = 5.5 Hz, 2H), 7.83 (d, *J* = 9.2 Hz, 6H), 7.72 (t, *J* = 5.3 Hz, 2H), 7.37 - 7.26 (m, 5H), 7.22 - 7.15 (m, 4H), 6.92 - 6.83 (m, 4H), 5.21 (d, *J* = 3.3 Hz, 4H), 4.99 - 4.95 (m, 4H), 4.48 (d, *J* = 8.4 Hz, 4H), 4.02 (s, 12H), 3.93 - 3.82 (m, 4H), 3.76 - 3.55 (m, 21H), 3.42 - 3.34 (m, 22H), 3.25 - 3.14 (m, 3H), 3.04 - 2.91 (m, 9H), 2.33 - 2.27 (m, 4H), 2.10 (s, 12H), 2.05 - 1.99 (m, 21H), 1.89 (s, 12H), 1.77 (d, *J* = 1.7 Hz, 12H), 1.51 - 1.42 (m, 20H), 1.28 - 1.16 (m, 16H).

Step M: To dichloromethane (10 mL) were sequentially added compound **3-13** (1 g, 338.16 μmol), triethylamine (119.76 mg, 1.18 mmol), and 4-dimethylaminopyridine (20.66 mg, 169.08 μmol), and then succinic anhydride (101.52 mg, 1.01 mmol) was added. The reaction mixture was stirred at room temperature for 48 hours. The reaction mixture was diluted with water (20 mL) and extracted with dichloromethane (50 mL). The organic phase was dried over anhydrous sodium sulfate, filtered under reduced pressure, and the filtrate was concentrated under reduced pressure. The residue was subjected to preparative HPLC (chromatographic column: Waters Xbridge C18, 150 mm × 50 mm × 10 μm; mobile phase: phase A: 10 mM ammonium bicarbonate aqueous solution; phase B: acetonitrile; 10 minutes) to obtain compound **D02.** $^1$H NMR (400 MHz, $CD_3CN$) $\delta$ = 9.68 (br s, 1H), 8.29 (br s, 1H), 8.15 (d, *J* = 2.5 Hz, 2H), 7.87 (s, 1H), 7.43 - 7.19 (m, 13H), 7.15 - 6.97 (m, 7H), 6.93 - 6.80 (m, 4H), 5.47 - 5.26 (m, 5H), 5.04 (dd, *J* = 2.7, 11.2 Hz, 4H), 4.62 - 4.48 (m, 4H), 4.25 - 3.92 (m, 18H), 3.87 - 3.65 (m, 22H), 3.54 - 3.34 (m, 13H), 3.10 - 3.01 (m, 8H), 2.44 - 2.38 (m, 6H), 2.32 - 2.28 (m, 2H), 2.20 - 2.09 (m, 22H), 2.00 (br s, 12H), 1.97 (d, *J* = 2.4 Hz, 4H), 1.96 - 1.92 (m, 12H), 1.87 (s, 12H), 1.64 - 1.41 (m, 24H), 1.34 - 1.13 (m, 12H). LC-MS (ESI) m/z: 1529.3 $[M+2H]^{2+}$.

**Preparation of Intermediate 3C:**

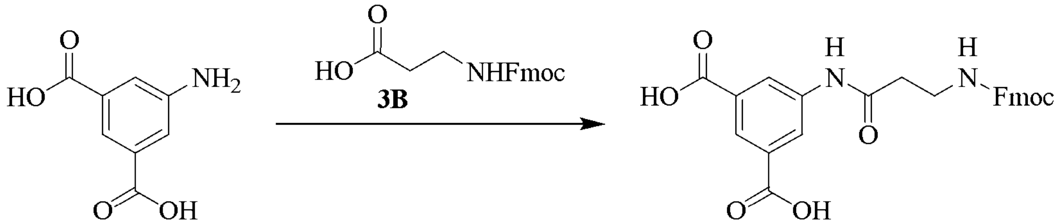


3A 3C

Compound **3B** (42.97 g, 138.01 mmol) was dissolved in *N,N*-dimethylformamide (500 mL), cooled to 0 to 10°C, and then HATU (57.72 g, 151.81 mmol) and *N,N*-diisopropylethylamine (21.40 g, 165.61 mmol) were added. The reaction

mixture was stirred at room temperature for 0.5 hours, and then compound **3A** (25.00 g, 138.01 mmol) was added. After the addition was completed, stirring was continued at room temperature for 1 hour. The reaction mixture was poured into dilute hydrochloric acid aqueous solution (23 mL of concentrated hydrochloric acid + 2.5 L of water) and stirred for 0.5 hours. After filtration, the filter cake was added to ethanol (650 mL) and stirred at 60°C for 1 hour. After naturally cooling to room temperature, the mixture was filtered under reduced pressure, and the filter cake was collected and dried under reduced pressure to obtain compound **3C.** $^1$H NMR (400 MHz, DMSO-$d_6$) $\delta$ = 10.34 (s, 1H), 8.46 (s, 2H), 8.15 (s, 1H), 7.95 (s, 1H), 7.87 (d, $J$ = 7.50 Hz, 2H), 7.67 (br d, $J$ = 7.38 Hz, 2H), 7.45 (t, $J$ = 5.50 Hz, 1H), 7.39 (t, $J$ = 7.44 Hz, 2H), 7.26 - 7.33 (m, 2H), 4.26 - 4.30 (m, 2H), 4.18 - 4.24 (m, 1H), 4.04 - 4.04 (m, 1H), 2.54 (t, $J$ = 5.57 Hz, 2H).

**Example 6: Synthesis of D03**

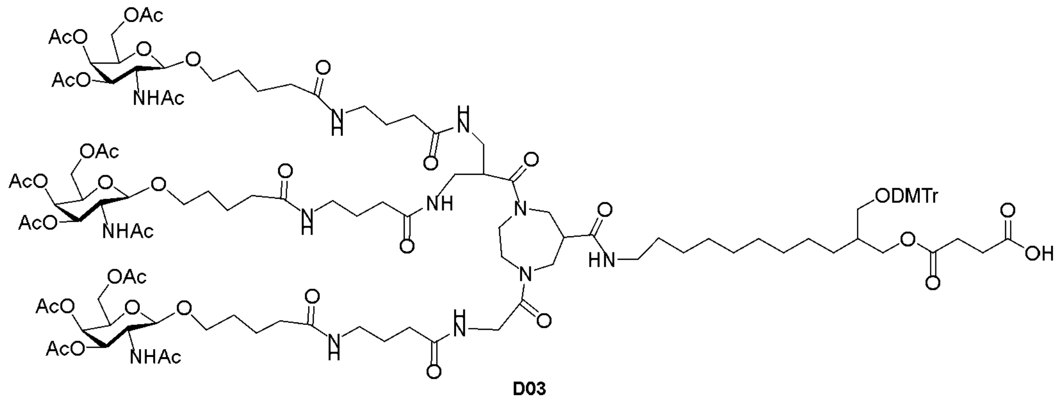

D03

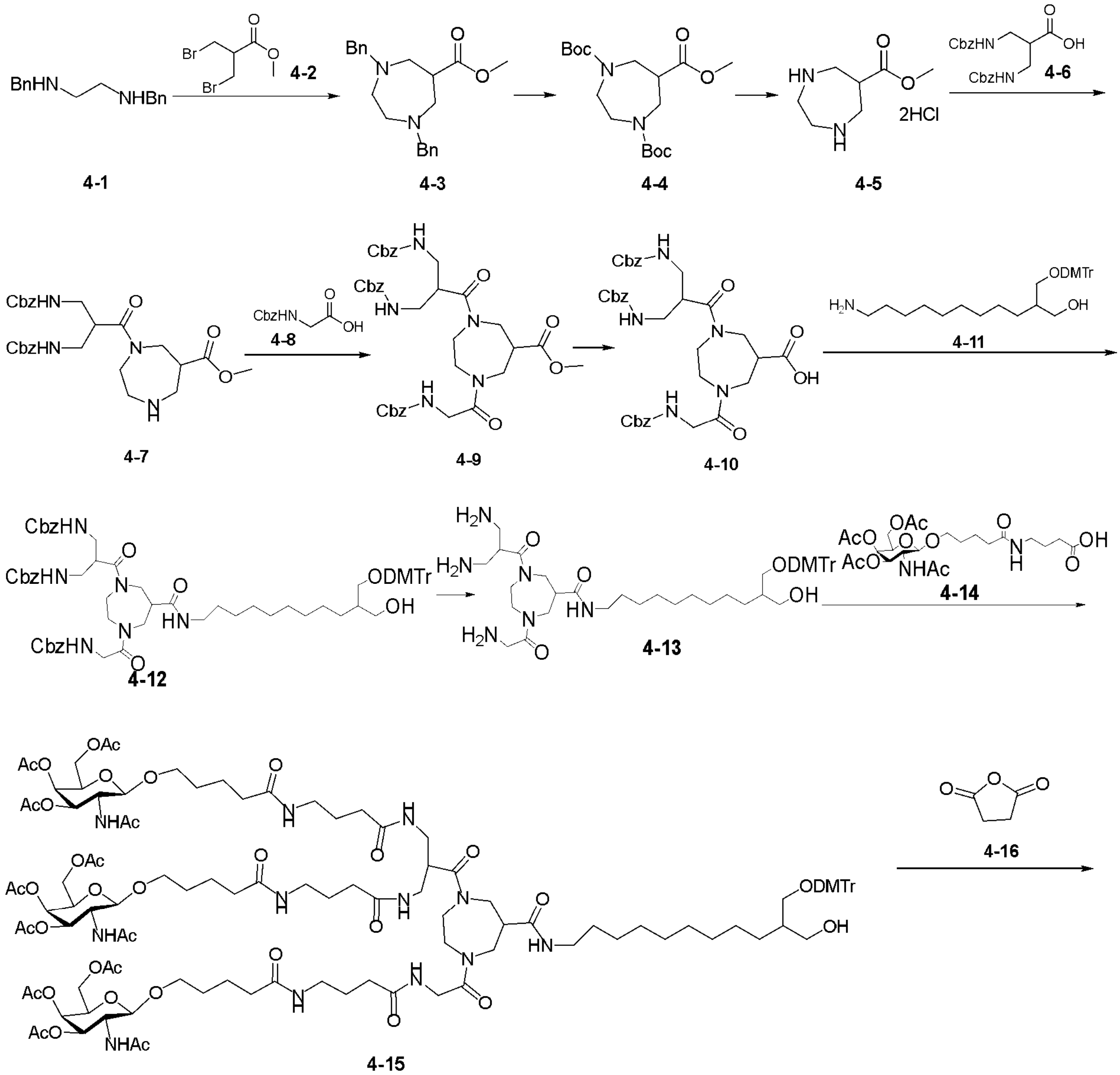

4-1 4-2 4-3 4-4 4-5 4-6

4-7 4-8 4-9 4-10 4-11

4-12 4-13 4-14

4-15 4-16

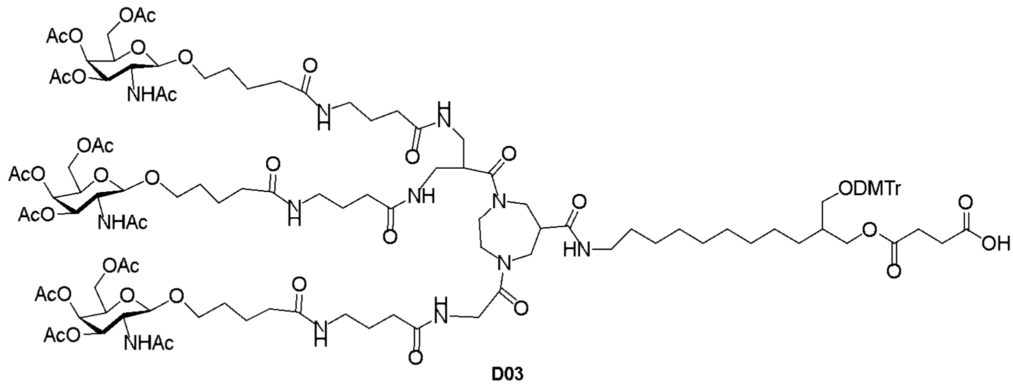

D03

**Step A:** To a solution of compound **4-2** (13.24 g, 50.93 mmol) in toluene (150 mL) at 25°C were added triethylamine (10.10 g, 99.86 mmol, 13.90 mL) and a solution of compound **4-1** (12 g, 49.93 mmol, 11.76 mL) in toluene (100 mL). The reaction was stirred at 100°C for 16 hours. Saturated sodium carbonate aqueous solution (200 mL) was added to the reaction mixture, and the phases were separated. The organic phase was dried over anhydrous sodium sulfate, filtered, and concentrated to obtain a crude product, which was separated by preparative HPLC (column: Agela Innoval ODS-2 250 mm × 100 mm × 10 μm; mobile phase: [water (0.225% formic acid)-acetonitrile]; acetonitrile%: 15% to 35%, 25 min) to obtain compound **4-3.**

**Step B:** A solution of compound **4-3** (12.6 g, 31.04 mmol), palladium on carbon (5 g, 10% content), and di-tert-butyl dicarbonate (14.90 g, 68.28 mmol, 15.69 mL) in methanol (120 mL) was purged with argon three times, and then purged with hydrogen three times. The reaction mixture was stirred at 25°C for 16 hours under a hydrogen atmosphere at normal pressure. The reaction mixture was filtered through diatomite and concentrated under reduced pressure. The crude product was purified by silica gel column chromatography to obtain compound **4-4.**

**Step C:** Compound **4-4** (7.5 g, 18.66 mmol) was dissolved in an ethyl acetate solution of hydrochloric acid (4 mol/L, 18.66 mL). After purging with nitrogen three times, the mixture was stirred under a nitrogen atmosphere at 25°C for 1 hour. After concentration under reduced pressure, compound **4-5** was obtained.

**Step D:** A solution of compound **4-5** (2.24 g, 9.68 mmol), compound **4-6** (2.2 g, 5.69 mmol), triethylamine (2.88 g, 28.47 mmol, 3.96 mL), and propylphosphonic anhydride (5.06 g, 7.95 mmol, 4.73 mL, 50% content) in N,N-dimethylformamide (25 mL) was purged with nitrogen three times, and then the reaction mixture was stirred at 25°C for 1 hour under a nitrogen atmosphere. The reaction mixture was added with water (50 mL) and then extracted with ethyl acetate (80 mL × 2). The combined organic phases were washed with saturated brine (100 mL × 1), dried over anhydrous sodium sulfate, filtered, and concentrated. The crude product was purified by preparative HPLC (column: Phenomenex luna C18 150 × 40 mm × 15 μm; mobile phase: [water (0.225% formic acid)-acetonitrile]; acetonitrile%: 14% to 44%, 15 min) to obtain compound **4-7.**

**Step E:** A solution of compound **4-7** (2.2 g, 4.06 mmol), compound **4-8** (1.50 g, 6.09 mmol), O-(benzotriazol-1-yl)-*N*,*N*,*N*',*N*'-tetramethyluronium tetrafluoroborate (2.22 g, 6.90 mmol), and triethylamine (1.23 g, 12.18 mmol, 1.69 mL) in *N,N*-dimethylformamide (20 mL) was purged with nitrogen three times, and then the reaction mixture was stirred at 25°C for 1 hour under a nitrogen atmosphere. The reaction mixture was added with water (80 mL) and then extracted with ethyl acetate (80 mL × 2). The combined organic phases were washed with saturated brine (100 mL × 1), dried over anhydrous sodium sulfate, filtered, and concentrated to obtain a crude product. The crude product was separated by silica gel column chromatography (eluent: petroleum ether/ethyl acetate, gradient elution) to obtain compound **4-9.**

**Step F:** To a mixed solution of compound **4-9** (2.5 g, 3.43 mmol) in methanol (22 mL) and water (7 mL) was added lithium hydroxide monohydrate (504.37 mg, 12.02 mmol). The reaction mixture was stirred at 25°C for 12 hours. Water (50 mL) was added to the reaction mixture. The aqueous phase was adjusted to pH = 4 to 5 with 2 M hydrochloric acid, then extracted with ethyl acetate (100 mL × 2). The combined organic phases were washed with saturated brine (50 mL). The organic phase was dried over anhydrous sodium sulfate, filtered, and the filtrate was concentrated to obtain compound **4-10.**

**Step G:** A solution of compound **4-10** (500 mg, 710.49 μmol), compound **4-11** (406.18 mg, 781.54 μmol), 2-(1*H*-benzotriazol-1-yl)-1,1,3,3-tetramethyluronium hexafluorophosphate (485.01 mg, 1.28 mmol), and triethylamine (215.68 mg, 2.13 mmol) in *N,N*-dimethylformamide (5 mL) was purged with nitrogen three times, and then the reaction mixture was stirred at 25°C under a nitrogen atmosphere for 1 hour. The reaction mixture was added with water (80 mL) and then extracted with dichloromethane (80 mL × 2). The combined organic phases were washed with saturated brine (100 mL × 1), dried over anhydrous sodium sulfate, filtered, and the filtrate was concentrated. The crude product was purified by preparative HPLC (column: Waters Xbridge C18 150 × 50 mm × 10 μm; mobile phase: [water (10 mM ammonium bicarbonate)-acetonitrile]; acetonitrile%: 57% to 87%, 10 min) to obtain compound **4-12.**

**Step H:** To a solution of compound **4-12** (320 mg, 203.93 μmol) in methanol (10 mL) was added palladium on

carbon (100 mg, 10% content). The reaction mixture was purged with argon three times, and then purged with hydrogen three times, and stirred at 25°C for 16 hours under a hydrogen atmosphere at normal pressure. The reaction mixture was filtered through diatomite, and the filtrate was concentrated under reduced pressure to obtain compound **4-13.**

**Step I:** A solution of compound **4-13** (178 mg, 221.66 μmol), compound **4-14** (413.14 mg, 775.80 μmol), 2-(1*H*-benzotriazol-1-yl)-1,1,3,3-tetramethyluronium hexafluorophosphate (336.25 mg, 886.63 μmol), and triethylamine (179.43 mg, 1.77 mmol) in *N,N*-dimethylformamide (4 mL) was purged with nitrogen three times, and then the reaction mixture was stirred at 25°C under a nitrogen atmosphere for 1 hour. The reaction mixture was added with water (100 mL) and extracted twice with 50 mL of DCM/i-PrOH (3/1). The combined organic phases were washed with saturated brine (150 mL × 1), dried over anhydrous sodium sulfate, and filtered. The filtrate was concentrated under reduced pressure, and purified by preparative HPLC (column: Waters Xbridge C18 150 × 50 mm, 10 μm; mobile phase: [water (10 mM ammonium bicarbonate)-acetonitrile]; acetonitrile%: 36% to 66%, 10 min) to obtain compound **4-15.**

**Step J:** A solution of compound **4-15** (240 mg, 97.49 μmol), compound **4-16** (34.15 mg, 341.21 μmol), 4-dimethylaminopyridine (11.91 mg, 97.49 μmol), and triethylamine (9.86 mg, 97.49 μmol) in dichloromethane (5 mL) was purged with nitrogen three times, and then the reaction mixture was stirred at 25°C under a nitrogen atmosphere for 16 hours. The reaction mixture was concentrated and purified by preparative HPLC (column: Waters Xbridge C18 150 × 50 mm × 10 μm; mobile phase: [water (10 mM ammonium bicarbonate)-acetonitrile]; acetonitrile%: 19% to 49%, 10 minutes) to obtain **compound D03.** LC-MS (ESI) m/z: 1172 $[M-2H]^{2-}$. $^1H$ NMR (400 MHz, DMSO-$d_6$) $\delta$ = 7.65 - 8.20 (m, 11 H), 7.28 - 7.39 (m, 4 H), 7.17 - 7.28 (m, 5 H), 6.78 - 7.03 (m, 4 H), 5.13 - 5.33 (m, 3 H), 4.89 - 5.10 (m, 3 H), 4.39 - 4.60 (m, 3 H), 3.94 - 4.15 (m, 15 H), 3.82 - 3.92 (m, 5 H), 3.64 - 3.81 (m, 12 H), 2.83 - 3.19 (m, 16 H), 2.08 - 2.19 (m, 14 H), 1.96 - 2.07 (m, 19 H), 1.86 - 1.92 (m, 9 H), 1.74 - 1.81 (m, 10 H), 1.54 - 1.65 (m, 7 H), 1.34 - 1.54 (m, 16 H), 1.03 - 1.33 (m, 16 H).

**Example 7: Synthesis of double-stranded siRNA analogs or conjugates thereof**

Synthesis of single-stranded oligoribonucleotides containing D1, D2, or D3: The oligoribonucleotides were synthesized using the phosphoramidite solid-phase synthesis technique. The synthesis was performed on a solid support prepared by covalently linking D01, D02, or D03 to controlled pore glass (amino CPG, 500 Å). All 2'-modified RNA phosphoramidites and auxiliary reagents were commercially available. All amides were sequentially dissolved in anhydrous acetonitrile, with the addition of molecular sieves (3 Å), and coupling was performed for 5 minutes using 5-ethylthio-1*H*-tetrazole (ETT) as the activator. Alternatively, a solution of 50 mM ((dimethylaminomethylidene)amino)-3*H*-1,2,4-dithiazoline-3-thione (DDTT) in anhydrous acetonitrile/pyridine (V/V = 1/1) was used to generate the phosphorothioate ester bond, with a reaction time of 3 minutes. All sequences were synthesized after final removal of the DMTr group.

Synthesis of single-stranded oligoribonucleotides without D1, D2, or D3: The oligoribonucleotides were synthesized according to the sequence design using the phosphoramidite solid-phase synthesis technique. The synthesis was performed on universal controlled pore glass CPG (500 Å). All 2'-modified RNA phosphoramidites and auxiliary reagents were commercially available. Modified R and E were obtained according to Examples 1, 2, and 3. All phosphoramidites were sequentially dissolved in anhydrous acetonitrile, with the addition of molecular sieves (3 Å), and coupling was performed for 5 minutes using 5-ethylthio-1*H*-tetrazole (ETT) as the activator. Alternatively, a solution of 50 mM ((dimethylaminomethylidene)amino)- 1,2,4-dithiazoline-3-thione (DDTT) in anhydrous acetonitrile/pyridine (V/V = 1/1) was used to generate the phosphorothioate ester bond, with a reaction time of 3 minutes. All sequences were synthesized after final removal of the DMTr group.

Cleavage and deprotection of oligomers bound to CPG: After the termination of solid-phase synthesis, the protecting groups were removed by treatment with a solution of 20% diethylamine in acetonitrile for 30 minutes, without cleaving the oligonucleotide from the CPG. Subsequently, the dried CPG was treated with concentrated aqueous ammonia at 40°C for 18 hours. After centrifugation, the supernatant was transferred to a new tube, and the CPG was washed with aqueous ammonia. The combined solutions were concentrated to obtain a solid mixture.

Purification of single-stranded oligoribonucleotides: The oligomers were purified by HPLC using NanoQ anion exchange. Buffer A consisted of 10 mM sodium perchlorate, 20 mM Tris, 1 mM EDTA, pH 7.4, with 20% acetonitrile, and Buffer B (500 mM sodium perchlorate, 20 mM Tris, 1 mM EDTA, pH 7.4, with 20% acetonitrile). The target product was isolated and desalted using a reverse-phase C18 column.

Double-stranded siRNA was generated by annealing single-stranded oligoribonucleotides: The single-stranded oligoribonucleotides to be annealed were prepared to 200 μM with sterile RNase Free $H_2O$ (free of RNase). Complementary strands were formed by combining equimolar solutions of single-stranded oligoribonucleotides. The annealing reaction system was set up as follows: A total volume of 100 μL of the mixture, 10 nmol, was placed in a 95°C water bath for 10 minutes (≥100 nmol required 20 minutes at high temperature), then rapidly transferred to a 60°C water bath. Annealing was completed upon natural cooling. The purity of the obtained double-stranded siRNA was detected by HPLC, while the molecular weights of the sense and antisense strands were confirmed by HRMS to verify the molecular weight of the double-stranded siRNA and identify it as the target product.

**Experimental Example 1: Evaluation of the inhibitory activity of test compounds against HBV using HepG2.2.15 cells**

**1. Experimental purpose:**

The purpose of this study was to evaluate the inhibitory activity of test compounds against HBV in HepG2.2.15 cells.

**2. Experimental materials:**

**1) Cells and main reagents**

HepG2.2.15 cells were provided by Shanghai WuXi AppTec Co., Ltd.
Transfection reagent Lipofectamine RNAiMAX (Invitrogen-13778-150).
Hepatitis B virus surface antigen quantitative detection kit (Autobio-CL-0310).
Hepatitis B virus e antigen quantitative detection kit (Autobio-CL-0312).
Fluorescent probe FastStart Universal Probe Master (Roche, 04914058001).

**2) Main instruments**

79001 Real Time PCR system (Applied Biosystems), Synergy 2 (BioTek), and cell counter (Vi-cellTM XR).

**3. Experimental procedures and methods:**

siRNA was transfected into HepG2.2.15 cells as follows:
On day 0, the siRNA was serially diluted in PBS to 8 concentrations. HepG2.2.15 cells were washed with DPBS, digested with trypsin, adjusted to an appropriate density, and seeded at 15,000 cells per well in a 96-well plate with 150 μL culture medium per well, while simultaneously transfecting the siRNA into HepG2.2.15 cells using Lipofectamine RNAiMax. Cells were cultured in a 5% $CO_2$, 37°C incubator for 3 days. The test siRNA was set at 8 concentrations with 3-fold dilution and 2 replicate wells.
On day 3, the culture medium was replaced with fresh culture medium and cultured for an additional 3 days.
On day 6, the cell culture supernatant was collected. The supernatant samples were tested for HBsAg and HBeAg according to the instructions of the hepatitis B virus surface antigen and e antigen quantitative detection kits, and HBV DNA was detected by qPCR.
The procedures for detecting HBsAg or HBeAg were briefly described as follows: 50 μL of sample and standard were added to the reaction plate, respectively, and then 50 μL of enzyme conjugate was added to each well. The mixture was shaken to mix well and incubated at 37°C for 60 minutes. The plate was then washed 5 times with the washing solution, and 50 μL of luminescent substrate was added to each well. After mixing well, the reaction was carried out at room temperature in the dark for 10 minutes. Finally, the chemiluminescence intensity was measured using a microplate reader.
The procedures for detecting HBV DNA were briefly described as follows: The qPCR reaction system was prepared, and supernatant samples or standard samples containing full-length HBV plasmid were added. The PCR reaction program was: 95°C for 10 minutes, followed by cycling mode: 95°C for 15 seconds and 60°C for 1 minute, for a total of 40 cycles. The HBV DNA content in the samples was calculated based on the Ct value of each sample.

Data analysis:

Percentage inhibition of HBsAg, HBeAg, or HBV DNA = (1 - HBsAg, HBeAg, or HBV DNA content in the sample / average HBsAg, HBeAg, or HBV DNA content in the RNAiMAX transfection reagent control) × 100%
The half-maximal effective concentration ($EC_{50}$) values of the compounds against HBV were calculated using GraphPad Prism software.

**4. Experimental results:** See Table 5.

**Table 5. $EC_{50}$ results of compounds in reducing HBsAg, HBeAg, and HBV DNA in Hep2.2.15 cells**

| Test compound | HBsAg $EC_{50}$ (nM) | HBeAg $EC_{50}$ (nM) | HBV DNA $EC_{50}$ (nM) |
|---|---|---|---|
| **Z7** | 0.05 | 0.16 | 0.07 |
| **Z8** | 0.22 | 0.78 | 0.27 |
| **Z9** | 0.12 | 0.60 | 0.24 |
| **Z10** | 0.02 | 0.07 | 0.03 |
| **Z13** | 0.01 | / | / |

Experimental conclusion: The compounds of the present disclosure have the biological activity of significantly reducing HBV HBsAg, HBeAg, and HBV DNA in HepG2.2.15 cells.

**Experimental Example 2: Evaluation of the inhibitory activity of test compounds against HBV using primary human hepatocytes (PHHs)**

**1. Experimental purpose:**

The HBV DNA content in the culture supernatant of primary human hepatocytes (PHHs) was detected by real-time quantitative PCR (real time-qPCR), the hepatitis B surface antigen and e antigen contents were measured by ELISA, and the total RNA content was determined. The $EC_{50}$ values of the compounds were used as indicators to evaluate the inhibitory effect of the compounds on HBV.

**2. Experimental materials:**

1) Cells and reagents:

Frozen primary human hepatocyte (PHH)
Hepatitis B virus surface antigen quantitative detection kit (Autobio-CL-0310).
Hepatitis B virus e antigen quantitative detection kit (Autobio-CL-0312).
RNA extraction kit (Qiagen).
FastKing cDNA first strand synthesis kit (TianGen).
Fluorescent probe FastStart Universal Probe Master (Roche, 04914058001).

2) Main instruments:

79001 Time PCR system (Applied Biosystems), Synergy 2 (BioTek), and cell counter (Vi-cellTM XR).

**3. Experimental procedures and methods:**

On day 0, the compound was serially diluted in PBS to 7 concentrations and added to the cell plate (22 μL/well). The frozen PHH was resuscitated, adjusted to a density of $6.67\times10^5$ cells/mL, and seeded into a 48-well plate (198 μL/well). The total volume per well was 220 μL. After mixing well, the plate was placed in an incubator and cultured overnight at 37°C with 5% $CO_2$. Each compound was tested at 7 concentrations with 3-fold dilution and 2 replicate wells.
On day 1, the culture medium in the culture plate was aspirated, and PHH was infected with D-type HBV (concentrated from HepG2.2.15 cell culture supernatant) at 100 GE/cell. The final concentration of DMSO in the culture medium was 2%.
On days 2, 4, and 6, the culture medium was replaced with fresh culture medium (without compounds).
On day 8, the cell culture supernatant was collected, and HBsAg and HBeAg were tested according to the instructions of the hepatitis B virus surface antigen and e antigen quantitative detection kits, while HBV DNA was detected by qPCR. Finally, the cells were collected to detect HBV RNA.
The procedures for detecting HBsAg or HBeAg were briefly described as follows: 50 μL of sample and standard were added to the reaction plate, respectively, and then 50 μL of enzyme conjugate was added to each well. The mixture was shaken to mix well and incubated at 37°C for 60 minutes. The reaction plate was then washed 5 times with the washing solution, and 50 μL of luminescent substrate was added to each well. After mixing well, the reaction was carried out at room temperature in the dark for 10 minutes. Finally, the chemiluminescence intensity was measured using a microplate reader.
The procedures for detecting HBV DNA were briefly described as follows: The qPCR reaction system was

prepared, and supernatant samples or standard samples containing full-length HBV plasmid were added, respectively. The PCR reaction program was: 95°C for 30 seconds; followed by denaturation at 95°C for 5 seconds and extension at 60°C for 34 seconds, for a total of 40 cycles. Finally, 95°C for 15 seconds and 60°C for 1 minute. The HBV DNA content in the samples was calculated based on the Ct value of each sample.

The procedures for detecting HBV RNA were briefly described as follows: Cells were collected, and RNA was extracted according to the instructions of the Qiagen-74182 RNA extraction kit, and then RNA was reverse transcribed into cDNA following the instructions of the FastKing cDNA first strand synthesis kit. The target gene cDNA was detected by qPCR with GAPDH as the internal reference gene.

The RNA expression level of the target gene in the samples was calculated based on the Ct value of each sample using the ΔΔCt relative quantification method. The relative expression level of the target gene was represented by $2^{-\Delta\Delta CT}$.

The calculation formula was as follows:

CT = average Ct value of the target gene - average Ct value of the internal reference gene;

ΔΔCT = ΔCT (administration group) - ΔCT (control group); relative mRNA expression level of the target gene = $2^{-\Delta\Delta CT}$

**4. Data analysis:**

Percentage inhibition of HBsAg, HBeAg, or HBV DNA = (1 - HBsAg, HBeAg, or HBV DNA content in the sample / average HBsAg, HBeAg, or HBV DNA content in the culture medium control group) × 100%

The half-maximal effective concentration ($EC_{50}$) of the compound against HBsAg, HBeAg, or HBV DNA was calculated using GraphPad Prism software.

**5. Experimental results:** See Table 6.

**Table 6. $EC_{50}$ results of compounds in reducing HBsAg, HBeAg, HBV DNA, and total RNA in PHH cells**

| Compound | HBsAg $EC_{50}$ (nM) | HBeAg $EC_{50}$ (nM) | HBV DNA $EC_{50}$ (nM) | Total RNA $EC_{50}$ (nM) |
|---|---|---|---|---|
| **Z1** | 1.34 | 3.50 | 6.73 | / |
| **Z2** | 1.77 | 4.31 | 9.08 | / |
| **Z3** | 2.19 | 3.77 | 1.82 | / |
| **Z4** | 2.94 | 9.61 | 7.75 | / |
| **Z5** | 2.79 | 9.44 | 11.06 | / |
| **Z6** | 0.97 | 2.29 | 0.76 | / |
| **Z7** | 3.26 | 2.11 | 2.43 | 13.09 |
| **Z8** | 4.65 | 5.54 | 2.68 | 7.49 |
| **Z9** | 2.70 | 3.47 | 2.03 | 3.82 |
| **Z10** | 4.59 | 11.26 | 3.46 | 4.05 |
| **Z13** | 0.15 | / | / | / |
| **Z14** | 0.14 | / | / | / |
| **Z15** | 0.10 | / | / | / |
| **Z16** | 0.28 | / | / | / |

Experimental conclusion: The compounds of the present disclosure have the biological activity of significantly reducing HBV HBsAg, HBeAg, HBV DNA, and RNA in PHH.

**Experimental Example 3: Evaluation of the competitive efficiency of non-fluorescently labeled siRNA with GalNac3-Cy5 in PHH**

**1. Research purpose**

The purpose of this study was to evaluate the competitive efficiency of non-fluorescently labeled test compounds with GalNac3-Cy5 in PHH by flow cytometry.

**2. Control compound GalNac3 and competitive compound:**

The control compound was GalNac3 (EE(ahGalNAc)$_3$ from Kornilova AY, Algayer B, Breslin M, Uebele V. Anal Biochem. 2012; 425(1): 43-46.), and the competitive compound was GalNac3 labeled with Cy5 fluorescence.

**3. Main reagents and cells**

**Table 7**

| Reagent | Supplier and Catalog Number |
|---|---|
| *In vitro* GRO-CP medium | Bioreclamation IVT-S03316 |
| Fetal bovine serum | ExCell Bio-FSP500 |
| Penicillin-streptomycin | Hyclone-SV30010 |
| DMEM medium | Gibco-11995-065 |
| Dulbecco's phosphate buffered saline (DPBS) | Corning-21-031-CVC |
| 0.25% Trypsin-EDTA | Gibco-25200072 |
| Cell staining buffer | Thermo Fisher-00-4222-26 |
| Cell fixation buffer | BD-554655 |
| Primary human hepatocyte (PHH) | BioreclamationIVT-M00995-P (lot: HDS) |

**4. Experimental scheme:**

On day 0, the resuscitated PHH suspension was adjusted to an appropriate density, and the cells were seeded into a 48-well plate.

On day 1, pre-mixed diluted test compounds and Cy5-labeled GalNac3 (with a final DMSO concentration of 2%) were added. The test compounds started at 20 μM, with 3-fold dilutions across 11 concentration points in single wells.

After 4 hours of incubation with the test compounds, the cells were digested, fixed, and the corresponding fluorescence intensity in PHH was measured by flow cytometry.

The control compound was unlabeled GalNac3, with both the test compound and control compound tested at 11 concentration points in single wells. The competitive compound was Cy5-labeled GalNac3 at a final concentration of 0.05 μM.

The $EC_{50}$ values were calculated by analyzing with Graphpad Prism software (four parameter logistic equations).

**Table 8. $EC_{50}$ results of the competitive efficiency of compounds with GalNac3-Cy5 in PHH**

| Test compound | $EC_{50}$ (μM) |
|---|---|
| **Z7** | 0.614 |
| **Z10** | 0.317 |

Experimental conclusion: The compounds of the present disclosure have good competitive efficiency relative to GalNac3-Cy5 in PHH.

**Experimental Example 4: Study on anti-HBV activity and safety in hepatitis B virus mouse model mediated by recombinant adeno-associated virus (AAV-HBV) serotype 8 vector**

**1. Experimental purpose:**

The AAV vector-mediated HBV transfection mouse model was a rapid and efficient HBV model. Utilizing the high hepatotropism of AAV8 vectors, the recombinant adeno-associated virus serotype 8 vector carrying 1.3 copies of the HBV genome (rAAV8-1.3HBV) could efficiently deliver the 1.3 copies of the HBV genome into hepatocytes via tail vein injection in mice. Due to the characteristics of AAV viral vectors, the mediated vectors were capable of sustaining long-term expression. The application of the AAV/HBV model enabled continuous replication of HBV DNA and expression of HBsAg and HBeAg in the mouse liver.

By employing the AAV-HBV mouse model, the levels of HBsAg, HBeAg, and DNA in mouse serum and mouse body weight were measured after treatment with the test compounds to evaluate their *in vivo* anti-HBV efficacy and safety.

**2. Experimental materials:**

C57BL/6 mice, PBS (RNase free) as the vehicle, test compounds, and recombinant virus rAAV8-1.3HBV. The main reagents for this project include QIAamp96 DNA Kit (Qiagen, 51162), FastStart Universal Probe Master (Rox) (Roche, 04914058001), Hepatitis B Virus Surface Antigen Detection Kit (Autobio, CL0310), Hepatitis B Virus e Antigen Detection Kit (Autobio, CL0918), PureLink™ Pro 96 Viral RNA/DNA Kit (Invitrogen, 12280-096A), and FastQuant RT Kit (with gDNase) (TIANGEN, KR106-02). The main instruments include: centrifuge (Beckman Allegra X-15R), multifunctional microplate reader (BioTek, Synergy 2), fluorescence quantitative PCR system (Applied Biosystems, 7900HT Fast Real-time PCR system), and microplate reader (Molecular Devices, SpectraMax 340PC384).

**3. Experimental methods:**

a) Mice were subcutaneously administered with the drug starting from day 34 post-viral injection, with doses of 3 or 30 mg/kg, and this day was designated as day 0. Before administration, all the mice were subjected to submaxillary blood sampling for plasma collection.
b) On days 0, 14, and 21 post-administration, the mice were subjected to blood sampling via submaxillary vein for plasma collection. The collected blood samples were anticoagulated with $K_2$-EDTA, and plasma was collected after centrifugation at 4°C, 7000 g/min for 10 minutes.
c) All the mice were subjected to blood sampling via submaxillary vein for plasma collection. Subsequently, the mice were euthanized by $CO_2$ inhalation, and plasma samples were collected via cardiac puncture, followed by liver sample collection.
d) The samples were sent for testing.

**4. Sample analysis:**

ELISA detection of HBsAg and HBeAg contents in mouse serum: The experimental procedures were performed according to the instructions of the HBsAg ELISA (Autobio, CL 0310) and HBeAg ELISA (Autobio, CL0918) kits.

Detection of HBV DNA contents in mouse plasma by qPCR: HBV DNA was extracted from the plasma, and the experimental procedures were conducted following the QIAamp 96 DNA Blood Kit instructions. The HBV DNA content in mouse plasma was detected by qPCR.

The data were presented as mean + standard error for each group of mouse samples, with n = 5 unless otherwise specified. Statistical analysis was performed using Student's t-test.

**5. Experimental results:**

a) The anti-HBV activity of the test compounds in the AAV-HBV mouse model was evaluated based on the HBsAg content in serum. The HBsAg content in mouse plasma was measured by ELISA. The results are shown in Table 9.

**Table 9. Percentage reduction of HBsAg on days 7, 14, and 21 post-administration in mice relative to day 0**

| Days of detection (days) | Z1 3 mg/kg | Z6 3 mg/kg | Z7 3 mg/kg | Z7 30 mg/kg | Z10 3 mg/kg | Z10 30 mg/kg |
|---|---|---|---|---|---|---|
| 7 | 98.4% | 82.4% | 91.2% | 99.6% | 92.8% | 99.7% |

(continued)

| Days of detection (days) | Z1<br>3 mg/kg | Z6<br>3 mg/kg | Z7<br>3 mg/kg | Z7<br>30 mg/kg | Z10<br>3 mg/kg | Z10<br>30 mg/kg |
|---|---|---|---|---|---|---|
| 14 | 78.4% | 69.3% | 78.8% | 99.2% | 86.0% | 99.8% |
| 21 | 58.3% | 42.4% | 67.7% | 97.4% | 79.6% | 99.6% |

b) The anti-HBV activity of the test compounds in the AAV-HBV mouse model was evaluated based on the HBeAg content in serum. The HBeAg content in mouse plasma was measured by ELISA. The results are shown in Table 10.

**Table 10. Percentage reduction of HBeAg on days 7, 14, and 21 post-administration in mice relative to day 0**

| Days of detection (days) | Z1<br>3 mg/kg | Z6<br>3 mg/kg | Z7<br>3 mg/kg | Z7<br>30 mg/kg | Z10<br>3 mg/kg | Z10<br>30 mg/kg |
|---|---|---|---|---|---|---|
| 7 | 89.3% | 45.4% | 73.7% | 93.5% | 78.5% | 97.0% |
| 14 | 82.4% | 30.8% | 60.5% | 89.5% | 71.7% | 96.4% |
| 21 | 78.1% | 6.0% | 44.0% | 77.4% | 55.8% | 93.8% |

c) The anti-HBV activity of the test compounds in the AAV-HBV mouse model was evaluated based on the DNA content in serum. The HBV DNA content in mouse plasma was measured by quantitative PCR. The results are shown in Table 11.

**Table 11. Percentage reduction of HBV DNA on days 7, 14, and 21 post-administration in mice relative to day 0**

| Days of detection (days) | Z1<br>3 mg/kg | Z6<br>3 mg/kg | Z7<br>3 mg/kg | Z7<br>30 mg/kg | Z10<br>3 mg/kg | Z10<br>30 mg/kg |
|---|---|---|---|---|---|---|
| 7 | 97.0% | 98.8% | 99.1% | 99.5% | 99.1% | 99.3% |
| 14 | 92.2% | 98.0% | 99.0% | 99.5% | 98.0% | 99.3% |
| 21 | 74.5% | 94.5% | 97.7% | 99.5% | 88% | 99.3% |

d) Changes in body weight. The body weight on day 0 was used as the baseline for comparison. In accordance with IACUC guidelines, a 20% weight loss was set as the humane endpoint. Any mouse exhibiting a weight loss exceeding 20% was removed from the experiment. No mice were removed due to weight loss in this experiment.

**Experimental conclusion:**

In this experiment, the test compounds significantly reduce HBsAg, HBeAg, and HBV DNA in the AAV-HBV mouse model. The mice exhibit good tolerance during the experimental procedure.

**Experimental Example 5: Evaluation of compound immunogenicity using hPBMC**

**Experimental purpose:** To evaluate the immunogenicity of test compounds using hPBMC

**Experimental materials and instruments:**

hPMBC cells: Freshly isolated peripheral blood mononuclear cells (PBMC) purchased from Shanghai Sailybio Medical Co., LTD.

Compounds:

**Table 12**

| Name | Brand-item number |
|---|---|
| Test compound | Self-prepared |
| LPS (TLR4 activator) | Beyotime-S1732-25mg |

Reagents:

**Table 13**

| Reagent name | Brand-item number |
|---|---|
| RPMI medium 1640(1×) | Gibco-22400-089 |
| Fetal bovine serum | Excell Bio-FSP500 |
| Penicillin-streptomycin solution | HyClone-SV30010 |
| Human ProcartaPlex Mix&Match 4-plex (IFN alpha, IFN beta, IL-6, TNF alpha) | Thermo PPX-04-MXWCXZF |

Experimental instruments:

**Table 14**

| Instrument name | Supplier | Instrument model |
|---|---|---|
| Centrifuge | Beckman | Allegra-X15R Centrifuge |
| Microscope | Nikon | ECLIPSE-TS100-F |
| Cell counter | Beckman | Vi-cell |
| Luminex®200™ | BIO-RAD | BIO-plex 200 |

**Experimental procedures:**

**Day 1: hPBMC cells were cultured at high density overnight**

The RPMI-1640 cell culture medium containing 10% FBS was prepared in the following ratio: 10% FBS, 1% Penicillin-streptomycin solution in RPMI Medium 1640 media (1×).

The newly purchased two batches of hPBMC, along with 50 mL centrifuge tubes, were centrifuged at 400×g, 25°C for 5 minutes.

The supernatant was discarded, and the hPBMCs were resuspended in 10 mL of culture medium, respectively, and cells were counted.

The cell density was adjusted to $10^7$ cells/mL and transferred to a 25 $cm^2$ cell culture flask, then incubated overnight in a 5% $CO_2$, 37 °C incubator.

**Day 2: hPBMC plating and compound treatment**

Compound dilution

The test compound was serially diluted starting from 15 μg/mL with a 3-fold gradient across 4 concentration points. The 10× gradient-diluted compound was prepared in a 96-well V-bottom plate according to the following dilution scheme. The reference compound GS9688 stock solution (20 mM) was serially diluted starting from 0.5 μM with a 2-fold gradient across 2 concentration points. The 10× gradient-diluted compound was prepared in a 96-well V-bottom plate according to the following dilution scheme. The reference compound LPS_TLR4 activator (specification: 25 mg/tube) was added with 1.25 mL of endotoxin-free physiological water to prepare a 20 mg/mL stock solution. It was then serially diluted starting from 1 μg/mL with a 4-fold gradient across 2 concentration points. The 10× gradient-diluted compound was

prepared in a 96-well V-bottom plate according to the following dilution scheme.

The hPBMCs cultured overnight the previous day were collected into a 50 mL centrifuge tube and centrifuged at 400×g for 5 minutes. The supernatant was removed, and the cell pellet was resuspended in 15 mL of RPMI-1640 medium containing 10% FBS.

The cells were counted using a cell counter, and the density of the resuspended cells was adjusted to $2.2\times10^6$ cells/mL.

10 μL of the compound from step 1 was transferred to a 96-well cell plate, followed by the addition of 90 μL of the cell suspension with adjusted density from step 3 to each well, resulting in a total volume of 100 μL per well and a cell count of $2.0\times10^6$ cells.

The cell plate was placed in a 5% $CO_2$, 37°C constant temperature incubator and cultured for 1 day.

**Day 3: hPBMC cytokine detection**

The contents of IFN alpha, IFN beta, IL-6, and TNF alpha in hPBMC supernatants were measured using the Human Procarta Plex Mix&Match 4-plex Kit, following the instructions of the kit. The general procedure is as follows:

1. The cytokine assay plate was labeled according to the hPBMC plating map.
2. The Magnetic Bead was vortexed for 1 minute, and 50 μL of Magnetic Bead was dispensed into each well of the assay plate using a multichannel pipette.
3. The assay plate was placed on a magnetic stand for 2 minutes to allow the magnetic beads to aggregate. The liquid was discarded, and 150 μL of wash solution was added. After standing for 30 seconds, the liquid was discarded to wash the magnetic beads in the assay plate. The washing step was repeated twice.
4. 50 μL of standard and test samples were added to each well of the assay plate.
5. The assay plate was sealed with the sealing film provided in the kit, covered with a black microplate lid, and incubated overnight at 4°C and 500 rpm on a plate shaker.

**Day 4: hPBMC cytokine detection plate reading**

The assay plate incubated overnight the previous day was transferred to room temperature and continued to shake at 500 rpm for 30 min. The plate was washed using the magnetic stand following the previous steps. All subsequent operations were performed in the dark:

25 μL of Detection Antibody Mixture was added to each well of the assay plate. The plate was sealed with the sealing film, covered with a black microplate lid, and incubated at room temperature and 500 rpm for 30 min on a plate shaker.

The plate washing steps were repeated as previously described.

50 μL of SAPE solution was added to each well, the plate was sealed with the sealing film, covered with a black microplate lid, and incubated at room temperature and 500 rpm for 30 min on a plate shaker.

The plate washing steps were repeated as previously described.

120 μL of Reading Buffer was added to each well of the assay plate. The plate was sealed with the sealing film and covered with a black assay plate lid, then incubated at room temperature and 500 rpm for 5 min on a plate shaker.

The sealing film was removed from the assay plate, and the assay plate was placed in the Luminex instrument to measure the reading.

The Luminex experiment was set up and concentration data were measured using the Bio-plex Manager 5.0 software on the Bio-plexTM200 system. The standard curve of the target factor was fitted with the 5-parameter logistic (5-PL) regression model (automatically fitted by Luminex), and the content of the target factor in the test samples was analyzed based on the fluorescence values measured by the instrument.

**Result analysis:**

The contents of IL-6, TNF-α, IFN-α, and IFN-beta in the supernatant of hPBMC treated with compounds were measured using Luminex to evaluate the immunogenicity of the compounds on hPBMC.

After donor 1 and donor 2 hPBMCs were treated with different doses of the test compounds, the contents of IFN-α, IFN-beta, IL-6, and TNF-α in the cell supernatant were detected. After donor 3 hPBMCs were treated with different doses of the test compounds, the contents of IL-6 and TNF-α in the cell supernatant were detected.

**Experimental results:**

**Table 15. Contents of IFN-α, IFN-beta, IL-6, and TNF-α in cell supernatants after treatment of donor 1 hPBMC with different doses of test compounds**

| Donor 1 | | | | |
|---|---|---|---|---|
| siRNA con (μg/mL) | IFN alpha (pg/mL) | IFN beta (pg/mL) | IL-6 (pg/mL) | TNF alpha (pg/mL) |
| | Z10 | Z10 | Z10 | Z10 |
| 15.00 | 0.81 | 4.48 | 3979.35 | 108.96 |
| 5.00 | 0.81 | 4.48 | 2130.05 | 30.51 |
| 1.67 | 0.81 | 4.48 | 2492.31 | 45.89 |
| 0.56 | 0.81 | 4.48 | 2226.75 | 42.96 |
| *Data for the PBS group: INF-alpha: 0.81 pg/mL; INF-beta: 4.48 pg/mL; IL-6: 2270.91 pg/mL; TNF-alpha: 33.24 pg/mL | | | | |

**Table 16. Contents of IFN-α, IFN-beta, IL-6, and TNF-α in cell supernatants after treatment of donor 2 hPBMC with different doses of test compounds**

| Donor 2 | | | | |
|---|---|---|---|---|
| siRNA con (μg/mL) | IFN alpha (pg/mL) | IFN beta (pg/mL) | IL-6 (pg/mL) | TNF alpha (pg/mL) |
| | Z10 | Z10 | Z10 | Z10 |
| 15.00 | 0.81 | 4.48 | 82.81 | 5.66 |
| 5.00 | 0.81 | 4.48 | 91.11 | 5.66 |
| 1.67 | 0.81 | 4.48 | 81.42 | 5.66 |
| 0.56 | 0.81 | 4.48 | 62.57 | 5.66 |
| *Data for the PBS group: INF-alpha: 0.81 pg/mL; INF-beta: 4.48 pg/mL; IL-6: 120.43 pg/mL; TNF-alpha: 5.66 pg/mL | | | | |

**Table 17. Contents of IL-6 and TNF-α in cell supernatants after treatment of donor 3 hPBMC with different doses of test compounds**

| siRNA con (μg/mL) | IL-6 (pg/mL) | | | | TNF alpha (pg/mL) | | | |
|---|---|---|---|---|---|---|---|---|
| | PBS | Z10 | Z13 | Z14 | PBS | Z10 | Z13 | Z14 |
| 45.00 | 6764 | 8089 | 7977 | 7532 | 193 | 160 | 160 | 188 |
| 15.00 | | 7807 | 7187 | 7596 | | 157 | 160 | 205 |

**Experimental conclusion:** The compounds of the present disclosure exhibit low immunogenicity risk to hPBMC.

**Experimental Example 6. Study on anti-HBV activity and safety in hepatitis B virus mouse model mediated by recombinant adeno-associated virus (AAV-HBV) serotype 8 vector**

**1. Experimental purpose:**

The AAV vector-mediated HBV transfection mouse model was a rapid and efficient HBV model. Utilizing the high hepatotropism of AAV8 vectors, the recombinant adeno-associated virus serotype 8 vector carrying 1.3 copies of the HBV genome (rAAV8-1.3HBV) could efficiently deliver the 1.3 copies of the HBV genome into hepatocytes via tail vein injection in mice. Due to the characteristics of AAV viral vectors, the mediated vectors were capable of sustaining long-term expression. The application of the AAV/HBV model enabled continuous replication of HBV DNA and expression of HBsAg and HBeAg in the mouse liver.

By employing the AAV-HBV mouse model, the levels of HBsAg, HBeAg, and DNA in mouse serum and mouse body weight were measured after treatment with the test compounds to evaluate their *in vivo* anti-HBV efficacy and safety.

**2. Experimental materials:**

C57BL/6 mice, PBS (RNase free) as the vehicle, test compounds, and recombinant virus rAAV8-1.3HBV. The main reagents for this project include QIAamp96 DNA Kit (Qiagen, 51162), FastStart Universal Probe Master (Rox) (Roche, 04914058001), Hepatitis B Virus Surface Antigen Detection Kit (Autobio, CL0310), Hepatitis B Virus e Antigen Detection Kit (Autobio, CL0918), PureLink™ Pro 96 Viral RNA/DNA Kit (Invitrogen, 12280-096A), and FastQuant RT Kit (with gDNase) (TIANGEN, KR106-02). The main instruments include: centrifuge (Beckman Allegra X-15R), multifunctional microplate reader (BioTek, Synergy 2), fluorescence quantitative PCR system (Applied Biosystems, 7900HT Fast Real-time PCR system), and microplate reader (Molecular Devices, SpectraMax 340PC384).

**3. Experimental methods:**

a) Mice were subcutaneously administered with the drug starting from day 34 post-viral injection, with doses of 2, 6, or 12 mg/kg, and this day was designated as day 0. Before adminiatrion, all the mice were subjected to submaxillary blood sampling for plasma collection.

b) On days 0, 14, 21, and 28 post-administration, the mice were subjected to blood sampling via submaxillary vein for plasma collection. The collected blood samples were anticoagulated with $K_2$-EDTA, and plasma was collected after centrifugation at 4°C, 7000 g/min for 10 minutes.

c) All the mice were subjected to blood sampling via submaxillary vein for plasma collection. Subsequently, the mice were euthanized by $CO_2$ inhalation, and plasma samples were collected via cardiac puncture, followed by liver sample collection.

d) The samples were sent for testing.

**4. Sample analysis:**

ELISA detection of HBsAg and HBeAg contents in mouse serum: The experimental procedures were performed according to the instructions of the HBsAg ELISA (Autobio, CL 0310) and HBeAg ELISA (Autobio, CL0918) kits.

Detection of HBV DNA contents in mouse plasma by qPCR: HBV DNA was extracted from the plasma, and the experimental procedures were conducted following the QIAamp 96 DNA Blood Kit instructions. The HBV DNA content in mouse plasma was detected by qPCR.

The data were presented as mean ± standard error for each group of mouse samples, with n = 5 unless otherwise specified. Statistical analysis was performed using Student's t-test.

**5. Experimental results:**

a) The anti-HBV activity of the test compounds in the AAV-HBV mouse model was evaluated based on the HBsAg content in serum. The HBsAg content in mouse plasma was measured by ELISA. The results are shown in Table 18.

**Table 18. Percentage reduction of HBsAg on days 7, 14, 21, and 28 relative to day 0 after administration of Z13 to mice**

| Dose | Percentage reduction of HBsAg | | | |
|---|---|---|---|---|
| | Day 7 | Day 14 | Day 21 | Day 28 |
| 2 mpk | 92.2% | 86.7% | 81.1% | 45.3% |
| 6 mpk | 99.7% | 99.2% | 98.3% | 90.1% |
| 12 mpk | 99.9% | 99.6% | 99.7% | 97.3% |

b) The anti-HBV activity of the test compounds in the AAV-HBV mouse model was evaluated based on the HBeAg content in serum. The HBeAg content in mouse plasma was measured by ELISA. The results are shown in Table 19.

**Table 19. Percentage reduction of HBeAg on days 7, 14, 21, and 28 relative to day 0 after administration of Z13 to mice**

| Dose | Percentage reduction of HBeAg | | | |
|---|---|---|---|---|
| | Day 7 | Day 14 | Day 21 | Day 28 |
| 2 mpk | 71.4% | 63.5% | 57.0% | 3.1% |
| 6 mpk | 94.0% | 91.3% | 86.2% | 78.0% |
| 12 mpk | 97.2% | 96.6% | 94.7% | 86.3% |

c) The anti-HBV activity of the test compounds in the AAV-HBV mouse model was evaluated based on the DNA content in serum. The HBV DNA content in mouse plasma was measured by quantitative PCR. The results are shown in Table 20.

**Table 20. Percentage reduction of HBV DNA on days 7, 14, 21, and 28 relative to day 0 after administration of Z13 to mice**

| Dose | Percentage reduction of HBV-DNA | | | |
|---|---|---|---|---|
| | Day 7 | Day 14 | Day 21 | Day 28 |
| 2 mpk | 99.6% | 99.8% | 99.3% | 98.3% |
| 6 mpk | >99.8% | >99.8% | >99.8% | 99.8% |
| 12 mpk | >99.5% | 99.5% | >99.5% | >99.5% |

d) Changes in body weight. The body weight on day 0 was used as the baseline for comparison. In accordance with IACUC guidelines, a 20% weight loss was set as the humane endpoint. Any mouse exhibiting a weight loss exceeding 20% was removed from the experiment. No mice were removed due to weight loss in this experiment.

**Experimental conclusion:**

In this experiment, the compound of the present disclosure significantly reduced HBsAg, HBeAg, and HBV DNA in the AAV-HBV mouse model, exhibiting a notable dose-response relationship. The mice exhibit good tolerance during the experimental procedure.

**Experimental Example 7: *In vitro* plasma stability study**

**1. Experimental materials**

a) CD-1 mouse plasma, SD rat plasma, human plasma;
b) Test compound: 50 μL of the test compound (1 mg/mL, solvent: nuclease-free water) was diluted with 450 μL of nuclease-free water to 100 μL/mL.
c) Control compound: Patisiran, supplier: MedChemExpress

**2. Experimental procedures**

a) The frozen plasma was thawed under cold water for 10-20 minutes, followed by centrifugation at 3220×g for 5 minutes.
b) Working solutions of the test compound and control compound were prepared. For each time point, 2 μL of the working solution was mixed with 98 μL of blank plasma in duplicate.
c) For T0 samples, the stop solution was added immediately after the addition of blank plasma and the working solutions of the test compound and control compound.
d) For samples at each time point (0.5, 1, 2, 4, 6, 8, and 24 hours) except T0, incubation was performed in a 37°C water bath.

e) At the end of each time point, 100 μL of an aqueous solution (containing 100 mM ammonium acetate (pH 10.0), 2 mM tris(2-carboxyethyl)phosphine hydrochloride, 1 mM ethylenediaminetetraacetic acid, and 750 ng/mL internal standard) was added and vortex-mixed for 60 seconds.
f) To each sample well, 100 μL of PCL (phenol/chloroform/isoamyl alcohol= 25:24:1) reagent and 200 μL of dichloromethane were added, thoroughly mixed, and centrifuged at 3220×g for 20 minutes.
g) The aqueous layer was transferred to a new 96-well plate, stored at 4°C, and subjected to LC-MS analysis.

**3. Data analysis**

The remaining percentage of test compounds after incubation in plasma is calculated as follows: remaining percentage (%) = 100 * (PAR at incubation time point / PAR at T0), where PAR is the peak area ratio of the analyte to the internal standard.
The incubation time points are T0 (0 min) and Tn (n = 0, 0.5, 1, 2, 4, 6, 8, 24 hours).

**4. Experimental results:** See Table 21

**Table 21. Plasma stability of compounds**

| Test compound | Time point (hr) | Remaining percentage | | | $T_{1/2}$ (hr) | $T_{1/2}$ (hr) | $T_{1/2}$ (hr) |
|---|---|---|---|---|---|---|---|
| | | Mouse | Rat | Human | Mouse | Rat | Human |
| Z13 | 0 | 100.0 | 100.0 | 100.0 | 0.1 | 0.1 | 0.3 |
| | 0.5 | 5.0 | 0.7 | 18.4 | | | |
| | 1 | 0.7 | 0.5 | 2.9 | | | |
| | 2 | 0.5 | 0.4 | 1.0 | | | |
| | 4 | 0.4 | 0.2 | 0.5 | | | |
| | 6 | 0.3 | 0.2 | 0.4 | | | |
| | 8 | 0.3 | 0.2 | 0.2 | | | |
| | 24 | 0.1 | 0.0 | 0.3 | | | |
| Patisiran | 0 | 100.0 | 100.0 | 100.0 | 0.2 | 0.1 | 0.5 |
| | 0.5 | 19.6 | 3.7 | 59.3 | | | |
| | 1 | 3.9 | 0.0 | 38.4 | | | |
| | 2 | 0.0 | 0.0 | 15.0 | | | |
| | 4 | 0.0 | 0.0 | 0.4 | | | |
| | 6 | 0.0 | 0.0 | 0.0 | | | |
| | 8 | 0.0 | 0.0 | 0.0 | | | |
| | 24 | 0.0 | 0.0 | 0.0 | | | |
| $T_{1/2}$ represents half-life | | | | | | | |

**Experimental conclusion:** The compound of the present disclosure exhibits rapid metabolic rates in the plasma of rats, mice, and humans.

**Experimental Example 8: *In vitro* liver S9 stability study**

**1. Experimental materials**

a) Liver S9: Human and animal S9 were purchased from qualified suppliers such as BioIVT and stored in a -80°C

freezer.

b) Control compound: Patisiran, Supplier: MedChemExpress, Batch No.: 155113

**2. Experimental procedures**

2.1 Preparation of buffer and working solution

Buffer: The buffer was prepared using nuclease-free ultrapure water containing 100 mM tris(hydroxymethyl) aminomethane hydrochloride, 1 mM $MgCl_2$, and 1× penicillin-streptomycin dual antibiotic solution. The pH of the solution was adjusted to 6.00 ± 0.10 using hydrochloric acid.

S9 working solution: The liver S9 solution was diluted to 1.05 mg/mL with buffer.

Test compound and control compound working solution: 1.00 mg/mL test compound and control compound were diluted to 2.00 μg/mL with nuclease-free water.

2.2 Experimental procedures

a) Seven 96-well incubation plates were prepared and labeled as T0, T1, T4, T8, T24, T48, and Blank48, respectively. The corresponding reaction time points for the first six incubation plates were 0, 1, 4, 8, 24, and 48 hours. The Blank48 plate was not supplemented with test compounds or control compounds, and the reaction was terminated after 48 hours of incubation. All time point samples were assayed in duplicate.

b) 190 μL of S9 working solution (protein concentration of 1.05 mg/mL) was added to the T0, T1, T4, T8, T24, T48, and Blank48 plates, respectively. Except for T0, the incubation plates were pre-incubated in a 37°C water bath for approximately 10 minutes.

c) After pre-incubation, 10 μL of the test compound or control stock solution was added to the T0, T1, T4, T8, T24, and T48 plates, respectively, while 10 μL of water was added to the Blank48 plate. Subsequently, the T1, T4, T8, T24, T48, and Blank48 (excluding T0) incubation plates were placed in a 37°C water bath to initiate the reaction. The final reaction volume was 200 μL. For sample wells containing liver S9, the protein concentration of liver S9 was 1.00 mg/mL; for sample wells containing the test compound or control stock solution, the final reaction concentration was 2.00 μg/mL.

d) For T0 samples, 200 μL of stop solution (an aqueous solution containing 2.00 mM tris(2-carboxyethyl)phosphine hydrochloride, 100 mM ammonium acetate, 1.00 mM ethylenediaminetetraacetic acid, and 750 ng/mL internal standard) was added and thoroughly mixed, followed by the addition of 200 μL of a mixed solution (phenol: chloroform: isoamyl alcohol = 25:24:1) and thorough mixing for 10 minutes. Subsequently, 400 μL of dichloromethane was added, thoroughly mixed, and centrifuged at 4°C and 3220×g for 20 minutes.

e) At the end of each incubation time point (1, 4, 8, 24, and 48 hours), the corresponding incubation plate was removed from the water bath and processed according to the T0 sample treatment method. 100 μL of supernatant from each sample was taken for LC-MS/MS analysis.

**3. Sample analysis**

The antisense strand of the test compounds in this study was analyzed and determined by liquid chromatography-tandem mass spectrometry (LC-MS/MS). The retention times of the analyte and internal standard, chromatogram acquisition, and chromatogram integration were processed using the Analyst software (Sciex, Framingham, MA, USA).

**4. Data analysis**

The *in vitro* elimination rate constant $k_e$ for the test compound and control compound was obtained by converting the ratio of the internal standard peak areas of the compounds in the following formula into remaining percentages:

$$\%\text{Remaining percentage} = \frac{\text{Peak area ratio of analyte to internal standard at any time point}}{\text{Peak area ratio of analyte to internal standard at 0 hours}} \times 100$$

$$C_t = C_0 \times e^{-k_e \times t} \quad \text{when } C_t = \frac{1}{2} C_0 \quad T_{1/2} = \frac{Ln2}{k_e} = \frac{0.693}{k_e}$$

The *in vitro* liver S9 intrinsic clearance ($CL_{int(S9)}$) and liver intrinsic clearance ($CL_{int(liver)}$) are calculated from $k_e$.

$CL_{int(S9)}$ = 0.693 / $T_{1/2}$ / S9 protein content (S9 concentration during incubation in mg/mL)

$$CL_{int(liver)} = CL_{int\ (S9)} \times \text{S9 protein content in liver (mg/g)} \times \text{liver-to-body weight ratio}$$

The parameters used in the formula are shown in Table 22 below.

**Table 22. Liver-to-body weight ratios and corresponding S9 protein contents for different species**

| Species | Liver-to-body weight ratio (g/kg Body Weight) | S9 protein content (mg/g liver weight) |
|---|---|---|
| Mouse | 88 | 121 |
| Rat | 40 | 140 |
| Human | 20 | 160 |

**5. Experimental results: See Table 23**

**Table 23. Liver S9 stability of compounds**

| Sample name | Rat liver S9 | | Human liver S9 | | Mouse liver S9 | |
|---|---|---|---|---|---|---|
| | $T_{1/2}$ (h) | Remaining percentage (T = 48 hours) | $T_{1/2}$ (h) | Remaining percentage (T = 48 hours) | $T_{1/2}$ (h) | Remaining percentage (T = 48 hours) |
| Z13 | 104.9 | 76.9% | 16.6 | 12.5% | 101.5 | 70.7% |
| Patisiran | 1.4 | 0.0% | 1.5 | 0.0% | 1.3 | 0.0% |
| *$T_{1/2}$ represents half-life | | | | | | |

**Experimental conclusion:** The antisense strand of the compound of the present disclosure exhibits good stability in liver S9 of mice, rats, and humans.

**Experimental Example 9: Off-target study of the RNA sequence of compounds of the present disclosure**

**1. Experimental introduction:**

The transcriptome refers to the complete set of RNA transcribed from a specific tissue or cell at a given time or under specific conditions, primarily including mRNA and non-coding RNA. Transcriptome sequencing, based on the Illumina sequencing platform, is utilized to study all mRNA transcribed from specific tissues or cells during a particular period. It serves as the foundation for research on gene function and structure, playing an important role in understanding organismal development and disease pathogenesis. With the advancement of gene sequencing technology and the reduction in sequencing costs, RNA-seq has become the primary method for transcriptome research due to its advantages of high throughput, high sensitivity, and broad application scope. The RNA-seq workflow primarily consists of two parts: library preparation and sequencing, and bioinformatics analysis.

**2. Research methods:**

After HepG2.2.15 cells were incubated with siRNA or PBS for 24 h, the cells were lysed to extract RNA, followed by RNA-seq analysis to identify differentially expressed genes between each siRNA group and the PBS control group. The enrichment of differentially expressed genes was detected by comparing different databases. HepG2.2.15 cells were transfected with 100 nM siRNA using Lipofectamine RNAiMax, cultured in a 5% $CO_2$ incubator at 37°C for 24 hours, and then the cells were collected for RNA extraction. Subsequently, the RNA samples underwent rigorous quality control,

primarily via the Agilent 2100 bioanalyzer to precisely assess RNA integrity.

**3. Library preparation and quality inspection:**

mRNA was obtained through two main methods: first, by utilizing the structural feature of most eukaryotic mRNA having a polyA tail, using Oligo(dT) magnetic beads to enrich polyA-tailed mRNA. Second, by removing ribosomal RNA from total RNA to obtain mRNA. The obtained mRNA was then randomly fragmented in NEB Fragmentation Buffer using divalent cations, followed by library preparation according to the standard NEB library preparation method or strand-specific library preparation method.

Standard NEB library preparation: The fragmented mRNA served as the template, with random oligonucleotides as primers, to synthesize the first strand of cDNA in the M-MuLV reverse transcriptase system. Subsequently, the RNA strand was degraded by RNaseH, and the second strand of cDNA was synthesized using dNTPs as raw materials in the DNA polymerase I system. The purified double-stranded cDNA underwent end repair, A-tailing, and sequencing adapter ligation. cDNA of approximately 250-300 bp were selected using AMPure XP beads, followed by PCR amplification. The PCR products were further purified with AMPure XP beads to ultimately obtain the library. The NEBNext® Ultra™ RNA Library Prep Kit for Illumina® was used for library preparation.

Strand-specific library preparation: The method for reverse transcription synthesis of the first chain of cDNA was identical to the standard NEB library preparation method. The difference was that when synthesizing the second chain, dTTP in dNTPs was replaced by dUTP. Subsequently, cDNA end repair, A-tailing, sequencing adapter ligation, and size selection were performed. The second strand of cDNA containing U was then degraded using USER enzyme prior to PCR amplification to obtain the library. Strand-specific libraries had many advantages, such as obtaining more valid information with the same amount of data; obtaining more accurate gene quantification, localization, and annotation information; and providing antisense transcripts and the expression level of a single exon in each isoform. The NEBNext® Ultra™ Directional RNA Library Prep Kit for Illumina® was used for library preparation.

Note: The sequencing adapters include three parts: P5/P7, index, and Rd1/Rd2 SP. Among them, P5/P7 are the PCR amplification primers and the binding sites for primers on the flow cell; the index provides information to distinguish different libraries, and Rd1/Rd2 SP, *i.e.,* read1/read2 sequence primer, is the binding region for sequencing primers. The sequencing process theoretically starts from Rd1/Rd2 SP and proceeds backward.

After library preparation was completed, the Qubit2.0 Fluorometer was first used for preliminary quantification, and the library was diluted to 1.5 ng/μL. Subsequently, the Agilent 2100 bioanalyzer was employed to detect the insert size of the library. Once the insert size met expectations, qRT-PCR was performed to accurately quantify the effective concentration of the library (effective library concentration was higher than 2 nM) to ensure library quality.

**4. Sequencing:**

After passing the library quality control, different libraries were pooled according to the effective concentration and the target data output, followed by Illumina sequencing. The fundamental principle of sequencing was Sequencing by Synthesis. Four fluorescently labeled dNTPs, DNA polymerase, and adapter primers were added to the flow cell for amplification. During the extension of the complementary strand in each sequencing cluster, the incorporation of each fluorescently labeled dNTP released a corresponding fluorescent signal. The sequencer captured these fluorescent signals, which were then converted into sequencing peaks by computer software, thereby obtaining the sequence information of the fragments to be tested.

**5. Experimental results:**

Differentially expressed gene analysis was performed using DESeq2 with a threshold of padj $\leq 0.05$ $|\log_2$Fold-Change$| \geq 1.0$. The statistical results are shown in Table 24 below.

**Table 24. Statistical results of differentially expressed genes**

| Comparison group name | Total number of differentially expressed genes | Up-regulated differentially expressed genes | Maximum up-regulation magnitude | Downregulated differentially expressed genes | Maximum down-regulation magnitude | Threshold |
|---|---|---|---|---|---|---|
| Z13 vs Control | 5 | 2 | 2.06 | 3 | 2.37 | DESeq2 padj≤0.05 \| $\log_2$FoldChange\| ≥1.0 |

**Experimental conclusion:** The off-target risk of the compound of the present disclosure is relatively low.

**Experimental Example 10: Preliminary safety study of compounds of the present disclosure in rats**

**1. Experimental materials**

a) Test compound: Compound of the present disclosure
b) Male rats (3), SPF grade, purchased from Beijing Vital River Laboratory Animal Technology Co., Ltd.

**2. Experimental procedures**

a) Preparation of formulation for administration: The vehicle was enzyme-free phosphate-buffered saline (pH 7.4, 1X). The test compounds were prepared as clear solutions at 15 mg/mL.
b) The body weight of the animals was measured prior to administration, and the administration volume was calculated based on body weight (according to the administration volume of 150 mpk). On the day of administration, the overall health status and body surface of the animals were observed.
c) A single subcutaneous injection of the test compound solution was administered on the first day, and cage-side observations were conducted daily.
d) At 336 hours, blood samples (approximately 1.5 mL) were collected via jugular venipuncture and placed into blood collection tubes containing dipotassium ethylenediaminetetraacetate anticoagulant, sodium citrate anticoagulant, and silica clot activator, respectively. The samples were then placed on wet ice for blood routine, blood coagulation, and blood biochemistry tests.

**3. Experimental results**

**The experimental results of compound Z13 are as follows:**

a) Body weight and behavioral monitoring: No abnormalities
b) Blood routine: No abnormalities in white blood cells (WBCs), neutrophils (NEUTs), lymphocytes (LYMPHs), monocytes (MONOs), eosinophils (EOSs), basophils (BASOs), red blood cells (RBCs), hemoglobin (HGB), hematocrit (HCT), mean corpuscular volume (MCV), mean corpuscular hemoglobin (MCH), mean corpuscular hemoglobin concentration (MCHC), red cell distribution width (RDW), platelet count (PLT), or mean platelet volume (MPV); one rat showed a slight increase in reticulocytes (RETs).
c) Blood coagulation: No abnormalities in prothrombin time (PT), activated partial thromboplastin time (APTT), or fibrinogen (FIB).
d) Blood biochemistry: No abnormalities in alanine aminotransferase (ALT) or aspartate aminotransferase (AST).

**4. Experimental conclusion:** The preliminary safety of the compound of the present disclosure is good.

## Claims

1. A double-stranded siRNA analog, a conjugate thereof, a salt thereof, or a salt of the conjugate thereof, comprising a sense strand and an antisense strand capable of forming a double-stranded region, wherein the double-stranded siRNA analog is any one selected from the group consisting of the double strands shown in Table 1, and each nucleotide on the double strand is independently and optionally modified.

2. The double-stranded siRNA analog, the conjugate thereof, the salt thereof, or the salt of the conjugate thereof according to claim 1, wherein the double-stranded siRNA analog is selected from the group consisting of S1, S2, S3, S4, S5, S6, S7, S8, S9, S10, S11, S12, S13, S14, S15, S16, S17, S18, S19, S20, S21, S22, S23, S24, S25, S26, S27, and S28.

3. The double-stranded siRNA analog, the conjugate thereof, the salt thereof, or the salt of the conjugate thereof according to claim 2, wherein the double-stranded siRNA analog is selected from the group consisting of S1, S2, S3, S4, S5, S6, S7, S8, S9, S10, and S11.

4. The double-stranded siRNA analog, the conjugate thereof, the salt thereof, or the salt of the conjugate thereof according to claim 1, wherein the double-stranded siRNA analog is any one selected from the group consisting of the double strands shown in Table 2.

5. The double-stranded siRNA analog, the conjugate thereof, the salt thereof, or the salt of the conjugate thereof according to claim 1, wherein the conjugate of the double-stranded siRNA analog or the salt of the conjugate thereof is formed by conjugating the double-stranded siRNA analog as defined in any one of claims 1-4 with a pharmaceutically acceptable conjugating group.

6. The double-stranded siRNA analog, the conjugate thereof, the salt thereof, or the salt of the conjugate thereof according to claim 5, wherein the pharmaceutically acceptable conjugating group comprises 1 to 5 GalNAc groups.

7. The double-stranded siRNA analog, the conjugate thereof, the salt thereof, or the salt of the conjugate thereof according to claim 6, wherein the pharmaceutically acceptable conjugating group is linked to the 3' end of the sense strand of the double-stranded siRNA analog.

8. The double-stranded siRNA analog, the conjugate thereof, the salt thereof, or the salt of the conjugate thereof according to claim 5, wherein the pharmaceutically acceptable conjugating group is selected from the group consisting of:

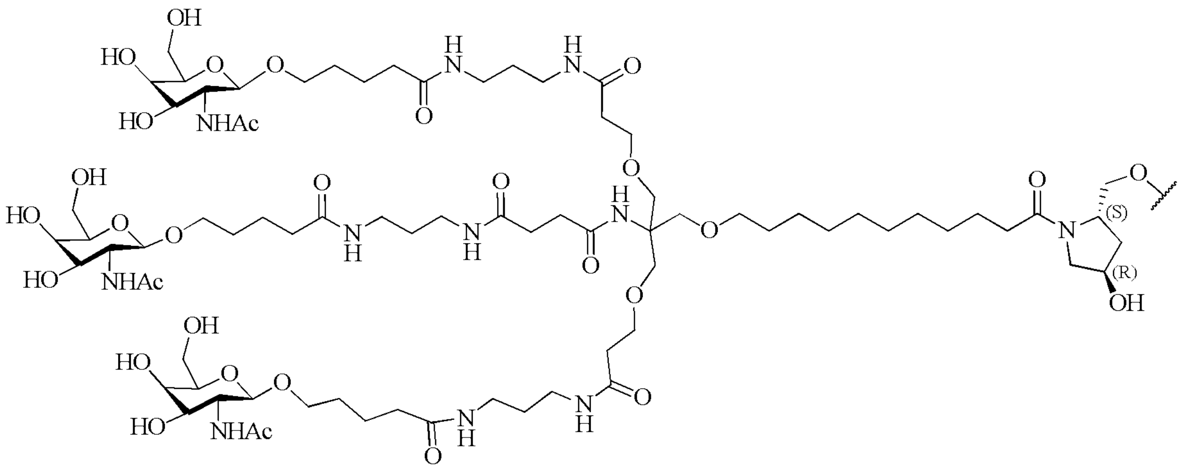

D1

,

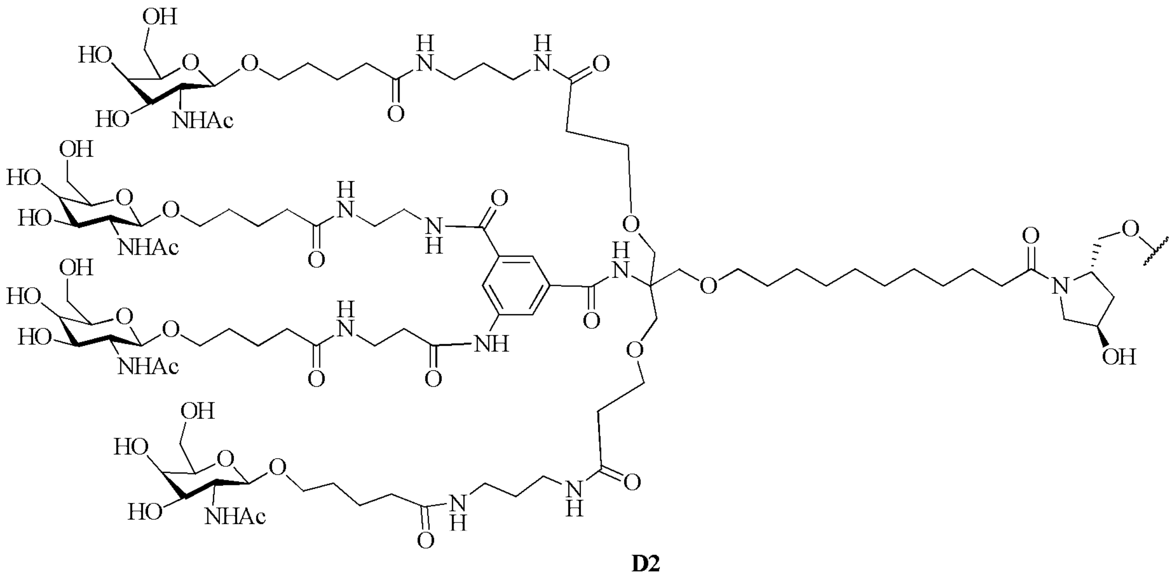

D2

,

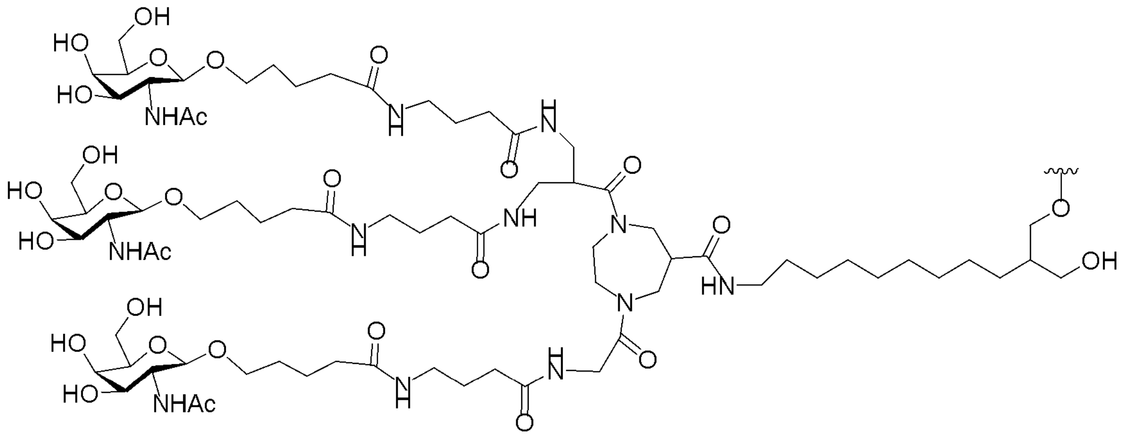

D3

,

and

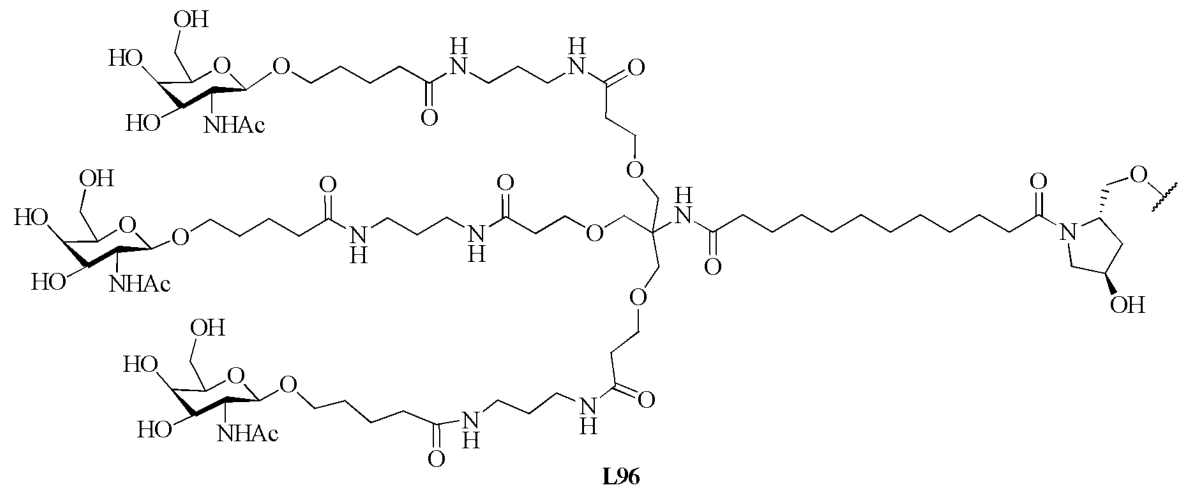

L96

.

**9.** A conjugate of a double-stranded siRNA analog or a salt thereof, wherein the conjugate of the double-stranded siRNA

analog or the salt thereof is selected from the group consisting of Z1, Z2, Z3, Z4, Z5, Z6, Z7, Z8, Z9, Z10, Z11, Z12, Z13, Z14, Z15, and Z16.

**10.** Use of the double-stranded siRNA analog, the conjugate thereof, the salt thereof, or the salt of the conjugate thereof as defined in any one of claims 1-9 in the manufacture of a medicament for treating hepatitis B.

## INTERNATIONAL SEARCH REPORT

International application No.
**PCT/CN2024/078398**

**A. CLASSIFICATION OF SUBJECT MATTER**

C12N15/113(2010.01)i； A61K31/713(2006.01)i； A61P31/20(2006.01)i

According to International Patent Classification (IPC) or to both national classification and IPC

**B. FIELDS SEARCHED**

Minimum documentation searched (classification system followed by classification symbols)

IPC:C12N,A61K,A61P

Documentation searched other than minimum documentation to the extent that such documents are included in the fields searched

Electronic data base consulted during the international search (name of data base and, where practicable, search terms used)

CNTXT, WPABSC, WPABS, ENTXT, CJFD, CNKI, 万方, WANFANG, 百度学术, BAIDU SCHOLAR, PubMed, ISI web of knowledge, bing: 南京明德, Medshine Discovery, 乙肝, HBV, 双链, double stranded, siRNA, 缀合物, conjugate+, 修饰, modifi+

**C. DOCUMENTS CONSIDERED TO BE RELEVANT**

| Category* | Citation of document, with indication, where appropriate, of the relevant passages | Relevant to claim No. |
|---|---|---|
| X | WO 2021110148 A1 (MEDSHINE DISCOVERY INC.) 10 June 2021 (2021-06-10)<br>description, page 2, paragraph 2-page 10, paragraph 2 | 1-10 |
| X | CN 104107437 A (XIAMEN CHENGKUN BIOTECHNOLOGY CO., LTD.) 22 October 2014 (2014-10-22)<br>claims 1-7 | 1-3, 10 |
| A | US 2022049249 A1 (SUZHOU RIBO LIFE SCIENCE CO., LTD.) 17 February 2022 (2022-02-17)<br>entire document | 1-10 |
| A | EP 3719125 A1 (SUZHOU RIBO LIFE SCIENCE CO., LTD.) 07 October 2020 (2020-10-07)<br>entire document | 1-10 |
| A | CN 110959011 A (SUZHOU RIBO LIFE SCIENCE CO., LTD.) 03 April 2020 (2020-04-03)<br>entire document | 1-10 |
| A | CN 108064162 A (IONIS PHARMACEUTICALS, INC.) 22 May 2018 (2018-05-22)<br>entire document | 1-10 |

☐ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **10 May 2024** | **17 May 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**
**Information on patent family members**

International application No.
**PCT/CN2024/078398**

| Patent document cited in search report | | | Publication date (day/month/year) | Patent family member(s) | | | Publication date (day/month/year) |
|---|---|---|---|---|---|---|---|
| WO | 2021110148 | A1 | 10 June 2021 | US | 2023220386 | A1 | 13 July 2023 |
| CN | 104107437 | A | 22 October 2014 | None | | | |
| US | 2022049249 | A1 | 17 February 2022 | WO | 2019105418 | A1 | 06 June 2019 |
| | | | | KR | 20200091414 | A | 30 July 2020 |
| | | | | EP | 3719128 | A1 | 07 October 2020 |
| | | | | EP | 3719128 | A4 | 27 October 2021 |
| | | | | JP | 2023134615 | A | 27 September 2023 |
| | | | | CA | 3083968 | A1 | 06 June 2019 |
| | | | | US | 2023132756 | A1 | 04 May 2023 |
| | | | | US | 11492620 | B2 | 08 November 2022 |
| | | | | TW | 201925469 | A | 01 July 2019 |
| | | | | TWI | 791696 | B | 11 February 2023 |
| | | | | JP | 2021504415 | A | 15 February 2021 |
| | | | | AU | 2018374219 | A1 | 21 May 2020 |
| | | | | AU | 2018374219 | B2 | 15 December 2022 |
| | | | | AU | 2018374219 | C1 | 11 May 2023 |
| EP | 3719125 | A1 | 07 October 2020 | JP | 2021503930 | A | 15 February 2021 |
| | | | | JP | 7360716 | B2 | 13 October 2023 |
| | | | | AU | 2018377716 | A1 | 09 April 2020 |
| | | | | RU | 2020118025 | A | 04 January 2022 |
| | | | | RU | 2020118025 | A3 | 09 March 2022 |
| | | | | US | 2022395526 | A1 | 15 December 2022 |
| | | | | CA | 3083970 | A1 | 06 June 2019 |
| | | | | TW | 201928057 | A | 16 July 2019 |
| | | | | TWI | 823879 | B | 01 December 2023 |
| | | | | JP | 2023179555 | A | 19 December 2023 |
| | | | | KR | 20200095483 | A | 10 August 2020 |
| | | | | WO | 2019105437 | A1 | 06 June 2019 |
| | | | | EP | 3719125 | A4 | 08 September 2021 |
| CN | 110959011 | A | 03 April 2020 | TW | 201929905 | A | 01 August 2019 |
| | | | | TWI | 826405 | B | 21 December 2023 |
| | | | | AU | 2018394875 | A1 | 09 April 2020 |
| | | | | AU | 2018394875 | B2 | 03 August 2023 |
| | | | | JP | 2021509402 | A | 25 March 2021 |
| | | | | JP | 7436030 | B2 | 21 February 2024 |
| | | | | US | 2020338201 | A1 | 29 October 2020 |
| | | | | US | 11633482 | B2 | 25 April 2023 |
| | | | | CA | 3087106 | A1 | 04 July 2019 |
| | | | | US | 2023257827 | A1 | 17 August 2023 |
| | | | | ZA | 202003833 | B | 26 January 2022 |
| | | | | JP | 2023171772 | A | 05 December 2023 |
| | | | | KR | 20200105812 | A | 09 September 2020 |
| | | | | KR | 102617947 | B1 | 27 December 2023 |
| | | | | EP | 3732185 | A1 | 04 November 2020 |
| | | | | EP | 3732185 | A4 | 10 November 2021 |
| | | | | WO | 2019128611 | A1 | 04 July 2019 |
| | | | | RU | 2020121741 | A | 01 February 2022 |
| CN | 108064162 | A | 22 May 2018 | JP | 2016526874 | A | 08 September 2016 |
| | | | | JP | 6387084 | B2 | 05 September 2018 |

Form PCT/ISA/210 (patent family annex) (July 2022)

**INTERNATIONAL SEARCH REPORT**
**Information on patent family members**

International application No.
**PCT/CN2024/078398**

| Patent document cited in search report | Publication date (day/month/year) | Patent family member(s) | Publication date (day/month/year) |
|---|---|---|---|
| | | JP 2020058370 A | 16 April 2020 |
| | | MX 2020004209 A | 13 August 2020 |
| | | WO 2014179620 A1 | 06 November 2014 |
| | | US 2016076032 A1 | 17 March 2016 |
| | | US 9714421 B2 | 25 July 2017 |
| | | IL 263843 A | 31 January 2019 |
| | | IL 263843 B | 31 March 2020 |
| | | KR 20190084138 A | 15 July 2019 |
| | | KR 102212275 B1 | 05 February 2021 |
| | | RS 60796 B1 | 30 October 2020 |
| | | EA 201891479 A1 | 30 November 2018 |
| | | EA 036584 B1 | 26 November 2020 |
| | | RU 2019124314 A | 21 August 2019 |
| | | MX 2019010441 A | 17 October 2019 |
| | | ES 2778442 T3 | 10 August 2020 |
| | | IL 261901 A | 31 October 2018 |
| | | IL 261901 B | 31 May 2020 |
| | | KR 20230006933 A | 11 January 2023 |
| | | JP 2016523515 A | 12 August 2016 |
| | | JP 6456362 B2 | 23 January 2019 |
| | | JP 2021020901 A | 18 February 2021 |
| | | JP 7177127 B2 | 22 November 2022 |
| | | HUE 050394 T2 | 30 November 2020 |
| | | US 2023151365 A1 | 18 May 2023 |
| | | US 2016017323 A1 | 21 January 2016 |
| | | US 2018273953 A1 | 27 September 2018 |
| | | US 10883104 B2 | 05 January 2021 |
| | | US 2015176007 A1 | 25 June 2015 |
| | | US 9145558 B2 | 29 September 2015 |
| | | AU 2017200365 A1 | 23 February 2017 |
| | | AU 2017200365 B2 | 08 November 2018 |
| | | AU 2017200365 C1 | 18 April 2019 |
| | | EP 2991656 A2 | 09 March 2016 |
| | | EP 2991656 A4 | 22 February 2017 |
| | | EP 2991656 B1 | 18 December 2019 |
| | | IL 242124 B | 28 February 2019 |
| | | EP 3633039 A1 | 08 April 2020 |
| | | IL 296543 A | 01 November 2022 |
| | | HRP 20201378 T1 | 27 November 2020 |
| | | AU 2014259759 A1 | 22 October 2015 |
| | | AU 2014259759 B2 | 18 June 2020 |
| | | US 2021395734 A1 | 23 December 2021 |
| | | US 2021130823 A1 | 06 May 2021 |
| | | IL 274064 A | 30 June 2020 |
| | | IL 274064 B | 30 June 2021 |
| | | IL 242132 B | 31 October 2018 |
| | | PT 2992098 T | 05 July 2019 |
| | | CA 2921162 A1 | 06 November 2014 |
| | | JP 2020039355 A | 19 March 2020 |
| | | LT 2992098 T | 10 July 2019 |

Form PCT/ISA/210 (patent family annex) (July 2022)

**INTERNATIONAL SEARCH REPORT**
**Information on patent family members**

International application No.
**PCT/CN2024/078398**

| Patent document cited in search report | Publication date (day/month/year) | Patent family member(s) | Publication date (day/month/year) |
|---|---|---|---|
| | | AU 2022202770 A1 | 19 May 2022 |
| | | AU 2014259757 A1 | 22 October 2015 |
| | | AU 2014259757 B2 | 02 March 2017 |
| | | JP 2023113843 A | 16 August 2023 |
| | | HK 1221404 A1 | 02 June 2017 |
| | | IL 264241 A | 28 February 2019 |
| | | IL 264241 B | 30 April 2020 |
| | | US 2018273952 A1 | 27 September 2018 |
| | | US 10927372 B2 | 23 February 2021 |
| | | UA 120287 C2 | 11 November 2019 |
| | | AU 2020207820 A1 | 06 August 2020 |
| | | MY 178929 A | 23 October 2020 |
| | | AU 2014259755 A1 | 22 October 2015 |
| | | AU 2014259755 B2 | 30 August 2018 |
| | | CR 20190269 A | 13 September 2019 |
| | | AU 2019200820 A1 | 28 February 2019 |
| | | AU 2019200820 B2 | 30 April 2020 |
| | | US 2016076030 A1 | 17 March 2016 |
| | | US 9932580 B2 | 03 April 2018 |
| | | KR 20210151260 A | 13 December 2021 |
| | | KR 102558571 B1 | 21 July 2023 |
| | | ES 2819213 T3 | 15 April 2021 |
| | | PT 3524680 T | 04 January 2021 |
| | | US 2020224198 A1 | 16 July 2020 |
| | | JP 2020007361 A | 16 January 2020 |
| | | JP 7429103 B2 | 07 February 2024 |
| | | MX 2015015263 A | 16 December 2016 |
| | | HK 1221486 A1 | 02 June 2017 |
| | | AU 2018267625 A1 | 13 December 2018 |
| | | AU 2018267625 B2 | 10 September 2020 |
| | | EP 2992009 A1 | 09 March 2016 |
| | | EP 2992009 A4 | 28 December 2016 |
| | | EP 2992009 B1 | 24 June 2020 |
| | | CL 2015003217 A1 | 08 July 2016 |
| | | AU 2019203674 A1 | 27 June 2019 |
| | | AU 2019203674 B2 | 25 March 2021 |
| | | SG 11201508800 WA | 27 November 2015 |
| | | NZ 740338 A | 29 April 2022 |
| | | RU 2018136140 A | 17 December 2018 |
| | | RU 2018136140 A3 | 27 April 2022 |
| | | IL 264580 A | 28 February 2019 |
| | | IL 264580 B | 30 April 2020 |
| | | CY 1123369 T1 | 31 December 2021 |
| | | MX 2020002184 A | 14 July 2020 |
| | | HK 1221475 A1 | 02 June 2017 |
| | | BR 112015027319 A2 | 26 September 2017 |
| | | BR 112015027319 A8 | 02 January 2018 |
| | | HUE 043697 T2 | 30 September 2019 |
| | | US 2021024923 A1 | 28 January 2021 |
| | | US 2022275365 A9 | 01 September 2022 |

Form PCT/ISA/210 (patent family annex) (July 2022)

**INTERNATIONAL SEARCH REPORT**
**Information on patent family members**

International application No.
**PCT/CN2024/078398**

| Patent document cited in search report | Publication date (day/month/year) | Patent family member(s) | | | Publication date (day/month/year) |
|---|---|---|---|---|---|
| | | CY | 1121879 | T1 | 14 October 2020 |
| | | KR | 20210014758 | A | 09 February 2021 |
| | | PE | 20152002 | A1 | 21 January 2016 |
| | | NZ | 631512 | A | 28 October 2016 |
| | | IL | 273184 | A | 30 April 2020 |
| | | IL | 273184 | B | 29 July 2021 |
| | | IL | 273205 | A | 30 April 2020 |
| | | EP | 2991661 | A1 | 09 March 2016 |
| | | EP | 2991661 | A4 | 15 February 2017 |
| | | EP | 2991661 | B1 | 13 March 2019 |
| | | DOP | 2021000095 | A | 15 September 2021 |
| | | CL | 2016002262 | A1 | 09 June 2017 |
| | | EP | 2992097 | A2 | 09 March 2016 |
| | | EP | 2992097 | A4 | 04 January 2017 |
| | | EP | 2992097 | B1 | 06 November 2019 |
| | | RU | 2015151203 | A | 02 June 2017 |
| | | RU | 2650510 | C2 | 16 April 2018 |
| | | WO | 2014179629 | A2 | 06 November 2014 |
| | | WO | 2014179629 | A3 | 22 January 2015 |
| | | WO | 2014179629 | A8 | 02 June 2016 |
| | | BR | 112015027321 | A2 | 26 September 2017 |
| | | BR | 112015027321 | A8 | 02 January 2018 |
| | | KR | 20180051678 | A | 16 May 2018 |
| | | KR | 102138781 | B1 | 28 July 2020 |
| | | EP | 3828275 | A1 | 02 June 2021 |
| | | IL | 283660 | A | 29 July 2021 |
| | | KR | 20160002976 | A | 08 January 2016 |
| | | KR | 102315836 | B1 | 22 October 2021 |
| | | SG | 11201508870 | VA | 27 November 2015 |
| | | HK | 1221403 | A1 | 02 June 2017 |
| | | PE | 20161430 | A1 | 06 January 2017 |
| | | AU | 2017203436 | A1 | 08 June 2017 |
| | | AU | 2017203436 | B2 | 18 October 2018 |
| | | IL | 242125 | B | 28 February 2019 |
| | | ES | 2730015 | T3 | 07 November 2019 |
| | | PH | 12015502493 | A1 | 22 February 2016 |
| | | KR | 20220108195 | A | 02 August 2022 |
| | | KR | 102651423 | B1 | 27 March 2024 |
| | | RU | 2015151199 | A | 05 June 2017 |
| | | RU | 2015151199 | A3 | 27 March 2018 |
| | | RU | 2697152 | C2 | 12 August 2019 |
| | | MX | 2015015234 | A | 03 October 2016 |
| | | ME | 03390 | B | 20 January 2020 |
| | | DK | 3524680 | T3 | 14 December 2020 |
| | | JP | 2018027091 | A | 22 February 2018 |
| | | JP | 6592486 | B2 | 16 October 2019 |
| | | JP | 2019056001 | A | 11 April 2019 |
| | | JP | 6639629 | B2 | 05 February 2020 |
| | | JP | 2021107408 | A | 29 July 2021 |
| | | JP | 7339294 | B2 | 05 September 2023 |

Form PCT/ISA/210 (patent family annex) (July 2022)

**INTERNATIONAL SEARCH REPORT**
**Information on patent family members**

International application No.
**PCT/CN2024/078398**

| Patent document cited in search report | Publication date (day/month/year) | Patent family member(s) | | | Publication date (day/month/year) |
|---|---|---|---|---|---|
| | | NZ | 753018 | A | 28 January 2022 |
| | | SG | 10201801507 | RA | 28 March 2018 |
| | | WO | 2014179627 | A2 | 06 November 2014 |
| | | WO | 2014179627 | A9 | 26 February 2015 |
| | | WO | 2014179627 | A3 | 16 April 2015 |
| | | EP | 2992098 | A2 | 09 March 2016 |
| | | EP | 2992098 | A4 | 11 January 2017 |
| | | EP | 2992098 | B1 | 27 March 2019 |
| | | BR | 112015027369 | A2 | 26 September 2017 |
| | | BR | 112015027369 | A8 | 02 January 2018 |
| | | BR | 112015027369 | B1 | 08 June 2021 |
| | | JP | 2016522817 | A | 04 August 2016 |
| | | JP | 6216444 | B2 | 18 October 2017 |
| | | JP | 2016522683 | A | 04 August 2016 |
| | | JP | 6995478 | B2 | 14 January 2022 |
| | | US | 2018044676 | A1 | 15 February 2018 |
| | | US | 10683499 | B2 | 16 June 2020 |
| | | JP | 2020074787 | A | 21 May 2020 |
| | | DOP | 2016000287 | A | 15 February 2017 |
| | | DK | 2992098 | T3 | 17 June 2019 |
| | | WO | 2014179626 | A2 | 06 November 2014 |
| | | WO | 2014179626 | A3 | 26 February 2015 |
| | | PL | 2992098 | T3 | 30 September 2019 |
| | | CA | 2921514 | A1 | 06 November 2014 |
| | | CA | 2921514 | C | 24 October 2023 |
| | | US | 2016090596 | A1 | 31 March 2016 |
| | | US | 9957504 | B2 | 01 May 2018 |
| | | NZ | 725538 | A | 26 February 2021 |
| | | JP | 2018183184 | A | 22 November 2018 |
| | | JP | 6652602 | B2 | 26 February 2020 |
| | | AU | 2021204244 | A1 | 22 July 2021 |
| | | AU | 2021204244 | B2 | 19 October 2023 |
| | | NZ | 631552 | A | 24 February 2017 |
| | | SG | 10201906382 | QA | 27 August 2019 |
| | | US | 2015126719 | A1 | 07 May 2015 |
| | | US | 9163239 | B2 | 20 October 2015 |
| | | RU | 2018112167 | A | 07 March 2019 |
| | | ZA | 201507218 | B | 27 September 2023 |
| | | SI | 2992009 | T1 | 30 October 2020 |
| | | IL | 273312 | A | 30 April 2020 |
| | | KR | 20240042220 | A | 01 April 2024 |
| | | CA | 2921518 | A1 | 06 November 2014 |
| | | NZ | 728517 | A | 24 December 2021 |
| | | US | 2016090595 | A1 | 31 March 2016 |
| | | US | 9932581 | B2 | 03 April 2018 |
| | | PH | 12019501191 | A1 | 01 March 2021 |
| | | US | 2014343123 | A1 | 20 November 2014 |
| | | US | 9127276 | B2 | 08 September 2015 |
| | | ZA | 201507216 | B | 30 August 2017 |
| | | JP | 2023012548 | A | 25 January 2023 |

Form PCT/ISA/210 (patent family annex) (July 2022)

**INTERNATIONAL SEARCH REPORT**
**Information on patent family members**

International application No.
**PCT/CN2024/078398**

| Patent document cited in search report | Publication date (day/month/year) | Patent family member(s) | | | Publication date (day/month/year) |
|---|---|---|---|---|---|
| | | EP | 3524680 | A1 | 14 August 2019 |
| | | EP | 3524680 | B1 | 11 November 2020 |
| | | NZ | 712737 | A | 27 August 2021 |
| | | US | 2018002693 | A1 | 04 January 2018 |
| | | AU | 2017200950 | A1 | 02 March 2017 |
| | | AU | 2017200950 | B2 | 17 January 2019 |
| | | MY | 198359 | A | 28 August 2023 |
| | | WO | 2014179625 | A1 | 06 November 2014 |
| | | MX | 2021008901 | A | 19 August 2021 |
| | | ES | 2885174 | T3 | 13 December 2021 |
| | | JP | 2020039354 | A | 19 March 2020 |
| | | JP | 6866459 | B2 | 28 April 2021 |
| | | AU | 2014259756 | A1 | 22 October 2015 |
| | | AU | 2014259756 | B2 | 23 February 2017 |
| | | US | 2015126720 | A1 | 07 May 2015 |
| | | US | 9181550 | B2 | 10 November 2015 |
| | | IL | 272617 | A | 31 March 2020 |
| | | UA | 121017 | C2 | 25 March 2020 |
| | | BR | 112015027377 | A2 | 29 August 2017 |
| | | BR | 112015027377 | A8 | 03 October 2017 |
| | | BR | 112015027377 | B1 | 10 January 2023 |
| | | EP | 3690049 | A1 | 05 August 2020 |
| | | MX | 2021008899 | A | 19 August 2021 |
| | | JP | 2022017514 | A | 25 January 2022 |
| | | KR | 20210037752 | A | 06 April 2021 |
| | | US | 2019367914 | A1 | 05 December 2019 |
| | | US | 10844379 | B2 | 24 November 2020 |
| | | MX | 2015015264 | A | 12 August 2016 |
| | | CA | 2921167 | A1 | 06 November 2014 |
| | | MX | 2015015220 | A | 12 January 2016 |
| | | AU | 2020233603 | A1 | 01 October 2020 |
| | | PL | 2992009 | T3 | 30 November 2020 |
| | | AU | 2024200296 | A1 | 08 February 2024 |
| | | JP | 2021074021 | A | 20 May 2021 |
| | | DK | 2992009 | T3 | 14 September 2020 |
| | | EA | 201592093 | A1 | 30 June 2016 |
| | | EA | 031393 | B1 | 28 December 2018 |
| | | IL | 284593 | A | 31 August 2021 |
| | | IL | 284593 | B | 01 October 2022 |
| | | IL | 284593 | B2 | 01 February 2023 |
| | | KR | 20160002977 | A | 08 January 2016 |
| | | AU | 2019204784 | A1 | 25 July 2019 |
| | | AU | 2019204784 | B2 | 27 January 2022 |
| | | AU | 2019204784 | C1 | 03 November 2022 |
| | | MX | 2015015239 | A | 03 October 2016 |
| | | KR | 20230113835 | A | 01 August 2023 |
| | | JP | 2024010070 | A | 23 January 2024 |
| | | AU | 2020217347 | A1 | 27 August 2020 |
| | | RU | 2015151204 | A | 02 June 2017 |
| | | RU | 2015151204 | A3 | 27 March 2018 |

Form PCT/ISA/210 (patent family annex) (July 2022)

**INTERNATIONAL SEARCH REPORT**
**Information on patent family members**

International application No.
**PCT/CN2024/078398**

| Patent document cited in search report | Publication date (day/month/year) | Patent family member(s) | | | Publication date (day/month/year) |
|---|---|---|---|---|---|
| | | RU | 2686080 | C2 | 24 April 2019 |
| | | AU | 2014259750 | A1 | 22 October 2015 |
| | | AU | 2014259750 | B2 | 28 February 2019 |
| | | HK | 1221485 | A1 | 02 June 2017 |
| | | KR | 20200090966 | A | 29 July 2020 |
| | | IL | 242126 | B | 31 January 2019 |
| | | KR | 20160003723 | A | 11 January 2016 |
| | | KR | 102424855 | B1 | 26 July 2022 |
| | | EP | 4155403 | A1 | 29 March 2023 |
| | | BR | 112015027322 | A2 | 26 September 2017 |
| | | BR | 112015027322 | A8 | 02 January 2018 |
| | | NZ | 631537 | A | 26 May 2017 |
| | | PH | 12018501963 | A1 | 20 July 2020 |
| | | AU | 2019202598 | A1 | 02 May 2019 |
| | | RU | 2015151202 | A | 06 June 2017 |
| | | RU | 2015151202 | A3 | 27 March 2018 |
| | | RU | 2670614 | C2 | 24 October 2018 |
| | | RU | 2670614 | C9 | 23 November 2018 |
| | | PT | 2992009 | T | 21 September 2020 |
| | | SI | 2992098 | T1 | 28 June 2019 |
| | | US | 2015126718 | A1 | 07 May 2015 |
| | | US | 9181549 | B2 | 10 November 2015 |
| | | DOP | 2015000268 | A | 30 November 2015 |
| | | RU | 2019110030 | A | 06 May 2019 |
| | | CR | 20150612 | A | 03 March 2016 |
| | | US | 2019055554 | A1 | 21 February 2019 |
| | | US | 11299736 | B1 | 12 April 2022 |
| | | CA | 2921509 | A1 | 06 November 2014 |
| | | RU | 2015151200 | A3 | 14 January 2019 |
| | | RU | 2015151200 | A | 11 September 2019 |
| | | RS | 58981 | B1 | 30 August 2019 |
| | | LT | 2992009 | T | 10 November 2020 |
| | | KR | 20160002975 | A | 08 January 2016 |
| | | KR | 101857707 | B1 | 14 May 2018 |
| | | IL | 284000 | A | 29 July 2021 |
| | | JP | 2016526018 | A | 01 September 2016 |
| | | JP | 6769866 | B2 | 14 October 2020 |
| | | EP | 3546579 | A1 | 02 October 2019 |
| | | HRP | 20190987 | T1 | 20 September 2019 |
| | | DK | 2991656 | T3 | 23 March 2020 |
| | | US | 2021087566 | A1 | 25 March 2021 |
| | | US | 11851655 | B2 | 26 December 2023 |
| | | SG | 10201801813 | YA | 27 April 2018 |
| | | KR | 20160002974 | A | 08 January 2016 |
| | | KR | 102235678 | B1 | 05 April 2021 |
| | | MX | 2019010443 | A | 17 October 2019 |
| | | IL | 270464 | B | 29 July 2021 |
| | | KR | 20210129257 | A | 27 October 2021 |
| | | KR | 102482890 | B1 | 30 December 2022 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 2023101774460
- CN 2023101914409
- CN 2023104534435
- CN 202311511833X
- CN 2024100843576

### Non-patent literature cited in the description

- **KORNILOVA AY** ; **ALGAYER B** ; **BRESLIN M** ; **UEBELE V**. *Anal Biochem.*, 2012, vol. 425 (1), 43-46